(19) Europäisches Patentamt
European Patent Office
Office européen des brevets

(11) EP 3 972 591 B1

(12) EUROPEAN PATENT SPECIFICATION

(45) Date of publication and mention of the grant of the patent:
**28.12.2022 Bulletin 2022/52**

(21) Application number: **20760507.2**

(22) Date of filing: **12.08.2020**

(51) International Patent Classification (IPC):
***A61K 31/427*** *(2006.01)* ***A61K 31/522*** *(2006.01)*
***A61K 31/496*** *(2006.01)* ***A61K 31/15*** *(2006.01)*
***A61K 31/404*** *(2006.01)* ***A61K 31/37*** *(2006.01)*
***A61K 31/138*** *(2006.01)* ***A61K 31/4164*** *(2006.01)*
***A61P 35/00*** *(2006.01)* ***A61P 31/00*** *(2006.01)*
***A61P 29/00*** *(2006.01)*

(52) Cooperative Patent Classification (CPC):
(C-Sets available)
**A61K 31/138; A61K 31/15; A61K 31/37; A61K 31/404; A61K 31/4164; A61K 31/427; A61K 31/496; A61K 31/522;** Y02A 50/30 (Cont.)

(86) International application number:
**PCT/GB2020/051920**

(87) International publication number:
**WO 2022/034275 (17.02.2022 Gazette 2022/07)**

(54) **PHARMACEUTICAL COMPOSITIONS COMPRISING FLUBENDAZOLE**

PHARMAZEUTISCHE ZUSAMMENSETZUNGEN MIT FLUBENDAZOL

COMPOSITIONS PHARMACEUTIQUES COMPRENANT DU FLUBENDAZOLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**30.03.2022 Bulletin 2022/13**

(73) Proprietor: **Zephapharm Ltd.**
**London W1W 6XB (GB)**

(72) Inventor: **TAYLOR, John.**
**London W1W 6XB (GB)**

(74) Representative: **Titmus, Craig Edward et al**
**Mathys & Squire**
**The Shard**
**32 London Bridge Street**
**London SE1 9SG (GB)**

(56) References cited:
- **ZANGER ULRICH M ET AL: "Cytochrome P450 enzymes in drug metabolism: Regulation of gene expression, enzyme activities, and impact of genetic variation", PHARMACOLOGY AND THERAPEUTICS, ELSEVIER, GB, vol. 138, no. 1, 16 January 2013 (2013-01-16), pages 103-141, XP029001361, ISSN: 0163-7258, DOI: 10.1016/J.PHARMTHERA.2012.12.007**
- **KENT L. KUNZE ET AL: "Isoform-selective mechanism-based inhibition of human cytochrome P450 1A2 by furafylline", CHEMICAL RESEARCH IN TOXICOLOGY, vol. 6, no. 5, 1 September 1993 (1993-09-01), pages 649-656, XP55118600, ISSN: 0893-228X, DOI: 10.1021/tx00035a009**
- **TASHINGA E BAPIRO ET AL: "Artemisinin and thiabendazole are potent inhibitors of cytochrome P450 1A2 (CYP1A2) activity in humans", EUROPEAN JOURNAL OF CLINICAL PHARMACOLOGY, SPRINGER, BERLIN, DE, vol. 61, no. 10, 1 November 2005 (2005-11-01), pages 755-761, XP019329977, ISSN: 1432-1041, DOI: 10.1007/S00228-005-0037-3**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SANTOSH CHAUHAN ET AL: "Pharmaceutical screen identifies novel target processes for activation of autophagy with a broad translational potential", NATURE COMMUNICATIONS, vol. 6, no. 1, 27 October 2015 (2015-10-27), XP055577961, DOI: 10.1038/ncomms9620 cited in the application**
- **CÁNOVÁ KRISTÝNA ET AL: "Anthelmintic Flubendazole and Its Potential Use in Anticancer Therapy", ACTA MEDICA LEKARSKE FAKULTY UNIVERZITY KARLOVY V HRADCI KRALOVE, vol. 60, no. 1, 1 January 2017 (2017-01-01), pages 5-11, XP55797271, CZ ISSN: 1211-4286, DOI: 10.14712/18059694.2017.44**
- **HOU ZHI-JIE ET AL: "Flubendazole, FDA-approved anthelmintic, targets breast cancer stem-like cells", ONCOTARGET, vol. 6, no. 8, 20 March 2015 (2015-03-20), pages 6326-6340, XP55797282, DOI: 10.18632/oncotarget.3436 Retrieved from the Internet: URL:https://www.ncbi.nlm.nih.gov/pmc/articles/PMC4467440/pdf/oncotarget-06-6326.pdf>**
- **NIXON GEMMA L. ET AL: "Repurposing and Reformulation of the Antiparasitic Agent Flubendazole for Treatment of Cryptococcal Meningoencephalitis, a Neglected Fungal Disease", ANTIMICROBIAL AGENTS AND CHEMOTHERAPY, vol. 62, no. 4, 8 January 2018 (2018-01-08), XP55797296, US ISSN: 0066-4804, DOI: 10.1128/AAC.01909-17 Retrieved from the Internet: URL:https://aac.asm.org/content/aac/62/4/e01909-17.full.pdf>**
- **MAINOU BERNARDO A. ET AL: "ABSTRACT", MBIO, vol. 4, no. 4, 30 August 2013 (2013-08-30) , XP55797309, DOI: 10.1128/mBio.00405-13 Retrieved from the Internet: URL:https://mbio.asm.org/content/mbio/4/4/e00405-13.full.pdf>**
- **YU CHEN GUANG ET AL: "Repositioning Flubendazole for Spinal Cord Injury", JOURNAL OF NEUROTRAUMA., vol. 36, no. 18, 15 September 2019 (2019-09-15), pages 2618-2630, XP55797328, US ISSN: 0897-7151, DOI: 10.1089/neu.2018.6160 Retrieved from the Internet: URL:https://www.ncbi.nlm.nih.gov/pmc/articles/PMC6727476/pdf/neu.2018.6160.pdf>**


(52) Cooperative Patent Classification (CPC): (Cont.)

C-Sets
**A61K 31/138, A61K 2300/00;**
**A61K 31/15, A61K 2300/00;**
**A61K 31/37, A61K 2300/00;**
**A61K 31/404, A61K 2300/00;**
**A61K 31/4164, A61K 2300/00;**
**A61K 31/427, A61K 2300/00;**
**A61K 31/496, A61K 2300/00;**
**A61K 31/522, A61K 2300/00**

## Description

### Field of the invention

The present invention relates to a pharmaceutical composition comprising flubendazole and a moderate or strong cytochrome P450 1A2 isoenzyme (CYP1A2) inhibitor for use in medicine. The invention also relates to pharmaceutical compositions comprising flubendazole and a CYP1A2 inhibitor for use in a method of treating a disease treatable by inhibition and/or disruption of microtubule structure and function.

### Background to the invention

Benzimidazole compounds represent a class of pharmacologically active compounds which are known to have activity at biological targets associated with the treatment of a range of diseases. Benzimidazoles such as flubendazole, mebendazole, nocodazole, benomyl, carbendazim, oxifendazole (oxfendazole), albendazole, ricobendazole (albendazole sulphoxide), thiabendazole, fenbendazole and triclabendazole are known to act as inhibitors and/or disruptors of microtubule structure and function in cells, with effects on tubulin polymerization. These effects are well-known in the art (e.g. Spagnuolo et al., 2010; Michaelis et al., 2015; Oh et al., 2018; Lin et al., 2019).

Benzimidazoles act on microtubules in animal cells, including cancer cells, as well as in parasitic cells and in fungal cells, resulting in arrest/inhibition of cell proliferation (Spagnuolo et al., 2010; Oh et al., 2018). Benzimidazoles are known to have antiparasitic activity including anthelminthic and antiprotozoal activity and antifungal activity. Benzimidazole compounds, including flubendazole, have also been found to have additional mechanisms of action such as impairment or reversion of epithelial-mesenchymal transition (EMT), which are relevant to all types of cancer / tumours, other non-cancer proliferative diseases, inflammatory diseases including autoimmune inflammatory diseases and gout, and fibrotic diseases (e.g. Cronstein and Terkeltaub, 2006; Kalluri and Weinberg, 2009; Dinarello, 2010; Hou et al., 2015; Wildenberg et al., 2017; Kralova et al., 2018; Yang et al., 2020).

Other therapeutic targets and/or mechanisms for flubendazole have been shown to include dysregulation of cuticle-associated gene expression (O'Neill et al., 2016); anti-tumour action by triggering cell apoptosis, and HER2 signaling (Zhou et al., 2018; Tao et al., 2019); p53-mediated apoptosis (Michaelis et al., 2015); anti-tumour action by suppressing the NF-κB signaling pathway (Tao et al., 2019); anti-tumour action by blocking STAT3 signaling and activating autophagy (including targeting ATG4B and EVA1A) (Chauhan et al., 2015; Panda et al., 2019; Agrotis and Ketteler., 2019; Zhen et al., 2020; Lin et al., 2019); induction of apoptosis, accompanied by G2/M phase accumulation, caspase-3/-7 activation and the dysregulation of STAT3 activation in triple-negative breast cancer cells, with inhibition of tumour growth, angiogenesis and lung and liver metastasis, coinciding with decreased MMP-2 and MMP-9 levels in circulating blood (Oh et al., 2018); suppression of cancer stem-like cells (Hou et al., 2015); inhibition of activation of glial fibrillary acidic protein, reduction in cyclin B1 expression and Bruton tyrosine kinase activation, B cell activation/proliferation and inflammation, and reduced B cell autoimmune response (Yu et al., 2019).

Flubendazole (IUPAC name: methyl *N*-[6-(4-fluorobenzoyl)-1*H*-benzimidazol-2-yl]carbamate) has been shown to have potent anti-proliferative activity by microtubule inhibition and/or disruption in mammalian cells including tumour cells, as well as in parasitic cells and in fungal cells. Flubendazole is also known to cause impairment or reversion of EMT.

Various studies of flubendazole and its medicinal properties have been carried out. Flubendazole was originally discovered and developed by Dr. Janssen and his research team, and was first approved for human use in 1980 to treat soil transmitted helminths, also called intestinal worms (Lachau-Durand et al., 2019).

Flubendazole potently inhibits proliferating cells, including parasites, fungal cells, tumour cells and non-tumour cells. The long-established mechanism of action of flubendazole is microtubule inhibition and/or disruption by the binding of animal tubulin, including mammalian cellular tubulin, and helminth, protozoal and fungal cellular tubulin.

Tubulin is vital to cell division and is therefore a cancer target for several widely used chemotherapy drugs, including paclitaxel, colchicine, and vincristine. The antiparasitic action of flubendazole is due to its action as a microtubule-disrupting agent acting to prevent the polymerisation of tubulin, causing parasites to die. Flubendazole also has antifungal activity. Flubendazole has been shown to have other mechanisms of action with therapeutic activity against various diseases.

Despite the potential therapeutic uses for flubendazole, its application in the clinic has been severely hampered by a number of pharmacokinetic (PK) and tolerability challenges and limitations. Low systemic bioavailability, unsuitable PK or unsuitable safety profiles of flubendazole formulations have limited the development of systemic clinical/therapeutic treatments based on flubendazole, as discussed in the published literature (e.g. Ceballos et al., 2011; Ceballos et al., 2014; O'Neill et al., 2016; Nixon et al., 2018; Geary et al., 2019). Flubendazole has previously been considered to be insufficiently bioavailable in various formulations, due to being too poorly systemically absorbed *via* the gastrointestinal tract. Furthermore, flubendazole was not locally tolerated at and around the injection site when administered parenterally. Clinical treatments, which require adequate systemic PK of therapeutic concentrations of flubendazole, while limiting

formation of toxic and/or inactive metabolites, have not been found following various research activities over many years. For at least these reasons, flubendazole development programmes for the treatment of various diseases have been terminated, thus requiring a novel approach for the clinical development of flubendazole for antiparasitic, antifungal, and anticancer treatments and the treatment of other diseases treatable by flubendazole.

Prior attempts to improve the therapeutic utility of flubendazole have been unsuccessful. By way of background, phase I metabolism of flubendazole includes hydrolysis of the carbamoyl methyl moiety accompanied by a decarboxylation (hydrolysed flubendazole) and a carbonyl reduction of flubendazole (reduced flubendazole), as shown below.

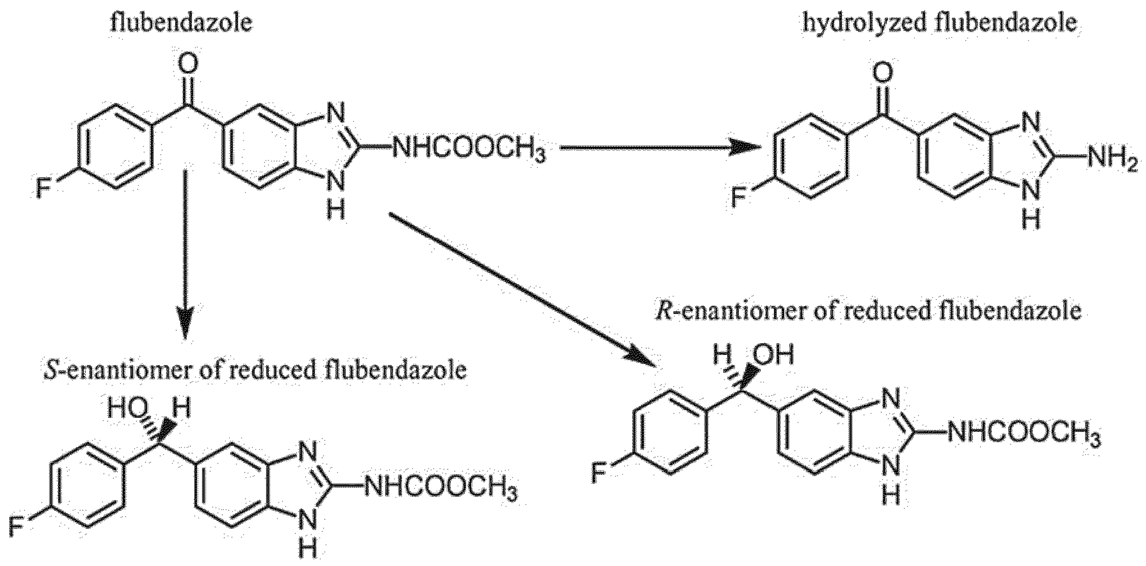


(Figure copied from Nobilis et al., 2007)

Involvement of carbonyl reductase 1 (CBR1) in the formation of reduced flubendazole has been shown in previous studies (Kubicek et al., 2019; and Stuchlikova et al., 2018). However, importantly from a clinical safety perspective, inhibition of CBR1 was found not to be a feasible PK improvement strategy for flubendazole, since CBR1 inhibits malignant behaviour and EMT, and also has been shown to inhibit metastasis of head and neck squamous cell carcinoma. CBR1 is present in a variety of organs including liver, kidney, breast, ovary and vascular endothelial cells, and plays an important role in protecting cells from oxidative stress *via* inactivation of highly reactive lipid aldehydes and controls fatty acid metabolism (Kajimura et al., 2019; Yun et al., 2020). Inhibition of CBR1 is therefore not a clinically viable option for improving the therapeutic utility of flubendazole.

Flubendazole has issues of low systemic bioavailability, which has led to extensive research and development of new formulations including amorphous formulations (Vialpando et al., 2016; Geary et al., 2019; Lachau-Durand et al., 2019) as well as a prodrug of flubendazole (UMF-078) which induced neurotoxicity problems, resulting in the termination of its development (Geary et al., 2019). It was concluded that no flubendazole treatment regimen could be selected by Janssen that would provide efficacy in humans in the treatment of filarial nematodes at safe exposure levels of flubendazole and its metabolites (Lachau-Durand et al., 2019).

Parenteral formulations of flubendazole have been developed showing excellent efficacy in animal models, but intramuscular injection of a flubendazole formulation has induced significant adverse local injection site reactions in patients, thus preventing further development of parenteral formulations with prolonged PK (Geary et al., 2019; Lachau-Durand et al., 2019; Sjoberg et al., 2019). Adverse local injection site reactions have also been observed in animals following subcutaneous dosing (Ceballos et al., 2015).

Either unacceptable local adverse effects or systemic effects due to the dose of flubendazole plus its toxic metabolites have terminated development for various therapeutic systemic uses. For example, Janssen discontinued development of an oral formulation of flubendazole to treat onchocerciasis in 2017 in view of safety concerns, despite best attempts by the company (Janssen / Johnson & Johnson) to fulfil its commitment to the 2012 London Declaration on Neglected Tropical Diseases (Press Release, Titusville, N.J., March 30, 2017).

Flubendazole drug development activities over the last >40 years have, to a greater extent, not been able to overcome issues of extent and variability of systemic exposure to flubendazole and its metabolites to allow development of better and/or acceptable benefit:risk scenarios for therapeutic uses of flubendazole (e.g. Janssen's discontinuation of development of an oral formulation of flubendazole to treat onchocerciasis in 2017). New systemic uses of flubendazole have therefore not been possible for clinically vital treatments of parasites, fungi, cancer and other diseases. As stated

by O'Neill et al. (2016), it remains a goal of reformulation efforts to recapitulate the high efficacy of parenteral flubendazole with an oral regimen.

In summary, despite the promising therapeutic potential of flubendazole, low systemic bioavailability has limited its use in the clinic. Flubendazole has previously been considered to be insufficiently bioavailable in various formulations due to being too poorly absorbed *via* the gastrointestinal tract followed by first-pass metabolism of absorbed flubendazole in the liver. Furthermore, flubendazole has been shown not to be locally tolerated when administered parenterally. Clinical treatments for patients, requiring adequate systemic exposure, including prolonged exposure, to therapeutic concentrations of flubendazole, while limiting formation of toxic and/or therapeutically inactive metabolites, have not been found to date. For the above reasons, development programmes for flubendazole for various diseases have been terminated, thus requiring a novel approach for the clinical development of flubendazole for therapeutic use.

Surprisingly, the Inventor has found that the PK of flubendazole is significantly improved (enhanced) by its combination use with a moderate or strong inhibitor of CYP1A2. The improved PK of flubendazole by this novel mechanism advantageously and unexpectedly improves its safety and efficacy (therapeutic effect) and benefit:risk in the treatment of diseases requiring systemic bioavailability of flubendazole.

The systemic bioavailability of flubendazole following oral administration has been a particular problem for treatment of any disease requiring its systemic availability at therapeutic concentrations, on the basis of its low and variable oral absorption and its rapid, extensive and variable first pass hepatic (intrinsic) clearance. The Inventor utilised human hepatocytes and liver microsomes to specifically research the effect of CYP1A2 inhibitors on flubendazole metabolism for the first time, and has unexpectedly resolved systemic bioavailability problems and improved the PK of this drug.

The improved PK, achieved by reducing intrinsic clearance, advantageously improves therapeutic treatment of diseases by prolonging flubendazole systemic exposure at therapeutic concentrations. Prolonged systemic exposure to flubendazole is considered important for efficacy in various diseases, such as filarial diseases (e.g. O'Neill et al., 2016; Sjoberg et al., 2019). A further advantage is that this correspondingly results in reduced exposure to flubendazole metabolites, which has important safety benefits for patients. Therefore, the novel CYP1A2 inhibition mechanism identified by the Inventor offers an entirely new approach for the treatment of diseases treatable by improved and/or sustained exposure to flubendazole.

The Inventor has surprisingly discovered that the PK of flubendazole can be significantly improved by moderate or strong inhibition of CYP1A2. The invention provides considerable real-world clinical advantages, and simultaneously avoids or reduces clinical safety concerns that have hampered attempts in the prior art.

Thus, improved therapeutic use of flubendazole may be achieved by co-administration with a moderate or strong CYP1A2 inhibitor. Advantages achieved by the present invention include:

- Improved PK of flubendazole, thereby increasing the systemic concentration of the active moiety and prolonging its half-life.
- Administration of lower doses of flubendazole, as the active agent, while prolonging, maintaining and improving therapeutic systemic concentrations of flubendazole.
- Reduction and / or elimination of variability in flubendazole systemic exposure, based on inhibition of variable CYP1A2 activities seen in patients. This is particularly important due to intra- and inter-individual variation (including genetic CYP1A2 polymorphisms) in CYP1A2 activity, and potential for CYP1A2 induction e.g. by smoking.
- Possibility of overcoming resistance problems in different therapeutic uses including treatment of parasitic diseases, fungal diseases, cancer, and other diseases.
- Reduction in the proportion of metabolites compared to parent drug (flubendazole) by its PK enhancement, and concomitant reduction in the toxic side effects of metabolites, while maintaining / prolonging flubendazole therapeutic concentrations in the systemic circulation.
- Reduction in the number of individual doses (e.g. tablets, capsules, oral solutions/suspensions) required each day and reduction in the dosing frequency. An associated advantage is improved medication adherence of patients.
- Improved gastro-intestinal absorption achieved by using lower doses or concentrations of flubendazole in oral formulations. Flubendazole has low solubility and its precipitation potential is increased in the gastrointestinal environment at higher concentrations (Ceballos et al., 2015).

To date, no publications for flubendazole have considered or recommended co-administration (including dose reduction where required for safety reasons) with drugs known to be moderate or strong CYP1A2 inhibitors for PK improvement. Specifically, it has not been established by research until now that flubendazole is metabolised by CYP1A2 and not metabolised to any significant extent by other CYP isoenzymes. Therefore, the present discovery of a method for improving PK of flubendazole using pharmaceutical compositions comprising flubendazole and a strong or moderate CYP1A2 inhibitor also serves to advise clinicians, other health professionals and patients of previously unforeseen drug-drug interaction risks of this combination. The present invention serves to reduce intrinsic clearance and prolong systemic

therapeutic concentrations of flubendazole after administration of flubendazole by co-administration of a moderate or strong CYP1A2 inhibitor, as shown in studies described herein, resulting in concomitant reduced exposure to toxic metabolites and/or therapeutically inactive metabolites. Thus, the novel form of PK improvement achieved by the present invention provides a much improved patient benefit:risk ratio.

Unexpectedly, flubendazole has been shown for the first time in CYP reaction phenotyping investigations to be a highly specific CYP1A2 substrate. Flubendazole gave a surprisingly short half-life value of 2.34 minutes in the presence of CYP1A2 recombinant enzyme. Furthermore, new studies using human liver microsomes and human cryopreserved hepatocytes, confirming this CYP1A2 specificity, reproducibly showed that the metabolism of flubendazole is markedly or completely inhibited by CYP1A2 inhibitors.

In human liver microsomes at the therapeutically relevant concentration of 1 μM flubendazole, the half-life of flubendazole was shown herein to be extended from 27.6 minutes without inhibitor to 394 minutes (6.6 hours) with 10 μM furafylline, a selective strong CYP1A2 inhibitor (i.e. >14 times increase in half-life).

In human cryopreserved hepatocytes using the therapeutically relevant concentration of 1 μM flubendazole, intrinsic (hepatic) clearance values showed strong (≥5-fold) or complete inhibition of flubendazole metabolism at therapeutically relevant concentrations of ≥3 μM furafylline, ≥3 μM fluvoxamine, ≥3 μM thiabendazole and ≥10 μM mexiletine. Flubendazole at 1 μM gave an intrinsic clearance value showing moderate (≥2 to <5-fold) inhibition of its metabolism at a therapeutically relevant concentration of 10 μM cimetidine in human cryopreserved hepatocytes.

The Inventor has surprisingly found that flubendazole is rapidly metabolised by CYP1A2, with a very short half-life value of 2.34 minutes in the presence of CYP1A2 recombinant enzyme. Metabolism of flubendazole in human liver microsomes and human hepatocytes is markedly inhibited by moderate and strong CYP1A2 inhibitors.

Moderate or strong CYP1A2 inhibitors according to the invention include, but are not limited to, furafylline, fluvoxamine, thiabendazole, mexiletine, 8-phenyltheophylline, ciprofloxacin, enoxacin, zafirlukast, cimetidine, and methoxsalen, or a pharmaceutically acceptable salt, solvate, hydrate or *N*-oxide thereof.

As detailed in International application number WO2020/165559 (PCT/ GB2020/050287), the Inventor surprisingly found that ricobendazole (albendazole sulfoxide) metabolism was not inhibited to the same extent by the strong CYP1A2 inhibitor, furafylline, thus demonstrating that PK enhancement of all benzimidazoles by moderate or strong CYP1A2 inhibitors is not predictable. Ricobendazole had low turnover in human liver microsomes and limited effect was observed with the addition of CYP1A2 inhibitors (furafylline and cimetidine). There was zero to 2.2-fold inhibition of intrinsic clearance of ricobendazole in human liver microsomes at most observed with the strong CYP1A2 inhibitor, furafylline, contrasting markedly with the strong (up to 14.3-fold) CYP1A2 inhibition of intrinsic clearance of flubendazole in human liver microsomes by furafylline as summarized hereinbelow. Ricobendazole also showed low turnover by recombinant CYP enzymes; therefore the contribution of specific CYP isoforms to the metabolism of ricobendazole was not conclusive, with only some evidence of very limited metabolic turnover by CYP1A2. This contrasts markedly with the data shown hereinbelow for flubendazole. Identifying benzimidazoles and CYP1A2 inhibitor combinations that exhibit the desired increase in PK of the benzimidazoles required extensive research by assessment of different human CYPs, human liver microsomes and human hepatocytes, in conjunction with various inhibitor drugs and substrate drugs. The Inventor has identified flubendazole as a surprisingly sensitive CYP1A2 substrate.

Intrinsic clearance of flubendazole is shown herein to be strongly (≥5-fold) or completely inhibited at therapeutically relevant concentrations of ≥3 μM furafylline, ≥3 μM fluvoxamine, ≥3 μM thiabendazole, and ≥10 μM mexiletine in human cryopreserved hepatocytes. Intrinsic clearance of flubendazole is shown herein to be moderately (≥2- to <5-fold) inhibited at a therapeutically relevant concentration of 10 μM cimetidine in human cryopreserved hepatocytes.

These CYP1A2 metabolism findings for flubendazole are unexpected and surprising. Moreover, they appear to contradict conclusions in earlier publications (e.g. Ceballos et al., 2014) that carbonyl reductases are the main enzymes involved in flubendazole metabolism, as well as from recent studies performed by researchers at Charles University, Hradec Králové, Czechia (Kubíček V et al., 2019). The findings of these prior studies are in contrast to the findings of studies described herein, which demonstrate that human hepatocyte (intrinsic) clearance of flubendazole is significantly inhibited by moderate and strong CYP1A2 inhibitors.

## Summary of the invention

The invention is defined according to the claims. The present invention provides a pharmaceutical composition comprising flubendazole or a pharmaceutically acceptable salt, solvate, hydrate or N-oxide thereof, and a moderate or strong cytochrome P450 1A2 isoenzyme (CYP1A2) inhibitor, wherein: (a) the moderate CYP1A2 inhibitor is: (i) a CYP1A2 inhibitor which reduces intrinsic clearance of flubendazole in human hepatocytes *ex vivo* by ≥two-fold to <five-fold compared to intrinsic clearance of flubendazole in human hepatocytes *ex vivo* in the absence of a CYP1A2 inhibitor; and/or (ii) a CYP1A2 inhibitor which increases the AUC *in vivo* of flubendazole by ≥two-fold to <five-fold compared to the AUC *in vivo* of flubendazole in the absence of a CYP1A2 inhibitor; and (b) the strong CYP1A2 inhibitor is: (i) a CYP1A2 inhibitor which reduces intrinsic clearance of flubendazole in human hepatocytes *ex vivo* by ≥five-fold compared

to intrinsic clearance of flubendazole in human hepatocytes *ex vivo* in the absence of a CYP1A2 inhibitor; and/or (ii) a CYP1A2 inhibitor which increases the AUC *in vivo* of flubendazole by ≥five-fold compared to the AUC *in vivo* of flubendazole in the absence of a CYP1A2 inhibitor.

In one embodiment, the moderate or strong CYP1A2 inhibitor is selected from furafylline, ciprofloxacin, enoxacin, fluvoxamine, zafirlukast, 8-phenyltheophylline, methoxsalen, thiabendazole, mexiletine and cimetidine, or a pharmaceutically acceptable salt, solvate, hydrate or *N*-oxide thereof.

In one embodiment, the strong CYP1A2 inhibitor is selected from fluvoxamine, thiabendazole, furafylline, mexiletine, 8-phenyltheophylline, ciprofloxacin, enoxacin, and zafirlukast, or a pharmaceutically acceptable salt, solvate, hydrate or *N*-oxide thereof.

In one embodiment, the strong CYP1A2 inhibitor is selected from fluvoxamine, thiabendazole, furafylline, and mexiletine, or a pharmaceutically acceptable salt, solvate, hydrate or *N*-oxide thereof.

In one embodiment, the moderate CYP1A2 inhibitor is selected from cimetidine and methoxsalen.

*Ex vivo* intrinsic clearance of flubendazole is preferably determined using human hepatocytes.

In one embodiment, the *ex vivo* intrinsic clearance of flubendazole is reduced by at least two-fold compared to flubendazole in the absence of a moderate or strong CYP1A2 inhibitor. In one embodiment, the *ex vivo* intrinsic clearance of flubendazole is reduced by at least five-fold compared to flubendazole in the absence of a strong CYP1A2 inhibitor.

In one embodiment, the flubendazole has a greater *in vivo* area under the curve (AUC) compared to flubendazole in the absence of a moderate or strong CYP1A2 inhibitor. In one embodiment, the flubendazole has an *in vivo* AUC at least two-fold more than flubendazole in the absence of a moderate or strong CYP1A2 inhibitor. In one embodiment, the flubendazole has an *in vivo* AUC at least five-fold more than flubendazole in the absence of a strong CYP1A2 inhibitor.

In one embodiment, the flubendazole has a longer *in vivo* half-life than flubendazole in the absence of a moderate of strong CYP1A2 inhibitor. In one embodiment, the *in vivo* half-life of the flubendazole is extended by more than 2, 3, 4, 6, 8, 10, 12, 14, 16, 18 or 20 times the *in vivo* half-life of flubendazole in the absence of the moderate or strong CYP1A2 inhibitor.

In one embodiment, the moderate or strong CYP1A2 inhibitor reduces the *ex vivo* intrinsic clearance of flubendazole by at least two-fold compared to flubendazole in the absence of a moderate or strong CYP1A2 inhibitor. In one embodiment, the strong CYP1A2 inhibitor reduces the *ex vivo* intrinsic clearance of flubendazole by at least five-fold compared to flubendazole in the absence of a strong CYP1A2 inhibitor.

In one embodiment, the moderate or strong CYP1A2 inhibitor increases the *in vivo* AUC of flubendazole compared to flubendazole in the absence of a moderate or strong CYP1A2 inhibitor. In one embodiment, the moderate or strong CYP1A2 inhibitor increases the *in vivo* AUC of flubendazole by at least two-fold more than flubendazole in the absence of a moderate or strong CYP1A2 inhibitor. In one embodiment, the strong CYP1A2 inhibitor increases the *in vivo* AUC of flubendazole by at least five-fold more than flubendazole in the absence of a strong CYP1A2 inhibitor.

In one embodiment, the moderate or strong CYP1A2 inhibitor extends the *in vivo* half-life of flubendazole. In one embodiment, the moderate or strong CYP1A2 inhibitor extends the *in vivo* half-life of flubendazole by more than 2, 3, 4, 6, 8, 10, 12, 14, 16, 18 or 20 times the *in vivo* half-life of flubendazole in the absence of the moderate or strong CYP1A2 inhibitor.

In one embodiment, the pharmaceutical composition of the invention is for use in medicine.

In one embodiment, the pharmaceutical composition of the invention is for use in a method of treating a disease treatable by inhibition and/or disruption of microtubule structure and function, wherein the disease treatable by inhibition and/or disruption of microtubule structure and function is: (a) cancer, optionally wherein the cancer is a haematological cancer or a solid tumour; and/or a proliferative disease; (b) an infectious disease; optionally wherein the infectious disease is a bacterial disease or a viral disease; optionally wherein the viral disease is an HIV infection; (c) a fungal disease; (d) an inflammatory disease; optionally wherein the inflammatory disease is: (i) an autoimmune disease; or (ii) gout; or (e) a fibrotic disease. Also described is a method of treating a disease treatable by inhibition and/or disruption of microtubule structure and function in a subject, the method comprising administering to a subject the pharmaceutical composition of the invention.

In one embodiment, the disease treatable by inhibition and/or disruption of microtubule structure and function is cancer, optionally wherein the cancer is a haematological cancer or a solid tumour.

In one embodiment, the disease treatable by inhibition and/or disruption of microtubule structure and function is an infectious disease. In one embodiment, the infectious disease is a parasitic disease. In one embodiment, the parasitic disease is a helminth disease. In one embodiment, the helminth disease is a filarial disease. In one embodiment, the parasitic disease is a protozoal disease. In one embodiment, the infectious disease is a bacterial disease or a viral disease. In one embodiment, the viral disease is an HIV infection.

In one embodiment, the disease treatable by inhibition and/or disruption of microtubule structure and function is a fungal disease.

In one embodiment, the disease treatable by inhibition and/or disruption of microtubule structure and function is a proliferative disease.

In one embodiment, the disease treatable by inhibition and/or disruption of microtubule structure and function is an inflammatory disease. In one embodiment, the inflammatory disease is an autoimmune disease. In one embodiment, the inflammatory disease is gout.

In one embodiment, the disease treatable by inhibition and/or disruption of microtubule structure and function is a fibrotic disease.

In one embodiment, the pharmaceutical composition of the invention is for use in a method of treating a disease treatable by impairment or reversion of epithelial-mesenchymal transition (EMT), wherein the disease treatable by impairment or reversion of EMT is: (a) cancer, optionally wherein the cancer is a solid tumour or a haematological cancer; and/or a proliferative disease; (b) an inflammatory disease; optionally wherein the inflammatory disease is: (i) an autoimmune disease; or (ii) gout; or (c) a fibrotic disease. Also described is a method of treating a disease treatable by impairment or reversion of EMT in a subject, the method comprising administering to a subject the pharmaceutical composition of the invention.

In one embodiment, the disease treatable by impairment or reversion of EMT is cancer, optionally wherein the cancer is a solid tumor or a haematological cancer.

In one embodiment, the disease treatable by impairment or reversion of EMT is a proliferative disease.

In one embodiment, the disease treatable by impairment or reversion of EMT is an inflammatory disease. In one embodiment, the inflammatory disease is an autoimmune disease. In one embodiment, the inflammatory disease is gout.

In one embodiment, the disease treatable by impairment or reversion of EMT is a fibrotic disease.

In one embodiment, the pharmaceutical composition of the invention is for use in a method of treating a neurodegenerative disease. Also described is a method of treating a neurodegenerative disease in a subject, the method comprising administering to the subject the pharmaceutical composition of the invention.

In one embodiment, the neurodegenerative disease is Alzheimer's disease.

In one embodiment, the pharmaceutical composition of the invention is for use in a method of treating liver disease. Also described is a method of treating liver disease in a subject, the method comprising administering to the subject the pharmaceutical composition of the invention.

In one embodiment, the pharmaceutical composition of the invention is for use in a method of treating a spinal cord injury. Also described is a method of treating a spinal cord injury in a subject, the method comprising administering to the subject the pharmaceutical composition of the invention.

In one embodiment, the pharmaceutical composition of the invention is for use in a method of treating myopathy. Also described is a method of treating myopathy in a subject, the method comprising administering to a subject the pharmaceutical composition of the invention.

In one embodiment, the method comprises administering flubendazole or a pharmaceutically acceptable salt, solvate, hydrate or *N*-oxide thereof to a subject concurrently, separately or sequentially with the moderate or strong CYP1A2 inhibitor.

In one embodiment, the cancer is a solid cancer, or a haematological cancer, optionally wherein the cancer is selected from Bronchial Tumors, Central Nervous System Cancer, Central Nervous System Embryonal Tumors, Carcinoma, Acute Myeloid Leukemia (AML), Carcinoid Tumor, Appendix Cancer, Astrocytomas, Chordoma, Atypical Teratoid/Rhabdoid Tumor, Sarcoma, Bladder Cancer, Thyroid Cancer, Primary Central Nervous System (CNS) Lymphoma, Extracranial Germ Cell Tumor, Esophageal Cancer, AIDS-Related Cancers, Hepatocellular (Liver) Cancer, Penile Cancer, Pleuropulmonary Blastoma, Gallbladder Cancer, Rhabdomyosarcoma, Waldenstrom Macroglobulinemia, Salivary Gland Cancer, Central Nervous System Germ Cell Tumors, Plasma Cell Neoplasm, Lip and Oral Cavity Cancer, Testicular Cancer, Extrahepatic Bile Duct Cancer, Ductal Carcinoma In Situ (DCIS), Nasopharyngeal Cancer, Nasal Cavity and Paranasal Sinus Cancer, Bone Cancer, Breast Cancer, Glioma, Hairy Cell Leukemia, Langerhans Cell Histiocytosis, Oral Cancer, Ependymoma, Cutaneous T-Cell Lymphoma, Gestational Trophoblastic Disease, Eye Cancer, Kaposi Sarcoma, Extragonadal Germ Cell Tumor, Gastric (Stomach) Cancer, Gastrointestinal Stromal Tumors (GIST), Papillomatosis, Small Intestine Cancer, Brain and Spinal Cord Tumors, Waldenstrom Macroglobulinemia, Pancreatic Cancer, Pharyngeal Cancer, Oropharyngeal Cancer, Paraganglioma, Nonmelanoma Skin Cancer, Myelodysplastic/Myeloproliferative Neoplasms, Squamous Cell Carcinoma, Malignant Fibrous Histiocytoma, Melanoma, Sezary Syndrome, Merkel Cell Carcinoma, Pituitary Tumor, Malignant Fibrous Histiocytoma of Bone and Osteosarcoma, Ovarian Cancer, Parathyroid Cancer, Skin Cancer, Mycosis Fungoides, Germ Cell Tumor, Fallopian Tube Cancer, Intraocular Melanoma, Leukemia, Pancreatic Neuroendocrine Tumors (Islet Cell Tumors), Endometrial Cancer, Lymphoma, Prostate Cancer, Renal Pelvis and Ureter Cancer, Osteosarcoma (Bone Cancer), Non-Hodgkin Lymphoma, Non-Small Cell Lung Cancer, Basal Cell Carcinoma, Laryngeal Cancer, Multiple Myeloma/Plasma Cell Neoplasm, Vaginal Cancer, Squamous Neck Cancer, Multiple Myeloma, Midline Tract Carcinoma Involving NUT Gene, Head and Neck Cancer, Heart Cancer, Intraocular (Eye), Renal Cell (Kidney) Cancer, Malignant Fibrous Histiocytoma of Bone, Liver Cancer, Rectal Cancer, Colon Cancer, Malignant Mesothelioma, Low Malignant Potential Tumor, Mouth Cancer, Soft Tissue Sarcoma, Hypopharyngeal Cancer, Wilms Tumor, Epithelial Cancer, Ewing Sarcoma Family of Tumors, Acute Lymphoblastic Leukemia (ALL), Retinoblastoma, Hodgkin Lymphoma, Brain Tumor/Cancer, Esthesioneuroblastoma, Embryonal Tumors, Cervical Cancer, Chronic Myeloproliferative Neoplasms, Pancreatic Neuroendocrine Tumors, Ureter and Renal Pelvis Cancer, Anal Cancer, Urethral Cancer, Brain Stem Cancer, Vulvar Cancer, Chronic Lymphocytic Leukemia (CLL), Uterine Sarcoma, Stomach (Gastric) Cancer, Brain Stem Glioma, Multiple Endocrine Neoplasia Syndromes, Myelodysplastic Syndromes, Craniopharyngioma, Small Cell Lung Cancer, Lip and Oral Cavity Cancer, Cutaneous T-Cell Lymphoma, Neuroblastoma, Acute Lymphoblastic Leukemia (ALL), Langerhans Cell Histiocytosis, Breast Cancer, Gastrointestinal Carcinoid Tumor, Paranasal Sinus and Nasal Cavity Cancer, Pheochromocytoma, Metastatic Squamous Neck Cancer with Occult Primary, Male Breast Cancer, Kidney (Renal) Cancer, Lung Cancer, Islet Cell Tumors, Extrahepatic Bile Duct Cancer, Endometrial Uterine Cancer, Chronic Myeloproliferative Neoplasms, Transitional Cell Cancer of the Renal Pelvis and Ureter, Thymoma and Thymic Carcinoma, Throat Cancer, Ewing Sarcoma, Chronic Myelogenous Leukemia (CML), Colorectal Cancer, Colon Cancer, Cardiac (Heart) Tumors, Burkitt Lymphoma, Carcinoma of Unknown Primary, Central Nervous System Atypical Teratoid/Rhabdoid Tumor, Childhood Cancers, and Non-Hodgkin Lymphoma, Adrenocortical Carcinoma, Adrenocortical Adenocarcinoma, Adrenocortical Adenoma, Kidney (Renal) Cancer, and P-gp expressing Multidrug Resistant Tumour.

In one embodiment, the fungal disease or parasitic disease is selected from helminth diseases and protozoal diseases, optionally selected from filarial diseases, onchocerciasis, hookworm, echinococcosis diseases, ascariasis, and enterobiasis, *Acanthocephalans, Plasmodium* spp., African trypanosomes, *Trypanosoma cruzi, Leishmania* spp., *Giardia* spp., *Trichomonas vaginalis, Entamoeba histolytica, Encephalitozoon* spp., *Acanthamoeba castellani,* and *Enterocytozoon bieneusi,* and fungal diseases, including *Cryptococcus neoformans* and other *Cryptococcus* species.

In one embodiment, the method comprises administering the composition to a human.

In one embodiment, the method comprises administering the pharmaceutical composition orally, intravenously, intramuscularly or subcutaneously.

Also described is a method for improving pharmacokinetics of flubendazole, the method comprising combining flubendazole or a pharmaceutically acceptable salt, solvate, hydrate, N-oxide, or prodrug thereof with a moderate or strong CYP1A2 inhibitor.

The present invention also provides a pharmaceutical composition comprising flubendazole or a pharmaceutically acceptable salt, solvate, hydrate or N-oxide thereof and a strong or moderate CYP1A2 inhibitor for use in a method of treatment of a human or animal subject by therapy.

The present invention also provides flubendazole or a pharmaceutically acceptable salt, solvate, hydrate or N-oxide thereof for use in a method of treatment of a human or animal subject by therapy, wherein the method of treatment comprises the concurrent, separate or sequential administration of a strong or moderate CYP1A2 inhibitor.

The present invention also provides a strong or moderate CYP1A2 inhibitor for use in a method of treatment of a human or animal subject by therapy, wherein the method of treatment comprises the concurrent, separate or sequential administration of a pharmaceutical composition comprising flubendazole.

Also described is use of flubendazole or a pharmaceutically acceptable salt, solvate, hydrate, *N*-oxide, or prodrug thereof and a strong or moderate CYP1A2 inhibitor in the manufacture of a medicament for the treatment of a human or animal subject by therapy.

Also described is a method of treatment of a human or animal subject by therapy comprising concurrent, separate or sequential administration of flubendazole or a pharmaceutically acceptable salt, solvate, hydrate, *N*-oxide, or prodrug thereof and a strong or moderate CYP1A2 inhibitor.

Also described is a method of producing an improved flubendazole formulation, the method comprising combining flubendazole with a strong or moderate CYP1A2 inhibitor.

It has surprisingly been discovered that improved (enhanced) PK of flubendazole, achieved by reducing its intrinsic (hepatic) clearance and the formation of metabolites, is achieved by inhibition of flubendazole metabolism by CYP1A2. The invention achieves improved PK of flubendazole by combining it with a strong or moderate CYP1A2 inhibitor. The present invention provides a means of improving the PK of flubendazole, resulting in improved, including prolonged, *in vivo* therapeutic activity.

## Brief description of the drawings

Figure 1 shows the stability of flubendazole in the presence of CYP1A2 recombinant P450 isoform of Example 1.
Figure 2 shows the stability of flubendazole in the presence of CYP2B6 recombinant P450 isoform of Example 1.
Figure 3 shows the stability of flubendazole in the presence of CYP2C8 recombinant P450 isoform of Example 1.
Figure 4 shows the stability of flubendazole in the presence of CYP2C9 recombinant P450 isoform of Example 1.
Figure 5 shows the stability of flubendazole in the presence of CYP2C19 recombinant P450 isoform of Example 1.
Figure 6 shows the stability of flubendazole in the presence of CYP2D6 recombinant P450 isoform of Example 1.

Figure 7 shows the stability of flubendazole in the presence of CYP3A4 recombinant P450 isoform of Example 1.

Figure 8 shows the stability of flubendazole in human liver microsomes in the absence of a CYP1A2 inhibitor of Example 2.

Figures 9 to 11 show the stability of flubendazole, in human liver microsomes in the presence of 3 μM, 10 μM, and 30 μM furafylline, respectively (strong CYP1A2 inhibitor) of Example 2.

Figure 12 shows the stability of flubendazole in assay 1, in human hepatocytes of Example 3.

Figure 13 shows the stability of flubendazole in assay 2, in human hepatocytes in the absence of a CYP1A2 inhibitor of Example 3.

Figures 14 to 17 show the stability of flubendazole in assay 2, in human hepatocytes in the presence of 1 μM, 3 μM, 10 μM, and 30 μM furafylline, respectively (strong CYP1A2 inhibitor) of Example 3.

Figures 18 to 21 show the stability of flubendazole in assay 2, in human hepatocytes in the presence of 3 μM, 10 μM, 30 μM, and 100 μM thiabendazole, respectively (strong CYP1A2 inhibitor) of Example 3.

Figures 22 to 25 show the stability of flubendazole in assay 2, in human hepatocytes in the presence of 3 μM, 10 μM, 30 μM, and 100 μM fluvoxamine, respectively (strong CYP1A2 inhibitor) of Example 3.

Figure 26 shows the stability of flubendazole in assay 3, in human hepatocytes in the absence of a CYP1A2 inhibitor of Example 3.

Figures 27 to 29 show the stability of flubendazole in assay 3, in human hepatocytes in the presence of 10 μM, 100 μM, and 300 μM cimetidine, respectively (moderate CYP1A2 inhibitor) of Example 3.

Figures 30 to 33 show the stability of flubendazole in assay 3, in human hepatocytes in the presence of 10 μM, 30 μM, 100 μM, and 300 μM mexiletine, respectively (strong CYP1A2 inhibitor) of Example 3.

## Detailed description of the invention

Certain features of the invention are described in more detail below.

In the following description, the term "active ingredient" or "active ingredients" of the compositions of the invention refers to flubendazole and the strong or moderate CYP1A2 inhibitor as indicated in the claims.

The active ingredients of the compositions of the invention may contain asymmetric or chiral centres, and therefore exist in different stereoisomeric forms. Unless otherwise specified, it is intended that all stereoisomeric forms of the active ingredients, including but not limited to, diastereomers, enantiomers and atropisomers, as well as mixtures thereof such as racemic mixtures, may form part of the compositions of the invention. Unless otherwise specified, active ingredients, containing one or more chiral centre, may be used in enantiomerically or diastereoisomerically pure form, or in the form of a mixture of isomers. If an active ingredient is referred to by name (e.g. by INN or drug trade name), and if that name is generally recognised in the pharmaceutical art as referring to one specific diastereomer, enantiomer or atropisomer, then the active ingredient referred to is typically that specific diastereomer, enantiomer or atropisomer.

Active ingredients of the compositions of the invention may exist in different tautomeric forms, and unless otherwise specified all such forms are embraced within the scope of the invention. The term "tautomer" or "tautomeric form" refers to structural isomers of different energies which are interconvertible *via* a low energy barrier. For example, proton tautomers (also known as prototropic tautomers) include interconversions *via* migration of a proton, such as keto-enol tautomerizations. Valence tautomers include interconversions by reorganization of some of the bonding electrons.

Active ingredients of the compositions of the invention in the solid state may exist in different amorphous or crystalline forms (e.g. polymorphic forms), and all such forms are embraced within the scope of the invention.

As used herein, a pharmaceutically acceptable salt of an active ingredient refers to a salt of an active ingredient which may be prepared by combining an active ingredient with a base or acid which is acceptable for administration to a human or animal subject.

As used herein, a pharmaceutically acceptable solvate of an active ingredient refers to a solid state complex comprising an active ingredient and molecules of a solvent which is acceptable for administration to a human or animal subject. The active ingredient may be dissolved in the solvent to form a solution or it may be suspended in the solvent to form a suspension. The solution or suspension may be for use as a medicament.

As used herein, a pharmaceutically acceptable hydrate of an active ingredient refers to a solvate in which the solvent is water.

As used herein, a pharmaceutically acceptable *N*-oxide of an active ingredient refers to an oxide of an active ingredient, which active ingredient in its unoxidised state comprises a basic amine or imine.

As used herein, a pharmaceutically acceptable prodrug of an active ingredient refers to a pharmaceutically acceptable substance which may be converted into an active ingredient *in vivo* when administered to a human or animal patient. A prodrug of an active ingredient may be formed by masking a moiety of the active ingredient with a pro-moiety. Prodrugs are described in further detail in Pro-drugs as Novel Delivery Systems, Vol. 14, ACS Symposium Series (T. Higuchi and W. Stella) and Bioreversible Carriers in Drug Design, Pergamon Press, 1987 (ed. E. B. Roche, American Pharmaceutical Association).

As used herein, a pharmaceutically acceptable active metabolite refers to a pharmaceutically acceptable substance which is formed *in vivo* when an ingredient is administered to a human or animal subject, and which has desired pharmacological activity.

As used herein, the term "benzimidazole compound" refers to a pharmacologically-active compound comprising a benzimidazole moiety. Flubendazole exerts its main therapeutic activity by inhibiting and/or disrupting microtubule structure and function in cells, with effects on tubulin polymerization. The therapeutic effects of flubendazole include direct effects of inhibition and/or disruption of microtubule structure and function, as well as downstream effects that result from inhibition and/or disruption of microtubule structure and function. Thus, the compositions of the invention may be for use in treating disease treatable by inhibition and/or disruption of microtubule structure and function, including disease treatable by other pharmacological actions of flubendazole including downstream effects that result from inhibition and/or disruption of microtubule structure and function. In one embodiment, the compositions of the invention are for use in treating cancers/tumour diseases (including solid tumours and haematological cancers), parasitic diseases, fungal diseases, non-cancer proliferative diseases, inflammatory diseases including autoimmune inflammatory diseases and gout, and fibrotic diseases (e.g. Cronstein and Terkeltaub, 2006; Kalluri and Weinberg, 2009; Dinarello, 2010; Hou et al., 2015; Wildenberg et al., 2017; Kralova et al., 2018; Yang et al., 2020).

Flubendazole, including pharmaceutically acceptable salts, solvates, hydrates or *N*-oxides thereof, is used in the compositions of the invention.

The Inventor has discovered that flubendazole is metabolised by CYP1A2 using recombinant human P450 enzymes, human liver microsome studies, and human hepatocyte studies. "Metabolised by CYP1A2" means that CYP1A2 is responsible at least in part for the metabolism of flubendazole *in vivo.* Flubendazole gave a surprisingly very short half-life value of 2.34 min in the presence of CYP1A2 recombinant enzyme.

The skilled person will appreciate that the pharmacokinetic improvements achieved by the invention also apply to mechanisms of action that do not involve inhibition and/or disruption of microtubule structure and/or function. In some embodiments, flubendazole has activity *via* one or more mechanisms including but not limited to the following:

- antifungal activity (Nixon et al., 2018)
- β-tubulin binding (Nixon et al., 2018; Sjoberg et al., 2019)
- inhibition of tubulin polymerization, resulting in cell death (Spagnuolo et al., 2010; Michaelis et al., 2015)
- dysregulation of cuticle-associated gene expression (O'Neill et al., 2016)
- disruption of microtubule architecture (Spagnuolo et al., 2010; Oh et al., 2018)
- arrest/inhibition of cell proliferation (Spagnuolo et al., 2010; Oh et al., 2018)
- anti-tumour action by triggering cell apoptosis, inhibition of microtubule function, and HER2 signaling (Zhou et al., 2018; Tao et al., 2019)
- p53-mediated apoptosis (Michaelis et al., 2015)
- anti-tumour action by suppressing the NF-κB signaling pathway (Tao et al., 2019)
- anti-tumour action by blocking STAT3 signaling and activating autophagy (including targeting ATG4B and EVA1A) (Chauhan et al., 2015; Panda et al., 2019; Agrotis and Ketteler., 2019; Zhen et al., 2020; Lin et al., 2019)
- induction of apoptosis, accompanied by G2/M phase accumulation, caspase-3/-7 activation and the dysregulation of STAT3 activation in triple-negative breast cancer cells, with inhibition of tumour growth, angiogenesis and lung and liver metastasis, coinciding with decreased MMP-2 and MMP-9 levels in circulating blood (Oh et al., 2018)
- suppression of cancer stem-like cells, EMT reversion (Hou et al., 2015)
- inhibition of activation of glial fibrillary acidic protein, reduction in cyclin B1 expression and Bruton tyrosine kinase activation, B cell activation/proliferation and inflammation, and reduced B cell autoimmune response (Yu et al., 2019)

Flubendazole is commercially available or can be prepared by known methods or by analogy with known methods. For example, flubendazole is available from commercial suppliers including Sigma-Aldrich®.

*CYP1A2 inhibitor*

The CYP1A2 inhibitor of the compositions of the invention is described in more detail below.

The CYP1A2 inhibitor according to the invention is a moderate CYP1A2 inhibitor or a strong CYP1A2 inhibitor. In some embodiments, the CYP1A2 inhibitor is a moderate CYP1A2 inhibitor. In some embodiments, the CYP1A2 inhibitor is a strong CYP1A2 inhibitor. Preferably, the CYP1A2 inhibitor is a strong CYP1A2 inhibitor.

In studies now performed and summarised hereinbelow, all inhibitors of CYP1A2 tested have surprisingly shown moderate or strong inhibition of the metabolism of flubendazole in human hepatocytes *ex vivo.*

As used herein, a CYP1A2 inhibitor is a substance which is capable of inhibiting the metabolism, i.e. the intrinsic clearance (CLint), of flubendazole by CYP1A2. Inhibited metabolism of flubendazole increases the *in vivo* area under the curve (AUC) of flubendazole, and increases the *in vivo* half-life of flubendazole.

As used herein, a moderate CYP1A2 inhibitor is a CYP1A2 inhibitor which inhibits intrinsic clearance of flubendazole in human hepatocytes *ex vivo* by ≥two-fold to <five-fold compared to intrinsic clearance of flubendazole in human hepatocytes *ex vivo* in the absence of a CYP1A2 inhibitor. A moderate CYP1A2 inhibitor typically increases the AUC *in vivo* of flubendazole by two-fold or more and less than five-fold compared to the AUC *in vivo* of flubendazole in the absence of a CYP1A2 inhibitor. Examples of moderate CYP1A2 inhibitors according to the invention include cimetidine and methoxsalen.

As used herein, a strong CYP1A2 inhibitor is a CYP1A2 inhibitor which inhibits intrinsic clearance ($CL_{int}$) of flubendazole in human hepatocytes *ex vivo* by ≥five-fold compared to intrinsic clearance of flubendazole in human hepatocytes *ex vivo* in the absence of a CYP1A2 inhibitor. A strong CYP1A2 inhibitor typically increases the AUC *in vivo* of flubendazole by five-fold or more compared to the AUC *in vivo* of flubendazole in the absence of a CYP1A2 inhibitor. Examples of strong CYP1A2 inhibitors according to the invention include xanthine derivatives such as furafylline and 8-phenyltheophylline, and ciprofloxacin, enoxacin, fluvoxamine, zafirlukast, mexiletine, and thiabendazole.

The moderate or strong CYP1A2 inhibitor of the invention extends the *in vivo* half-life of flubendazole by more than or equal to 2, 3, 4, 6, 8, 10, 12, 14, 16, 18 or 20 times the *in vivo* half-life of flubendazole in the absence of the moderate or strong CYP1A2 inhibitor.

The moderate or strong CYP1A2 inhibitor of the invention may be selected from furafylline, fluvoxamine, thiabendazole, mexiletine, 8-phenyltheophylline, ciprofloxacin, enoxacin, zafirlukast, cimetidine, or methoxsalen, or a pharmaceutically acceptable salt, solvate, hydrate or *N*-oxide thereof.

In one embodiment, the strong CYP1A2 inhibitor according to the invention is furafylline. In one embodiment, the strong CYP1A2 inhibitor according to the invention is fluvoxamine. In one embodiment, the strong CYP1A2 inhibitor according to the invention is thiabendazole. In one embodiment, the strong CYP1A2 inhibitor according to the invention is mexiletine. In one embodiment, the strong CYP1A2 inhibitor according to the invention is 8-phenyltheophylline. In one embodiment, the strong CYP1A2 inhibitor according to the invention is ciprofloxacin. In one embodiment, the strong CYP1A2 inhibitor according to the invention is enoxacin. In one embodiment, the strong CYP1A2 inhibitor according to the invention is zafirlukast. In one embodiment, the moderate CYP1A2 inhibitor of the invention is cimetidine. In one embodiment, the moderate CYP1A2 inhibitor of the invention is methoxsalen.

As shown hereinbelow, mexiletine, thiabendazole, furafylline and fluvoxamine were surprisingly found to be strong CYP1A2 inhibitors of flubendazole metabolism, while cimetidine was surprisingly found to be a moderate CYP1A2 inhibitor of flubendazole metabolism. These data demonstrate that flubendazole is a surprisingly sensitive substrate for these CYP1A2 inhibitors in the studies performed.

Thiabendazole is a preferred strong CYP1A2 inhibitor according to the present invention. Thiabendazole has been approved as an anthelminthic drug for human use and is currently used as a veterinary (animal use) anthelminthic drug. Thiabendazole has shown anti-tumour activity *in vivo,* and has vascular disrupting properties (Cha et al., 2012). Thiabendazole has an inhibition constant (Ki) value for CYP1A2 inhibition of 1.54 μM and an IC50 value for CYP1A2 inhibition of 0.83 μM (Bapiro et al., 2005; Thelingwani et al., 2009).

Thiabendazole has now been shown to be a strong inhibitor of flubendazole metabolism, based on studies reported in the examples hereinbelow, in accordance with the definition of a strong CYP1A2 inhibitor as provided above. Furthermore, like flubendazole, thiabendazole is a benzimidazole drug with antiproliferative including anticancer activity and antiparasitic activity. Therefore, potential pharmacodynamic synergy (antitumour and/or antiproliferative efficacy) as well as pharmacokinetic synergy (CYP1A2 inhibition of flubendazole metabolism) may be observed with the flubendazole-thiabendazole combination of the present invention.

Furafylline is a preferred strong CYP1A2 inhibitor according to the present invention. Furafylline has now been used as a strong CYP1A2 inhibitor of flubendazole metabolism in human hepatocytes and human liver microsomes *ex vivo* using the CYP1A2 inhibitor, cimetidine, as comparator in human hepatocytes. As a surprisingly sensitive substrate of CYP1A2, flubendazole intrinsic clearance was moderately inhibited by cimetidine, and strongly inhibited by furafylline.

Furafylline is a methylxanthine derivative in clinical development in the mid-1980s, which was originally intended to be developed for the treatment of respiratory diseases. Furafylline has previously been shown to strongly inhibit metabolism of caffeine, a sensitive CYP1A2 substrate, with a 7-10 fold increase in elimination half-life of caffeine in humans from 5-7 hours to 50 hours (Tarrus et al., 1987). Furafylline is a non-competitive inhibitor of CYP1A2 inhibition and has IC50 values of 0.027 μM and 0.07 μM in human liver microsomes for CYP1A2 inhibition of phenacetin-O-deethylase activity (Sesardic et al., 1990; Obach et al., 2007). Furafylline is a potent and selective time-dependent inhibitor of CYP1A2 and strong CYP1A2 inhibitor, as shown by its strong inhibition of metabolism of caffeine, a sensitive CYP1A2 substrate. Furafylline has now been shown to be a strong inhibitor of flubendazole metabolism, based on human hepatocyte and human liver microsome *ex vivo* studies reported in the examples hereinbelow, where strong concentration-related inhibition was observed, in accordance with the definition of a strong CYP1A2 inhibitor as provided above.

Fluvoxamine is also a preferred strong CYP1A2 inhibitor according to the present invention. Fluvoxamine has now been shown to be a strong inhibitor of flubendazole metabolism, based on studies reported in the examples hereinbelow, in accordance with the definition of a strong CYP1A2 inhibitor as provided above. Fluvoxamine has previously

been shown to potently inhibit CYP1A2 phenacetin O-deethylation using human liver microsomes with IC50 values for CYP1A2 inhibition of 0.035 μM and 0.029 μM and Ki of 0.011 μM (Obach et al., 2006; and Karjalainen et al., 2008). According to drug labelling, fluvoxamine is a strong CYP1A2 inhibitor *in vitro* and *in vivo*.

Furthermore, fluvoxamine has shown anti-tumour activity against glioblastoma by disrupting actin polymerization leading to suppression of glioblastoma migration and invasion (Hayashi et al., 2016). Therefore, potential pharmacodynamic synergy (antitumour and/or antiproliferative efficacy) as well as pharmacokinetic synergy (CYP1A2 inhibition of flubendazole metabolism) may be observed with the flubendazole-fluvoxamine combination of the present invention.

Mexiletine is also a preferred strong CYP1A2 inhibitor according to the present invention.

Any compound, which is capable in this CYP1A2 metabolic pathway of effecting an inhibition of intrinsic clearance ($CL_{int}$) in human hepatocytes *ex vivo* of flubendazole (hereinbelow established as a CYP1A2 substrate) of ≥two-fold (e.g. ≥five-fold), or an increase in the AUC of flubendazole *in vivo* by two-fold or more (e.g. five-fold or more), is considered to be a moderate or strong CYP1A2 inhibitor according to the present invention. The moderate or strong CYP1A2 inhibitor may, for example, be a compound which is known to have moderate or strong CYP1A2 inhibitory activity. However, the compositions of the invention are not limited thereto and the moderate or strong CYP1A2 inhibitor may be a known substance which is not currently known to be a moderate or strong CYP1A2 inhibitor, or a novel compound which is a moderate or strong CYP1A2 inhibitor.

As shown by the data presented herein, cimetidine (previously described as a weak CYP1A2 inhibitor) has unexpectedly been shown to act as a moderate inhibitor of flubendazole metabolism by CYP1A2, demonstrating that flubendazole is a surprisingly sensitive CYP1A2 substrate.

Weak, moderate and strong CYP1A2 inhibitors are discussed in *Drug Interactions & Labelling - Drug Development and Drug Interactions: Table of Substrates, Inhibitors and Inducers,* published by the United States Food and Drug Administration (US FDA) and in *Guideline CPMP/EWP/560/95 /Rev. 1 Corr.* 2, Committee for Human Medicinal Products (CHMP), published by the European Medicines Agency (EMA).

As used herein, a weak CYP1A2 inhibitor is a substance which inhibits intrinsic clearance ($CL_{int}$) in human hepatocytes *ex vivo* of flubendazole by <two-fold compared to intrinsic clearance in human hepatocytes *ex vivo* of flubendazole in the absence of a CYP1A2 inhibitor, or increases the AUC plasma value of flubendazole *in vivo* by less than two-fold compared to the AUC *in vivo* of flubendazole in the absence of a CYP1A2 inhibitor. It follows from the definitions of strong, moderate and weak CYP1A2 inhibitors above that the strong or moderate CYP1A2 inhibitor in the compositions of the invention is not a weak CYP1A2 inhibitor, or a salt, solvate, hydrate, *N*-oxide, prodrug or active metabolite thereof. Preferred CYP1A2 inhibitors according to the invention are strong CYP1A2 inhibitors selected from furafylline, fluvoxamine, thiabendazole, mexiletine, 8-phenyltheophylline, ciprofloxacin, enoxacin, and zafirlukast, and pharmaceutically acceptable salts, solvates, hydrates or N-oxides thereof.

Strong or moderate CYP1A2 inhibitors are commercially available, or can be prepared by known methods or by analogy with known methods. For example, furafylline, thiabendazole, fluvoxamine and mexiletine are available from commercial suppliers including Sigma-Aldrich®.

*Uses in methods of treatment*

Methods of treatment by therapy of a human or animal subject are discussed in more detail below. As used herein the term "method of treatment" or "methods of treatment" refers to the treatment by therapy of a human or animal subject.

By combining flubendazole with a strong or moderate CYP1A2 inhibitor, the compositions of the invention improve the PK of flubendazole. Advantages provided by the invention include improved predictability of the systemic bioavailability, prolonged exposure, reduced variability in systemic bioavailability of flubendazole between individual subjects (patients) and on an intra-subject basis, and hence improved efficacy / therapeutic activity of flubendazole. The invention also achieves improved therapeutic safety by reducing the formation of toxic metabolites.

Thus, low systemic availability of flubendazole, as seen in humans, may be due to metabolism (in particular by CYP1A2 metabolism during first pass hepatic metabolism) to a much greater extent than previously thought. Systemic bioavailability and PK of flubendazole is advantageously improved by administration with a strong or moderate CYP1A2 inhibitor, according to the invention. Inhibition of the metabolism of flubendazole by use of a moderate or strong CYP1A2 inhibitor, and not by use of a weak CYP1A2 inhibitor, advantageously reduces intra-subject and inter-subject (patient) variability in systemic therapeutic concentrations of flubendazole as well as reduces formation of toxic metabolites. Intra-subj ect variation can occur by exposure to dietary and environmental compounds including for example cigarette smoke and oral contraceptive steroids which induce CYP1A2 (Zhou et al., 2009). Inter-subject variation in CYP1A2 enzyme activity occurs as a result of environmental factors as well as genetic polymorphism (Kapelyukh et al., 2019).

The compositions of the invention can be used to enable flubendazole to achieve the desired mechanisms of action systemically, as well as reduce formation of toxic metabolites and inactive metabolites systemically. Accordingly, the method of treatment may comprise, systemically, one or more mechanisms including but not limited to the following:

- antifungal activity (Nixon et al., 2018)
- β-tubulin binding (Nixon et al., 2018; Sjoberg et al., 2019)
- inhibition of tubulin polymerization, resulting in cell death (Spagnuolo et al., 2010; Michaelis et al., 2015)
- dysregulation of cuticle-associated gene expression (O'Neill et al., 2016)
- disruption of microtubule architecture (Spagnuolo et al., 2010; Oh et al., 2018)
- arrest/inhibition of cell proliferation (Spagnuolo et al., 2010; Oh et al., 2018)
- anti-tumour action by triggering cell apoptosis, inhibition of microtubule function, and HER2 signaling (Zhou et al., 2018; Tao et al., 2019)
- p53-mediated apoptosis (Michaelis et al., 2015)
- anti-tumour action by suppressing the NF-κB signaling pathway (Tao et al., 2019)
- anti-tumour action by blocking STAT3 signaling and activating autophagy (including targeting ATG4B and EVA1A) (Chauhan et al., 2015; Panda et al., 2019; Agrotis and Ketteler., 2019; Zhen et al., 2020; Lin et al., 2019)
- induction of apoptosis, accompanied by G2/M phase accumulation, caspase-3/-7 activation and the dysregulation of STAT3 activation in triple-negative breast cancer cells, with inhibition of tumour growth, angiogenesis and lung and liver metastasis, coinciding with decreased MMP-2 and MMP-9 levels in circulating blood (Oh et al., 2018)
- suppression of cancer stem-like cells, EMT reversion (Hou et al., 2015)
- inhibition of activation of glial fibrillary acidic protein, reduction in cyclin B1 expression and Bruton tyrosine kinase activation, B cell activation/proliferation and inflammation, and reduced B cell autoimmune response (Yu et al., 2019)

In some embodiments, the pharmaceutical composition of the invention is for use in medicine.

In some embodiments, the pharmaceutical composition of the invention is for use in a method of treating a disease treatable by flubendazole. Also described is a method of treating or preventing a disease treatable by flubendazole, the method comprising administering a pharmaceutical composition comprising flubendazole and a moderate or strong CYP1A2 inhibitor to the subject.

In some embodiments, the pharmaceutical composition of the invention is for use in a method of treating a disease treatable by inhibition and/or disruption of microtubule structure and function, wherein the disease treatable by inhibition and/or disruption of microtubule structure and function is: (a) cancer, optionally wherein the cancer is a haematological cancer or a solid tumour; and/or a proliferative disease; (b) an infectious disease; optionally wherein the infectious disease is a bacterial disease or a viral disease; optionally wherein the viral disease is an HIV infection; (c) a fungal disease; (d) an inflammatory disease; optionally wherein the inflammatory disease is: (i) an autoimmune disease; or (ii) gout; or (e) a fibrotic disease.

Also described is a method of treating a disease treatable by inhibition and/or disruption of microtubule structure and function in a subject, the method comprising administering a pharmaceutical composition comprising flubendazole and a moderate or strong CYP1A2 inhibitor to the subject.

In one embodiment, the disease treatable by inhibition and/or disruption of microtubule structure and function is cancer. In one embodiment, the cancer is a haematological cancer or a solid tumour.

In one embodiment, the disease treatable by inhibition and/or disruption of microtubule structure and function is a parasitic disease or a fungal disease.

In one embodiment, the disease treatable by inhibition and/or disruption of microtubule structure and function is a spinal cord injury.

In one embodiment, flubendazole binds to tubulin, optionally β-tubulin.

In one embodiment, the method comprises inhibition of tubulin polymerisation. In one embodiment, the method comprises promotion of tubulin depolymerisation. In one embodiment, the method comprises disruption of microtubule architecture and/or function. In one embodiment, the method comprises inhibition of angiogenesis.

In some embodiments, the pharmaceutical composition of the invention is for use in a method of treating cancer. In one embodiment, the composition is for use in treating a haematological cancer, such as leukaemia, lymphoma or myeloma. In one embodiment, the method of treating cancer is a method of treating a solid tumour. Also described is a method of treating cancer in a subject, the method comprising administering a pharmaceutical composition comprising flubendazole and a moderate or strong CYP1A2 inhibitor to the subject. The method of treating cancer may be a method of treating a haematological cancer, such as leukaemia, lymphoma or myeloma. The method of treating cancer may be a method of treating a solid tumour.

The method may comprise arresting or inhibiting cell proliferation. The method may comprise inducing apoptosis, optionally p53-mediated apoptosis. The method may comprise inhibiting cell migration. The method may comprise inhibiting tumour growth. The method may comprise suppressing the NF-κB signaling pathway. The method may comprise activating autophagy, optionally wherein said method comprise blocking the STAT3 signaling pathway. The method may comprise activating autophagy by targeting ATG4B and EVA1A.

In some embodiments, the pharmaceutical composition of the invention is for use in a method of treating a neurodegenerative disease. In one embodiment, the composition is for use in treating Alzheimer's disease. Also described

is the composition for use in treating an aging-associated disorder or condition. Also described is a method of treating a neurodegenerative disease in a subject, the method comprising administering a pharmaceutical composition comprising flubendazole and a moderate or strong CYP1A2 inhibitor to the subject. The neurodegenerative disease may be Alzheimer's disease or an aging associated disorder or condition.

In some embodiments, the pharmaceutical composition of the invention is for use in a method of treating a disease treatable by impairment or reversion of epithelial-mesenchymal transition (EMT) wherein the disease treatable by impairment or reversion of EMT is: (a) cancer, optionally wherein the cancer is a solid tumour or a haematological cancer; and/or a proliferative disease; (b) an inflammatory disease; optionally wherein the inflammatory disease is: (i) an autoimmune disease; or (ii) gout; or (c) a fibrotic disease. Also described is a method of treating a disease treatable by impairment or reversion of EMT in a subject, the method comprising administering a pharmaceutical composition comprising flubendazole and a moderate or strong CYP1A2 inhibitor to the subject.

In one embodiment, the disease treatable by impairment or reversion of EMT is cancer. In one embodiment, the disease treatable by impairment or reversion of EMT is a solid tumour or haematological cancer. In one embodiment, the disease treatable by impairment or reversion of EMT is a tumour. In one embodiment, the disease treatable by impairment or reversion of EMT is a non-cancer proliferative disease. In one embodiment, the disease treatable by impairment or reversion of EMT is an inflammatory disease, such as an autoimmune disease or gout. In one embodiment, the disease treatable by impairment or reversion of EMT is a fibrotic disease.

In some embodiments, the pharmaceutical composition of the invention is for use in a method of treating liver disease. Also described is a method of treating liver disease in a subject, the method comprising administering a pharmaceutical composition comprising flubendazole and a moderate or strong CYP1A2 inhibitor to the subject.

In some embodiments, the pharmaceutical composition of the invention is for use in a method of treating myopathy. Also described is a method of treating myopathy in a subject, the method comprising administering a pharmaceutical composition comprising flubendazole and a moderate or strong CYP1A2 inhibitor to the subject.

In some embodiments, the pharmaceutical composition of the invention is for use in a method of treating spinal cord injury. Also described is a method of treating spinal cord injury in a subject, the method comprising administering a pharmaceutical composition comprising flubendazole and a moderate or strong CYP1A2 inhibitor to the subject. Flubendazole may be used to treat spinal cord injury by inhibition of activation of glial fibrillary acidic protein, reduction in cyclin B1 expression and Bruton tyrosine kinase activation, B cell activation/proliferation and inflammation, and reduced B cell autoimmune response (Yu et al., 2019).

In some embodiments, the pharmaceutical composition of the invention is for use in a method of treating or preventing an infectious disease, such as a bacterial disease or a viral disease, including HIV infection. Also described is a method of treating or preventing an infectious disease, such as a bacterial disease or a viral disease, including HIV infection in a subject, the method comprising administering a pharmaceutical composition comprising flubendazole and a moderate or strong CYP1A2 inhibitor to the subject.

In some embodiments, the pharmaceutical composition of the invention is for use in a method of treating or preventing an infectious disease, such as a parasitic disease. Also described is a method of treating or preventing an infectious disease, such as a parasitic disease in a subject, the method comprising administering a pharmaceutical composition comprising flubendazole and a moderate or strong CYP1A2 inhibitor to the subject.

In one embodiment, the parasitic disease is a helminth disease. The helminth disease may be caused by nematodes, cestodes or trematodes. In one embodiment, the parasitic disease is filarial disease. In one embodiment, the parasitic disease is onchocerciasis. In one embodiment, the parasitic disease is hookworm disease. In one embodiment, the parasitic disease is an echinococcosis disease. In one embodiment, the parasitic disease is ascariasis. In one embodiment, the parasitic disease is enterobiasis. In one embodiment, the parasitic disease is trichinosis. In one embodiment, the parasitic disease is acanthocephaliasis.

In one embodiment, that parasitic disease is a protozoal disease. In one embodiment, the protozoal disease is caused by *Plasmodium* spp., African trypanosomes, *Trypanosoma cruzi, Leishmania* spp., *Giardia* spp., *Trichomonas vaginalis, Entamoeba histolytica, Encephalitozoon* spp., *Acanthamoeba castellani,* or *Enterocytozoon bieneusi.*

In one embodiment, the disease is a fungal disease. In one embodiment, the fungal disease is caused by *Cryptococcus neoformans* or other *Cryptococcus* species.

The pharmaceutical composition of the invention may be for use in a method of treating a disease which is susceptible to amelioration by action at a biological target at which flubendazole is active. Also described is a method of treatment which may be a method of treating a disease which is susceptible to amelioration by action at a biological target at which flubendazole is active. Diseases which are susceptible to amelioration by action at a biological target at which flubendazole is active include, but are not limited to haematological cancers, solid tumours including cancer, other non-cancer proliferative diseases, inflammatory diseases including autoimmune inflammatory diseases and gout, fibrotic diseases, parasitic diseases including helminth diseases (nematodes, cestodes, trematodes) e.g. filarial diseases, onchocerciasis, hookworm, echinococcosis diseases, ascariasis, enterobiasis, and *Trichinella spiralis,* and *Acanthocephalans* and protozoal diseases e.g. *Plasmodium* spp., African trypanosomes, *Trypanosoma cruzi, Leishmania* spp., *Giardia*

spp., *Trichomonas vaginalis, Entamoeba histolytica, Encephalitozoon* spp., *Acanthamoeba castellani, Enterocytozoon bieneusi,* and fungal diseases , including *Cryptococcus neoformans* and other *Cryptococcus* species, bacterial diseases, and viral diseases.

Flubendazole may also be used to activate autophagy with potential for treatment of cancers (including by targeting ATG4B and EVA1A), neurodegenerative diseases including Alzheimer's disease, aging-associated disorders and conditions, inflammatory diseases and infectious diseases including bacterial diseases and viral diseases such as HIV infection, liver diseases, myopathies (Chauhan et al., 2015; Panda et al., 2019; Agrotis and Ketteler., 2019; Zhen et al., 2020).

In some embodiments, the cancer, e.g. solid cancer, or haematological cancer, may be selected from Bronchial Tumors, Central Nervous System Cancer, Central Nervous System Embryonal Tumors, Carcinoma, Acute Myeloid Leukemia (AML), Carcinoid Tumor, Appendix Cancer, Astrocytomas, Chordoma, Atypical Teratoid/Rhabdoid Tumor, Sarcoma, Bladder Cancer, Thyroid Cancer, Primary Central Nervous System (CNS) Lymphoma, Extracranial Germ Cell Tumor, Esophageal Cancer, AIDS-Related Cancers, Hepatocellular (Liver) Cancer, Penile Cancer, Pleuropulmonary Blastoma, Gallbladder Cancer, Rhabdomyosarcoma, Waldenstrom Macroglobulinemia, Salivary Gland Cancer, Central Nervous System Germ Cell Tumors, Plasma Cell Neoplasm, Lip and Oral Cavity Cancer, Testicular Cancer, Extrahepatic Bile Duct Cancer, Ductal Carcinoma In Situ (DCIS), Nasopharyngeal Cancer, Nasal Cavity and Paranasal Sinus Cancer, Bone Cancer, Breast Cancer, Glioma, Hairy Cell Leukemia, Langerhans Cell Histiocytosis, Oral Cancer, Ependymoma, Cutaneous T-Cell Lymphoma, Gestational Trophoblastic Disease, Eye Cancer, Kaposi Sarcoma, Extragonadal Germ Cell Tumor, Gastric (Stomach) Cancer, Gastrointestinal Stromal Tumors (GIST), Papillomatosis, Small Intestine Cancer, Brain and Spinal Cord Tumors, Waldenstrom Macroglobulinemia, Pancreatic Cancer, Pharyngeal Cancer, Oropharyngeal Cancer, Paraganglioma, Nonmelanoma Skin Cancer, Myelodysplastic/Myeloproliferative Neoplasms, Squamous Cell Carcinoma, Malignant Fibrous Histiocytoma, Melanoma, Sezary Syndrome, Merkel Cell Carcinoma, Pituitary Tumor, Malignant Fibrous Histiocytoma of Bone and Osteosarcoma, Ovarian Cancer, Parathyroid Cancer, Skin Cancer, Mycosis Fungoides, Germ Cell Tumor, Fallopian Tube Cancer, Intraocular Melanoma, Leukemia, Pancreatic Neuroendocrine Tumors (Islet Cell Tumors), Endometrial Cancer, Lymphoma, Prostate Cancer, Renal Pelvis and Ureter Cancer, Osteosarcoma (Bone Cancer), Non-Hodgkin Lymphoma, Non-Small Cell Lung Cancer, Basal Cell Carcinoma, Laryngeal Cancer, Multiple Myeloma/Plasma Cell Neoplasm, Vaginal Cancer, Squamous Neck Cancer, Multiple Myeloma, Midline Tract Carcinoma Involving NUT Gene, Head and Neck Cancer, Heart Cancer, Intraocular (Eye), Renal Cell (Kidney) Cancer, Malignant Fibrous Histiocytoma of Bone, Liver Cancer, Rectal Cancer, Colon Cancer, Malignant Mesothelioma, Low Malignant Potential Tumor, Mouth Cancer, Soft Tissue Sarcoma, Hypopharyngeal Cancer, Wilms Tumor, Epithelial Cancer, Ewing Sarcoma Family of Tumors, Acute Lymphoblastic Leukemia (ALL), Retinoblastoma, Hodgkin Lymphoma, Brain Tumor/Cancer, Esthesioneuroblastoma, Embryonal Tumors, Cervical Cancer, Chronic Myeloproliferative Neoplasms, Pancreatic Neuroendocrine Tumors, Ureter and Renal Pelvis Cancer, Anal Cancer, Urethral Cancer, Brain Stem Cancer, Vulvar Cancer, Chronic Lymphocytic Leukemia (CLL), Uterine Sarcoma, Stomach (Gastric) Cancer, Brain Stem Glioma, Multiple Endocrine Neoplasia Syndromes, Myelodysplastic Syndromes, Craniopharyngioma, Small Cell Lung Cancer, Lip and Oral Cavity Cancer, Cutaneous T-Cell Lymphoma, Neuroblastoma, Acute Lymphoblastic Leukemia (ALL), Langerhans Cell Histiocytosis, Breast Cancer, Gastrointestinal Carcinoid Tumor, Paranasal Sinus and Nasal Cavity Cancer, Pheochromocytoma, Metastatic Squamous Neck Cancer with Occult Primary, Male Breast Cancer, Kidney (Renal) Cancer, Lung Cancer, Islet Cell Tumors, Extrahepatic Bile Duct Cancer, Endometrial Uterine Cancer, Chronic Myeloproliferative Neoplasms, Transitional Cell Cancer of the Renal Pelvis and Ureter, Thymoma and Thymic Carcinoma, Throat Cancer, Ewing Sarcoma, Chronic Myelogenous Leukemia (CML), Colorectal Cancer, Colon Cancer, Cardiac (Heart) Tumors, Burkitt Lymphoma, Carcinoma of Unknown Primary, Adrenocortical Carcinoma, Adrenocortical Adenocarcinoma, Adrenocortical Adenoma, Central Nervous System Atypical Teratoid/Rhabdoid Tumor, Childhood Cancers, Non-Hodgkin Lymphoma, Kidney (Renal) Cancer, and P-gp expressing Multidrug Resistant Tumors.

In some embodiments, the cancer may be Sarcoma, Ovarian Cancer, Kidney (Renal) Cancer, Melanoma, Colorectal Cancer, Colon Cancer, Lung Cancer, Brain Tumour/Cancer, Adrenocortical Carcinoma, Adrenocortical Adenocarcinoma, Adrenocortical Adenoma, P-gp expressing Multidrug Resistant Tumors, or Neuroblastoma.

In some embodiments, the pharmaceutical composition of the invention is for use in a method of treating non-cancer proliferative disease, inflammatory disease including autoimmune inflammatory disease and gout, a fibrotic disease, a parasitic disease or a fungal disease.

In some embodiments, the compositions of the invention are for use in treating a human. The subject of the methods of treatment described herein may be a human. The human may be an adult human. The human may be a child in which the CYP1A2 isoenzyme is active, even if it has not yet reached adult levels of activity. The World Health Organisation (WHO/CDS/CPE/PVC/2002.4, 2002) states that the metabolism and catabolism of mebendazole (a benzimidazole that is structurally related to flubendazole and has been shown to be metabolized similarly) attains adult levels of activity in children aged from 10 to 24 months. Accordingly, in some embodiments the human subject may be a child aged 10 months or older (e.g. 24 months or older). In some embodiments, the human subject may be a child younger than 10 months with evidence of CYP1A2 activity.

In some embodiments, the compositions of the invention may be for use in treating a non-human animal, e.g. a mammal or a bird. The subject of the methods of treatment described herein may be a non-human animal, e.g. a mammal or a bird. The non-human animal is typically an animal in which CYP1A2 is expressed. Animals in which CYP1A2 is expressed include mouse, rat, quail, rabbit, chicken, cat, dog, sheep, pig, cow, horse and monkey, showing that CYP1A2 expression is extensively conserved across animal species.

The compositions of the present invention can be administered in a variety of dosage forms, for example orally such as in the form of tablets, capsules, sugar- or film-coated tablets, liquid solutions or suspensions or parenterally, for example intramuscularly, intravenously or subcutaneously. The compositions may therefore be given by injection, infusion, or by inhalation or nebulisation. The compositions are preferably given by oral administration.

The compositions of the present invention are preferably for (e.g. suitable for) oral administration.

The dosage of each active ingredient in the compositions of the present invention depends on a variety of factors including the age, weight and condition of the patient and the route of administration. Daily dosages can vary within wide limits and will be adjusted to the individual requirements in each particular case. Typically, however, the dosage adopted for each route of administration when a compound is administered alone to adult humans is 0.0001 to 650 mg/kg or 0.001 to 650 mg/kg, most commonly in the range of 0.001 to 75 mg/kg, 0.01 to 75 mg/kg or 0.001 to 10 mg/kg body weight. For example, the dosage adopted for each route of administration when a compound is administered alone to adult humans may be 0.05 to 25 mg/kg or 0.01 to 1 mg/kg. Such a dosage may be given, for example, from 1 to 5 times daily. For intravenous injection, a suitable daily dose is from 0.0001 to 50 mg/kg body weight, 0.0001 to 10 mg/kg body weight or 0.0001 to 1 mg/kg body weight. Preferably, for intravenous injection, the suitable daily dose is from 0.01 to 50 mg/kg body weight or preferably from 0.05 to 25 mg/kg body weight. A daily dosage can be administered as a single dosage or according to a divided dose schedule. A unit dose form such as a tablet or a capsule will usually contain 1-500 mg or 1-250 mg of active ingredient. For example, either of the active ingredients of the compositions of the present invention could be administered to a human patient at a dose of between 1-1500 mg, 1-1000 mg, 1-500 mg or 100-250 mg either once a day, twice or three times a day. Dosages for non-human animals may be calculated based on the description of human dosages set out above.

The dosage of the strong or moderate CYP1A2 inhibitor in the compositions of the invention is typically the lowest dosage required in order to achieve satisfactory inhibition of metabolism (intrinsic clearance) of flubendazole. The dosage of the strong or moderate CYP1A2 inhibitor in the compositions of the invention is typically lower than would be used if the CYP1A2 inhibitor were being administered as a single active ingredient for a therapeutic target(s) other than CYP1A2 inhibition. The ability to dose the strong or moderate CYP1A2 inhibitor at levels typically lower than if the strong or moderate CYP1A2 inhibitor were being administered as a single active ingredient may provide an advantageous reduction of side effects resulting from the strong or moderate CYP1A2 inhibitor. However, in certain circumstances, the CYP1A2 inhibitor (e.g. fluvoxamine or thiabendazole) may have therapeutic activity (e.g. anticancer or antiparasitic activity) requiring a higher dosage to achieve additional therapeutic activity, over and above that required to achieve satisfactory CYP1A2 inhibition of flubendazole metabolism. Under such circumstances, the dosage of the CYP1A2 inhibitor may be increased to therapeutic levels above that required only for CYP1A2 inhibition.

The pharmaceutical composition of the invention may further comprise a pharmaceutically acceptable adjuvant, diluent or carrier. Conventional procedures for the selection and preparation of suitable pharmaceutical formulations are described in, for example, Pharmaceuticals - The Science of Dosage Form Designs, M. E. Aulton, Churchill Livingstone, 1988.

Depending on the mode of administration, the pharmaceutical composition will preferably comprise from 0.05 to 99% w/w (percent by weight), more preferably from 0.05 to 80% w/w, still more preferably from 0.10 to 70% w/w, and even more preferably from 0.10 to 50% w/w, of the combined weight of the active ingredients, all percentages by weight being based on total composition. Described is a process for the preparation of a pharmaceutical composition of the invention which comprises mixing the active ingredients as hereinbefore described with a pharmaceutically acceptable adjuvant, diluent or carrier.

The compositions of the invention may be administered in a variety of dosage forms. Thus, they can be administered orally, for example as tablets, capsules, troches, lozenges, aqueous or oily suspensions, solutions, dispersible powders or granules. The compositions of the invention may also be administered parenterally, whether subcutaneously, intravenously, intramuscularly, intrasternally, transdermally, by infusion techniques or by inhalation or nebulisation. The compositions may also be administered as suppositories. Solid oral forms of the pharmaceutical composition of the invention may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulfates; and, in general, non-toxic and pharmacologically inactive substances used in pharmaceutical formulations. Such pharmaceutical preparations may be manufactured in known manner, for example, by means of mixing, granulating, tableting, sugar coating, or film coating

processes.

Liquid dispersions for oral administration may be solutions, syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, propylene glycol or polyvinyl alcohol. The suspension or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride. Further suitable carriers for suspensions include sterile water, hydroxypropyl-β-cyclodextrin, hydroxypropylmethyl cellulose (HPMC), polysorbate 80, polyvinylpyrrolidone (PVP), aerosol AOT (i.e. sodium 1,2-bis(2-ethylhexoxycarbonyl)ethanesulphonate), D-α-Tocopherol polyethylene glycol 1000 succinate, pluronic F127 and/or captisol (i.e. sulfobutylether-beta-cyclodextrin).

The compounds of the invention may, for example, be formulated as aqueous suspensions or aqueous solutions in a carrier containing one or more of the following excipients at concentrations such as:

(i) 0.5% w/v hydroxypropylmethyl cellulose (HPMC);
(ii) 0.67% w/v polyvinylpyrrolidone (PVP)/0.33% w/v aerosol AOT (sodium 1,2-bis(2-ethylhexoxycarbonyl)ethanesulphonate);
(iii) 1 % w/v pluronic F 127;
(iv) 30% w/v propylene glycol;
(v) 40% w/v hydroxypropyl-β-cyclodextrin;
(vi) 40% w/v β-cyclodextrin;
(vii) 40% w/v methyl-p-cyclodextrin;
(viii) 40% w/v captisol (i.e. sulfobutylether-β-cyclodextrin).
(ix) 0.5% w/v polysorbate 80; and
(x) 10% w/v polyethylene glycol (PEG)
(xi) 20% w/v D-α-Tocopherol polyethylene glycol 1000 succinate

The carriers may be prepared by standard procedures known to those of skill in the art. For example, each of the carriers (i) to (xi) may be prepared by weighing the required amount of excipient into a suitable vessel, adding approximately 80% of the final volume of water and magnetically or mechanically stirring until a solution is formed. The carrier is then made up to volume with water. The aqueous suspensions of the active ingredients of the compositions may be prepared by weighing the required amount of each active ingredient into a suitable vessel, adding 100% of the required volume of carrier and magnetically or mechanically stirring. Solutions for injection or infusion may contain as carrier, for example, sterile water or preferably they may be in the form of sterile, aqueous, isotonic saline solutions.

In one embodiment, the flubendazole or a pharmaceutically acceptable salt, solvate, hydrate or N-oxide thereof is administered to a subject concurrently with the strong or moderate CYP1A2 inhibitor.

In one embodiment, the flubendazole or a pharmaceutically acceptable salt, solvate, hydrate or N-oxide thereof is administered to a subject separately to the strong or moderate CYP1A2 inhibitor.

In one embodiment, the flubendazole or a pharmaceutically acceptable salt, solvate, hydrate or N-oxide thereof is administered to a subject sequentially to administration of the strong or moderate CYP1A2 inhibitor.

## Examples

*Example 1: Study to Investigate the Cytochrome P450 Reaction Phenotyping of the Test Compound, Flubendazole, Using Recombinant Enzymes*

The objective of this study was to determine the stability of the test compound, flubendazole, in the presence of CYP1A2, CYP2B6, CYP2C8, CYP2C9, CYP2C19, CYP2D6 and CYP3A4 recombinant cytochrome P450 enzymes.

Flubendazole (1 μM) was incubated with recombinant enzymes and NADPH for 45 min and samples taken at the required time points. The amount of test compound remaining at each time point was analysed by LC-MS/MS. Incubations in control recombinant enzymes were also performed to assess non-enzymatic mediated stability. Appropriate control compounds for each isoform were also incubated, and gave appropriate results.

Bactosomes™ (cDNA expressed human P450 enzyme preparations co-expressed with human NADPH cytochrome P450 reductase) for each P450 isoform, 0.1 M phosphate buffer pH 7.4 and test compound (final flubendazole substrate concentration 1 μM) were pre-incubated at 37 °C prior to the addition of NADPH (final concentration 1 mM) to initiate the reaction. Incubations were also performed using control Bactosomes™ (no cytochrome P450 enzymes present) to reveal any non-enzymatic degradation. Control compounds known to be metabolised specifically by each P450 isoform were included. All incubations were performed singularly for each test compound. Each compound was

incubated for 0, 5, 15, 30 and 45 min with each isoform. The reactions were stopped by transferring an aliquot of incubate to quench solvent at the appropriate time points, in a 1:3 ratio. The termination plates were centrifuged at 3,000 rpm for 20 min at 4 °C to precipitate the protein. Following protein precipitation, the sample supernatants were analysed using LC-MS/MS, along with internal standard (1:1 supernatant to internal standard). The control compounds behaved as expected in the assays. The results for flubendazole can be found in Table 1, and specifically for CYP1A2 in Figure 1.

Following correction for any loss in the incubations with the control Bactosomes™, from a plot of In peak area ratio (compound peak area/internal standard peak area) against time, the gradient of the line was determined. Subsequently, half-life ($t_{½}$) was calculated using the equations below:

$$\text{Elimination rate constant (k)} = (\text{- gradient})$$

$$\text{Half-life } (t_{½})(\text{min}) = \frac{0.693}{k}$$

A minus half-life value is interpreted as flubendazole not being metabolised.

Flubendazole gave a surprisingly very short half-life value of 2.34 min in the presence of CYP1A2 recombinant enzyme. There was little or no turnover half-life ($t_{1/2}$) ≥ 147 min) of flubendazole in the presence of CYP2B6, CYP2C8, CYP2C9, CYP2C19, CYP2D6 or CYP3A4 recombinant enzymes.

**Table 1: Cytochrome P450 reaction phenotyping data for flubendazole**

| Compound | Isoform | Cytochrome P450 Reaction Phenotyping | | | | | | | |
|---|---|---|---|---|---|---|---|---|---|
| | | $t_{½}$ (min) | SE $t_{½}$ (min) | n | Compound Remaining (% of 0 min) | | | | |
| | | | | | 0 min | 5 min | 15 min | 30 min | 45 min |
| Flubendazole | 1A2 | 2.34 | 0.290 | 3[a] | 100 | 38.6 | 1.30 | 0.0440 | 0.00200 |
| | 2B6 | -674* | 545 | 5 | 100 | 96.5 | 95.8 | 97.8 | 104 |
| | 2C8 | 690 | 256 | 5 | 100 | 100 | 101 | 99.4 | 95.3 |
| | 2C9 | 5720 | 20900 | 5 | 100 | 97.4 | 101 | 97.9 | 99.0 |
| | 2C19 | 815 | 273 | 5 | 100 | 99.5 | 97.7 | 98.8 | 95.5 |
| | 2D6 | 147 | 17.8 | 5 | 100 | 93.1 | 90.0 | 85.6 | 78.9 |
| | 3A4 | -4150* | 18200 | 5 | 100 | 94.6 | 98.2 | 100 | 97.5 |

a = 30 and 45 minute time points excluded
* minus value shows that flubendazole was Not Metabolised in this assay

The stability of flubendazole in the presence of CYP1A2 recombinant P450 isoform is shown in Figure 1, and for the other CYP isoforms in Figures 2 to 7.

In conclusion, flubendazole was very rapidly metabolised by the CYP1A2 recombinant enzyme. There was little or no turnover half-life ($t_{1/2}$ ≥ 147 min) of flubendazole in the presence of CYP2B6, CYP2C8, CYP2C9, CYP2C19, CYP2D6 or CYP3A4 recombinant enzymes.

*Example 2: Study to Investigate the Stability of the Test Compound, Flubendazole, in Human Liver Microsomes in the Absence and Presence of the Inhibitor Furafylline*

The objective of this study was to determine and comparatively assess the stability of flubendazole in human liver microsomes in the absence of inhibitor and in the presence of the CYP1A2 inhibitor furafylline (3, 10 and 30 $\mu$M).

Flubendazole (1 $\mu$M) was incubated with human liver microsomes (in the absence and presence of inhibitor) and NADPH for 45 min and samples taken at the required time points. The amount of test compound remaining at each time point was analysed by LC-MS/MS. An incubation in the absence of NADPH was also performed to assess non-CYP mediated stability.

In the pre-screen assay, a minus cofactor control incubation was included for each compound tested where 0.1 M phosphate buffer pH 7.4 (final substrate concentration 1 $\mu$M) were pre-incubated at 37 °C prior to the addition of NADPH (final concentration 1 mM) to initiate the reaction. A minus cofactor control incubation was included for each

compound tested where 0.1 M phosphate buffer pH 7.4 was added instead of NADPH (minus NADPH). Each test compound was incubated for 0, 5, 15, 30 and 45 min. The control (minus NADPH) was incubated for 45 min only. The samples were analysed for flubendazole using LC-MS/MS. In this pre-screen assay, appropriate control compounds (dextromethorphan and verapamil) were also incubated, and gave the appropriate results. Flubendazole gave an intrinsic clearance ($CL_{int}$) value of 43.7 μL/min/mg protein in the absence of inhibitor in human liver microsomes in the pre-screen assay.

For cytochrome P450 reaction phenotyping by chemical inhibitor (furafylline), human liver microsomes (final protein concentration 0.5 mg/mL), CYP1A2 specific (strong, time-dependent) inhibitor (3, 10 or 30 μM furafylline; final methanol concentration 0.08 %), 0.1 M phosphate buffer pH 7.4 and NADPH (final concentration 1 mM) were pre-incubated at 37 °C prior to the addition of flubendazole or phenacetin (final substrate concentration, 1 μM; final DMSO concentration 0.25%) to initiate the reaction. Test compound (flubendazole or the CYP1A2 substrate, phenacetin) was incubated without inhibitor and in the presence of each specific inhibitor concentration. A minus cofactor control incubation was included for each compound tested where 0.1 M phosphate buffer pH 7.4 was added instead of cofactor (minus NADPH). Each test compound was incubated for 0, 5, 15, 30 and 45 min. The control (minus NADPH) was incubated for 45 min only. The samples were analysed for flubendazole or phenacetin (positive control CYP1A2 substrate) using LC-MS/MS. The reactions were stopped by transferring an aliquot of incubate to acetonitrile at the appropriate time points, in a 1:3 ratio. The termination plates were centrifuged at 3,000 rpm for 20 min at 4 °C to precipitate the protein. Following protein precipitation, the sample supernatants were analysed using LC-MS/MS, along with internal standard (1:1 supernatant to internal standard).

From a plot of ln peak area ratio (compound peak area/internal standard peak area) against time, the gradient of the line was determined. Subsequently, half-life and intrinsic clearance were calculated using the equations below:

$$\text{Elimination rate constant (k)} = (\text{- gradient})$$

$$\text{Half-life } (t_{½})(\text{min}) = \frac{0.693}{k}$$

$$\text{Intrinsic clearance } (CL_{int})(\mu L/\text{min/mg protein}) = \frac{V\,x\,0.693}{t_{1/2}}$$

where V = Incubation volume (μL)/Microsomal protein (mg)

Flubendazole clearance showed marked decreases in the presence of furafylline with intrinsic clearance values of 14.6, 3.52 and 5.39 μL/min/mg protein in the presence of 3, 10 and 30 μM furafylline respectively, compared to 50.3 μL/min/mg protein in the absence of inhibitor in human liver microsomes. These results are shown in Figures 8 to 11. Phenacetin, a positive control CYP1A2 probe substrate, gave an intrinsic clearance value of 38.6 μL/min/mg protein in the absence of inhibitor in human liver microsomes. Phenacetin clearance showed similar marked decreases, as seen with flubendazole, with intrinsic clearance values of 5.45, 2.41 and 2.88 μL/min/mg protein in the presence of 3, 10 and 30 μM furafylline respectively.

The results for this study are summarized in Table 2.

**Table 2: Metabolic stability (with CYP1A2 inhibitor, furafylline) data for flubendazole, and the control compound, phenacetin**

| Compound | Inhibitor Conc (μM) | Metabolic Stability (With or Without Chemical Inhibitor, Furafylline) (Species=Human) | | | | |
|---|---|---|---|---|---|---|
| | | $CL_{int}$ (μL/min/mg protein) | SE $CL_{int}$ | $t_{½}$ (min) | n | Comments |
| Flubendazole | None | 50.3 | 1.05 | 27.6 | 5 | No Inhibitor. |
| | Furafylline 3 μM | 14.6 | 1.45 | 95.1 | 5 | Plus 3 μM furafylline. |
| | Furafylline 10 μM | 3.52 | 2.42 | 394 | 5 | Plus 10 μM furafylline. |
| | Furafylline 30 μM | 5.39 | 6.25 | 257 | 5 | Plus 30 μM furafylline. |

(continued)

| Compound | Inhibitor Conc (μM) | Metabolic Stability (With or Without Chemical Inhibitor, Furafylline) (Species=Human) | | | | |
|---|---|---|---|---|---|---|
| | | $CL_{int}$ (μL/min/mg protein) | SE $CL_{int}$ | $t_{1/2}$ (min) | n | Comments |
| Phenacetin | None | 38.6 | 6.91 | 35.9 | 5 | No inhibitor. |
| | Furafylline 3 μM | 5.45 | 0.570 | 254 | 5 | Plus 3 μM furafylline. |
| | Furafylline 10 μM | 2.41 | 2.32 | 575 | 5 | Plus 10 μM furafylline. |
| | Furafylline 30 μM | 2.88 | 1.81 | 482 | 5 | Plus 30 μM furafylline. |

In conclusion, flubendazole intrinsic clearance in human liver microsomes was strongly decreased in the presence of the strong CYP1A2 inhibitor furafylline by up to 14.3 fold. This was highly comparable with phenacetin, a CYP1A2 probe substrate, with intrinsic clearance in human liver microsomes which was strongly decreased in the presence of the strong CYP1A2 inhibitor furafylline by up to 16 fold.

*Example 3: Study to Investigate the Stability of the Test Compound, Flubendazole, in Human Cryopreserved Hepatocytes in the Absence and Presence of the Inhibitors Cimetidine, Mexiletine, Thiabendazole, Fluvoxamine and Furafylline*

The objective of this study was to determine the stability of the test compound, flubendazole, in the presence of human cryopreserved hepatocytes in the absence and presence of furafylline (1, 3, 10 and 30 μM), fluvoxamine (3, 10, 30 and 100 μM), thiabendazole (3, 10, 30 and 100 μM), mexiletine (10, 30, 100 and 300 μM), and cimetidine (10, 30, 100 and 300 μM).

After an initial pre-screening assay (assay 1), flubendazole (1 μM) was incubated in assay 2 with cryopreserved human hepatocytes for 60 min in the absence and presence of furafylline at 1, 3, 10 and 30 μM, fluvoxamine at 3, 10, 30 and 100 μM, or thiabendazole at 3, 10, 30 and 100 μM, and samples taken at the required time points (0, 5, 10, 20, 40 and 60 min). Flubendazole (1 μM) was incubated in assay 3 with cryopreserved human hepatocytes for 60 min in the absence and presence of mexiletine at 10, 30, 100 and 300 μM, and cimetidine at 10, 30, 100 and 300 μM, and samples taken at the required time points (0, 5, 10, 20, 40 and 60 min). In each assay, the amount of test compound remaining at each time point was analysed by LC-MS/MS. Appropriate control compounds were also incubated along with ethoxycoumarin, which was incubated in the absence and presence of furafylline, fluvoxamine, thiabendazole, mexiletine or cimetidine at the same concentrations.

In these human hepatocyte stability investigations, Williams E media supplemented with 2 mM L-glutamine and 25 mM HEPES, test compound (final substrate concentration 1 μM; final DMSO concentration 0.25 %) and, where applicable, the selected inhibitor (furafylline at 1, 3, 10 and 30 μM, fluvoxamine at 3, 10, 30 and 100 μM, thiabendazole at 3, 10, 30 and 100 μM, mexiletine at 10, 30, 100 and 300 μM, and cimetidine at 10, 30, 100 and 300 μM, final methanol concentration 0.08 %), were pre-incubated at 37 °C prior to the addition of a suspension of cryopreserved hepatocytes (final cell density $0.5 \times 10^6$ viable cells/mL in Williams E media supplemented with 2 mM L glutamine and 25 mM HEPES) to initiate the reaction. The final incubation volume was 500 μL. Two control compounds (verampamil and umbelliferone) were included, alongside appropriate vehicle control. A positive control compound, ethoxycoumarin, for CYP1A2 (as CYP1A2 substrate) was included. The positive control compound was incubated without inhibitor and in the presence of each inhibitor at the same concentrations as used with flubendazole as substrate. The reactions were stopped by transferring 50 μL of incubate to 100 μL acetonitrile containing internal standard at the appropriate time points (0, 5, 10, 20, 40 and 60 min). The termination plates were centrifuged at 2500 rpm at 4 °C for 30 min to precipitate the protein. Following protein precipitation, the sample supernatants were analysed by LC-MS/MS, optimised for analysis of flubendazole.

Two control compounds (verampamil and umbelliferone) were included in the assay and if the values for these compounds were not within the specified limits (where available) the results were rejected and the experiment repeated. In these assays, the control compounds gave acceptable results. Where observed, a minus half-life value is interpreted as flubendazole not being metabolised.

The CYP1A2 substrate positive control compound, ethoxycoumarin, as well as the two control compounds behaved as expected in the assay and all intrinsic clearance values were within acceptable limits.

From a plot of ln peak area ratio (compound peak area/internal standard peak area) against time, the gradient of the line was determined. Subsequently, half-life ($t_{½}$) and intrinsic clearance ($CL_{int}$) were calculated using the equations below:

$$\text{Elimination rate constant (k)} = (\text{- gradient})$$

$$\text{Half-life } (t_{½})(\text{min}) = \frac{0.693}{k}$$

$$\text{Intrinsic clearance } (CL_{int})(\mu\text{L/min/million cells}) = \frac{V \times 0.693}{t_{½}}$$

where V = Incubation volume (μL)/Number of cells

Flubendazole was metabolised by cryopreserved human hepatocytes, as shown in Figure 12, in assay 1 (pre-screening assay) with flubendazole giving an intrinsic clearance value of 15.2±1.09 μL/min/$10^6$ cells in the presence of human cryopreserved hepatocytes. Verampamil and umbelliferone gave intrinsic clearance values of 76.5±4.78 and 129±2.93 μL/min/$10^6$ cells in presence of human cryopreserved hepatocytes.

In assay 2, flubendazole gave an intrinsic clearance value of 8.62±2.23 μL/min/$10^6$ cells in the absence of inhibitor in human cryopreserved hepatocytes. The positive control compounds (ethoxycoumarin, verampamil, umbelliferone) behaved as expected in the assay and all intrinsic clearance values were within acceptable limits. Verampamil and umbelliferone gave intrinsic clearance values of 78.5±1.92 and 136±8.14 μL/min/$10^6$ cells in presence of human cryopreserved hepatocytes. Flubendazole gave intrinsic clearance values of 2.05±2.04, 1.70±0.885, -3.07±1.05* and -3.30±0.869* μL/min/$10^6$ cells in the presence of 1, 3, 10 and 30 μM furafylline respectively, -4.27±0.987*, 2.96±1.17, - 12.5±2.43* and -10.2±1.59* μL/min/$10^6$ cells in the presence of 3, 10, 30 and 100 μM thiabendazole respectively and -0.256±0.329*, 3.95±0.941, -0.0753±0.999* and - 3.77±0.389* μL/min/$10^6$ cells in the presence of 3, 10, 30 and 100 μM fluvoxamine respectively, in the human cryopreserved hepatocytes. The results are shown in Tables 3 and 4, and in Figures 13 to 25. (* minus values show that flubendazole was Not Metabolised in the assay).

In assay 3, flubendazole gave an intrinsic clearance value of 16.7±2.06 μL/min/$10^6$ cells in the absence of inhibitor in human cryopreserved hepatocytes. The positive control compounds (ethoxycoumarin, verampamil, umbelliferone) behaved as expected in the assay and all intrinsic clearance values were within acceptable limits. Verampamil and umbelliferone gave intrinsic clearance values of 73.1±4.70 and 131±9.59 μL/min/$10^6$ cells in presence of human cryopreserved hepatocytes. Flubendazole gave intrinsic clearance values of 5.43±1.90, -5.17±1.93*, 10.9±1.27 and 9.19±1.70 μL/min/$10^6$ cells in the presence of 10, 30, 100 and 300 μM cimetidine respectively, and 0.861±0.522, -9.75±1.79**, -4.44±1.16** and 5.85±1.25 μL/min/$10^6$ cells in the presence of 10, 30, 100 and 300 μM mexiletine respectively in human cryopreserved hepatocytes. The results are shown in Tables 5 and 6, and in Figures 26 to 33.

*Based on previous experience of the performing laboratory using cimetidine, 30 μM data is considered to be an outlier. Furthermore, the result for cimetidine at 30 μM using ethoxycoumarin as substrate in Table 6 gives an expected result (** minus values show that flubendazole was Not Metabolised in the assay).

Altogether, the results are shown in Tables 3, 4, 5 and 6, and in Figures 12 to 33.

**Table 3: Hepatocyte stability data for flubendazole (at 1 μM) with or without CYP1A2 inhibitors (assay 2)**

| Inhibitor | $CL_{int}$ (μL/min/$10^6$ cells) | SE $CL_{int}$ | $t_{½}$ (min) | n |
|---|---|---|---|---|
| None | 8.62 | 2.23 | 161 | 6 |
| Furafylline (1 μM) | 2.05 | 2.04 | 677 | 6 |
| Furafylline (3 μM) | 1.70 | 0.885 | 816 | 6 |
| Furafylline (10 μM) | -3.07* | 1.05 | -452* | 6 |
| Furafylline (30 μM) | -3.30* | 0.869 | -421* | 6 |
| Fluvoxamine (3 μM) | -0.256* | 0.329 | -5420* | 6 |
| Fluvoxamine (10 μM) | 3.95 | 0.941 | 351 | 6 |
| Fluvoxamine (30 μM) | -0.0753* | 0.999 | -18400* | 6 |
| Fluvoxamine (100 μM) | -3.77* | 0.389 | -367* | 6 |

(continued)

| Inhibitor | $CL_{int}$ (μL/min/$10^6$ cells) | SE $CL_{int}$ | $t_{½}$ (min) | n |
|---|---|---|---|---|
| Thiabendazole (3 μM) | -4.27* | 0.987 | -325* | 6 |
| Thiabendazole (10 μM) | 2.96 | 1.17 | 468 | 6 |
| Thiabendazole (30 μM) | -12.5* | 2.43 | -111* | 6 |
| Thiabendazole (100 μM) | -10.2* | 1.59 | -136* | 6 |
| n = number of time points used to fit curve; SE = Standard error of the curve fitting<br>* minus value shows that flubendazole was Not Metabolised in this assay | | | | |

**Table 4: Hepatocyte stability data for ethoxycoumarin (positive control) with or without CYP1A2 inhibitors and for the control compounds (assay 2)**

| Compound | Inhibitor | $CL_{int}$ (μL/min/$10^6$ cells) | SE $CL_{int}$ | $t_{½}$ (min) | n |
|---|---|---|---|---|---|
| Verapamil | None | 78.5 | 1.92 | 17.7 | 6 |
| Umbelliferone | None | 136 | 8.14 | 10.2 | 4 a |
| Ethoxycoumarin | None | 25.4 | 1.58 | 54.5 | 6 |
| Ethoxycoumarin | Furafylline (1 μM) | 12.7 | 1.20 | 109 | 6 |
| Ethoxycoumarin | Furafylline (3 μM) | 10.4 | 1.22 | 133 | 6 |
| Ethoxycoumarin | Furafylline (10 μM) | 5.55 | 0.612 | 250 | 6 |
| Ethoxycoumarin | Furafylline (30 μM) | 2.78 | 1.39 | 498 | 6 |
| Ethoxycoumarin | Fluvoxamine (3 μM) | 4.50 | 0.899 | 308 | 6 |
| Ethoxycoumarin | Fluvoxamine (10 μM) | 2.37 | 0.904 | 586 | 6 |
| Ethoxycoumarin | Fluvoxamine (30 μM) | 3.33 | 1.05 | 417 | 6 |
| Ethoxycoumarin | Fluvoxamine (100 μM) | 4.82 | 0.827 | 287 | 6 |
| Ethoxycoumarin | Thiabendazole (3 μM) | 3.59 | 0.858 | 386 | 6 |
| Ethoxycoumarin | Thiabendazole | 1.63 | 1.20 | 849 | 6 |
| Ethoxycoumarin | Thiabendazole | -2.60* | 2.39 | -533* | 6 |
| Ethoxycoumarin | Thiabendazole (100 μM) | 2.89 | 1.35 | 479 | 6 |
| n = number of time points used to fit curve; SE = Standard error of the curve fitting<br>[a] = 40 and 60 minute time points excluded<br>* minus value shows that flubendazole was Not Metabolised in this assay | | | | | |

**Table 5: Hepatocyte stability data for flubendazole (at 1 μM) with or without CYP1A2 inhibitors (assay 3)**

| Inhibitor | $CL_{int}$ (μL/min/$10^6$ cells) | SE $CL_{int}$ | $t_{½}$ (min) | n |
|---|---|---|---|---|
| None | 16.7 | 2.06 | 82.8 | 6 |
| Mexiletine (10 μM) | 0.861 | 0.522 | 1610 | 6 |
| Mexiletine (30 μM) | -9.75* | 1.79 | -142* | 6 |
| Mexiletine (100 μM) | -4.44* | 1.16 | -312* | 6 |
| Mexiletine (300 μM) | 5.85 | 1.25 | 237 | 6 |

(continued)

| Inhibitor | $CL_{int}$ (μL/min/$10^6$ cells) | SE $CL_{int}$ | $t_{½}$ (min) | n |
|---|---|---|---|---|
| Cimetidine (10 μM) | 5.43 | 1.90 | 255 | 6 |
| Cimetidine (30 μM) | -5.17** | 1.93 | -268** | 6 |
| Cimetidine (100 μM) | 10.9 | 1.27 | 128 | 6 |
| Cimetidine (300 μM) | 9.19 | 1.70 | 151 | 6 |
| n = number of time points used to fit curve; SE = Standard error of the curve fitting<br>* minus value shows that flubendazole was Not Metabolised in this assay<br>** Based on previous experience of the laboratory using cimetidine, 30 μM data is considered to be an outlier. Result for cimetidine at 30 μM using ethoxycoumarin as substrate in Table 6 gives an expected result. | | | | |

**Table 6: Hepatocyte stability data for ethoxycoumarin (positive control) with or without CYP1A2 inhibitors and for the control compounds (assay 3)**

| Compound | Inhibitor | $CL_{int}$ (μL/min/$10^6$ cells) | SE $CL_{int}$ | $t_{½}$ (min) | n |
|---|---|---|---|---|---|
| Verapamil | None | 73.1 | 4.70 | 19.0 | 6 |
| Umbelliferone | None | 131 | 9.59 | 10.5 | 4[a] |
| Ethoxycoumarin | None | 20.0 | 2.27 | 69.3 | 6 |
| Ethoxycoumarin | Mexiletine (10 μM) | 3.60 | 2.18 | 385 | 6 |
| Ethoxycoumarin | Mexiletine (30 μM) | -7.54* | 2.13 | -184* | 6 |
| Ethoxycoumarin | Mexiletine (100 μM) | -12.8* | 2.52 | -109* | 6 |
| Ethoxycoumarin | Mexiletine (300 μM) | 4.81 | 1.63 | 288 | 6 |
| Ethoxycoumarin | Cimetidine (10 μM) | 6.00 | 3.39 | 231 | 6 |
| Ethoxycoumarin | Cimetidine (30 μM) | 16.5 | 1.96 | 84.1 | 6 |
| Ethoxycoumarin | Cimetidine (100 μM) | 17.4 | 1.69 | 79.8 | 6 |
| Ethoxycoumarin | Cimetidine (300 μM) | 15.5 | 1.97 | 89.3 | 6 |
| n = number of time points used to fit curve; SE = Standard error of the curve fitting<br>[a] = 40 and 60 minute time points excluded<br>* minus value shows that flubendazole was Not Metabolised in this assay | | | | | |

The positive control compounds (ethoxycoumarin, verampamil, umbelliferone) behaved as expected in the assays and all intrinsic clearance values were within acceptable limits. Flubendazole was metabolised by cryopreserved human hepatocytes. Metabolism was decreased in the presence of furafylline (at least 4.2 fold, with >5-fold reduction at 3 μM), thiabendazole (at least 2.9 fold, with >5-fold reduction at 3 μM), fluvoxamine (at least 2.2 fold, with >5-fold reduction at 3 μM), mexiletine (at least 2.9 fold, with >5-fold reduction at 10 μM) and cimetidine (1.5 fold to 3.1 fold). In conclusion, furafylline, thiabendazole, fluvoxamine, and mexiletine all behaved as strong CYP1A2 inhibitors of flubendazole, and cimetidine behaved as a moderate CYP1A2 inhibitor of flubendazole. These data confirm the surprising result for flubendazole as a sensitive CYP1A2 substrate, on the basis of these data, with inhibition of intrinsic clearance of flubendazole of greater than two-fold by moderate or strong CYP1A2 inhibitors of flubendazole, as substrate.

**References**

Agrotis A, Ketteler R. On ATG4B as Drug Target for Treatment of Solid Tumours-The Knowns and the Unknowns. Cells 2020, 9, 53; doi:10.3390/cells9010053

Bapiro TE, Sayi J, Hasler JA, Jande M, Rimoy G, Masselle A, Masimirembwa CM. Artemisinin and thiabendazole are potent inhibitors of cytochrome P450 1A2 (CYP1A2) activity in humans. Eur J Clin Pharmacol 2005; 61: 755-761 DOI 10.1007/s00228-005-0037-3

Ceballos L, Elissondo C, Bruni SS, Denegri G, Lanusse C, Alvarez L. Comparative Performances of Flubendazole and Albendazole in Cystic Echinococcosis: Ex Vivo Activity, Plasma/Cyst Disposition, and Efficacy in Infected Mice. Antimicrobial Agents and Chemotherapy 2011; 55 (12): 5861-5867

Ceballos L, Mackenzie C, Geary T, Alvarez L, Lanusse C. Exploring the Potential of Flubendazole in Filariasis Control: Evaluation of the Systemic Exposure for Different Pharmaceutical Preparations. PLoS Negl Trop Dis 2014; 8 (5): e2838. doi: 10.1371/journal.pntd.0002838

Ceballos L, Alvarez L, Mackenzie C, Geary T, Lanusse C. Pharmacokinetic comparison of different flubendazole formulations in pigs: A further contribution to its development as a macrofilaricide molecule. International Journal for Parasitology: Drugs and Drug Resistance 2015; 5: 178-184

Cha HJ, Byrom M, Mead PE, Ellington AD, Wallingford JB, et al. Evolutionarily Repurposed Networks Reveal the Well-Known Antifungal Drug Thiabendazole to Be a Novel Vascular Disrupting Agent. PLoS Biol 2012; 10 (8): e1001379. doi: 10.1371/journal.pbio.1001379

Chauhan S, Ahmed Z, Bradfute SB, Arko-Mensah J. Pharmaceutical screen identifies novel target processes for activation of autophagy with a broad translational potential. Nature Communications 2015; 6: 8620 DOI: 10.1038/ncomms9620

Cronstein BN, Terkeltaub R. The inflammatory process of gout and its treatment. Arthritis Research & Therapy 2006; 8 (Suppl 1): S3

Dinarello CA. Anti-inflammatory Agents: Present and Future. Cell 2010; 140; 935-950 Geary TG, Mackenzie CD, Silber SA. Flubendazole as a macrofilaricide: History and background. PLoS Negl Trop Dis 2019; 13 (1): e0006436. https://doi.org/10.1371/journal. pntd.0006436

Hayashi K, Michiue H, Yamada H, Takata K. Fluvoxamine, an anti-depressant, inhibits human glioblastoma invasion by disrupting actin polymerization. Nature Scientific Reports 2016; 6: 23372 DOI: 10.1038/srep23372

Hou Z-J, Luo X, Zhang W, Peng F, Cui B, Wu S-J, Zheng F-M, Xu J, Xu L-Z, Long Z-J, Wang X-T, Li G-H, Wan X-Y, Yang Y-L, Liu Q. Flubendazole, FDA-approved anthelmintic, targets breast cancer stem-like cells. Oncotarget 2015; 6 (8): 6326-6340

https://www.jnj.com/media-center/press-releases/janssen-discontinues-development-of-flubendazole-formulation-to-treat-onchocerciasis

Kajimura T, Sato S, Murakami A et al. Overexpression of carbonyl reductase 1 inhibits malignant behaviors and epithelial mesenchymal transition by suppressing TGF-β signaling in uterine leiomyosarcoma cells. Oncology Letters 2019; 18: 1503-1512

Kalluri R, Weinberg RA. The basics of epithelial-mesenchymal transition. J. Clin. Invest. 2009; 119: 1420-1428

Karjalainen MJ, Neuvonen PJ, Backman JT.In vitro Inhibition of CYP1A2 by Model Inhibitors, Anti-Inflammatory Analgesics and Female Sex Steroids: Predictability of in vivo Interactions. Basic & Clinical Pharmacology & Toxicology 2008; 103: 157-165

Kralova V, Hanušová V, Caltová K, Špaček P, Hochmalová M, Skálová L, Rudolf E. Flubendazole and mebendazole impair migration and epithelial to mesenchymal transition in oral cell lines. Chem Biol Interact. 2018; 293: 124-132. doi: 10.1016/j.cbi.2018.07.026. Epub 2018 Jul 31

Kubicek V, Skálová L, Skarka A, Králová V, Holubová J et al. Carbonyl Reduction of Flubendazole in the Human Liver: Strict Stereospecificity, Sex Difference, Low Risk of Drug Interactions. Front. Pharmacol. 2019; 10:600. doi: 10.3389/fphar.2019.00600

Lachau-Durand S, Lammens L, van der Leede B, Van Gompel J, Bailey G, Engelen M, Lampo A. Preclinical toxicity and pharmacokinetics of a new orally bioavailable flubendazole formulation and the impact for clinical trials and risk/benefit to patients. PLoS Negl Trop Dis 2019; 13(1): e0007026. https://doi.org/10.1371/journal. pntd.0007026

Lin S, Yang L, Yao Y, Xu L et al. Flubendazole demonstrates valid antitumor effects by inhibiting STAT3 and activating autophagy. Journal of Experimental & Clinical Cancer Research 2019; 38: 293 https://doi.org/10.1186/s13046-019-1303-z

Michaelis M, Agha B, Rothweiler F, et al. Identification of flubendazole as potential anti-neuroblastoma compound in a large cell line screen. Sci Rep. 2015; (5) 8202 DOI: 10.1038/srep08202

Nixon GL, McEntee L, Johnson A, Farrington N, Whalley S, Livermore J, Natal C, Washbourn G, Bibby J, Berry N, Lestner J, Truong M, Owen A, Lalloo D, Charles I, Hope W. Repurposing and Reformulation of the Antiparasitic Agent Flubendazole for Treatment of Cryptococcal Meningoencephalitis, a Neglected Fungal Disease. Antimicrobial Agents and Chemotherapy 2018; 62 (4): e01909-17

Nobilis M, Jira T, Lisa M. et al. Achiral and chiral high-performance liquid chromatographic determination of flubendazole and its metabolites in biomatrices using UV photodiode-array and mass spectrometric detection. Journal of Chromatography A. 2007; 1149: 112-120

Obach RS, Walsky RL, Venkatakrishnan K et al. The Utility of in Vitro Cytochrome P450 Inhibition Data in the Prediction of Drug-Drug Interactions. Journal of Pharmacology and Experimental; Therapeutics 2006; 316: 336-348

Obach RS, Walsky RL, Venkatakrishnan K. Mechanism-Based Inactivation of Human Cytochrome P450 Enzymes and the Prediction of Drug-Drug Interactions. Drug Metabolism and Disposition 2007; 35: 246-255

Oh E, Kim Y-J, An H, Sung D. Flubendazole elicits anti-metastatic effects in triple-negative breast cancer via STAT3 inhibition. Int. J. Cancer 2018: 143; 1978-1993

O'Neill M, Mansour A, DiCosty U, Geary J, Dzimianski M, McCall SD, McCall JW, Mackenzie CD, Geary TG. An In Vitro/In Vivo Model to Analyze the Effects of Flubendazole Exposure on Adult Female Brugia malayi. PLoS Negl Trop Dis 2016; 10(5): e0004698. doi:10.1371/journal. pntd.0004698

Panda PK, Fahrner A, Vats S,Seranova E, Sharma V, Chipara M, Desai P, Torresi J, Rosenstock T, Kumar D and Sarkar S. Chemical Screening Approaches Enabling Drug Discovery of Autophagy Modulators for Biomedical Applications in Human Diseases. Front. Cell Dev. Biol. 2019; 7: 38. doi: 10.3389/fcell.2019.00038

Sesardic D, Boobis AR, Murray BP et al. Furafylline is a potent and selective inhibitor of cytochrome P450IA2 in man Br. J. clin. Pharmac. (1990), 29, 651-663

Sjoberg HT, Pionnier N, Aljayyoussi G, Metuge HM, Njouendou AJ, Chunda VC, et al. Short-course, oral flubendazole does not mediate significant efficacy against Onchocerca adult male worms or Brugia microfilariae in murine infection models. PLoS Negl Trop Dis 2019; 13(1): e0006356. https://doi.org/10.1371/journal. pntd.0006356

Spagnuolo PA, Hu JY, Hurren R, et al. The antihelmintic flubendazole inhibits microtubule function through a mechanism distinct from Vinca alkaloids and displays preclinical activity in leukemia and myeloma. Blood. 2010; 115 (23): 4824-4833

Stuchlíková LR, Králová V, Lněničková K et al. The metabolism of flubendazole in human liver and cancer cell lines. Drug Test Anal. 2018; 10: 1139-1146

Tao J, Zhao H, Xie X, Luo M et al. The anthelmintic drug flubendazole induces cell apoptosis and inhibits NF-κB signaling in esophageal squamous cell carcinoma. OncoTargets and Therapy 2019:12; 471-478

Tarrus E, Cami J, Roberts DJ et al. Accumulation of caffeine in healthy volunteers treated with furafylline. Br. J. clin. Pharmac. 1987; 23: 9-18

Thelingwani RS, Zvada SP, Dolgos H, Ungell A-L, Masimirembwa CM. In Vitro and in Silico Identification and Characterization of Thiabendazole as a Mechanism-Based Inhibitor of CYP1A2 and Simulation of Possible Pharmacokinetic Drug-Drug Interactions. Drug Metabolism and Disposition 2009; 37: 1286-1294

Vialpando M, Smulders S, Bone S et al. Evaluation of Three Amorphous Drug Delivery Technologies to Improve the Oral Absorption of Flubendazole. Journal of Pharmaceutical Sciences 2016; 105: 2782-2793

Wildenberg ME, Levin AD, Ceroni A, Guo Z, Koelink PJ, Hakvoort TBM, Westera L, Bloemendaal FM, Brandse JF, Simmons A, D'Haens GR, Ebner D, van den Brink GR. Benzimidazoles Promote Anti-TNF Mediated Induction of Regulatory Macrophages and Enhance Therapeutic Efficacy in a Murine Model. Journal of Crohn's and Colitis 2017, 1480-1490

Yang J et al. Guidelines and definitions for research on epithelial-mesenchymal transition. Consensus Statement. Nature Reviews. Molecular Cell Biology 2020; 21: 341-352

Yu CG, Bondada V, Ghoshal S, Singh R et al. Repositioning Flubendazole for Spinal Cord Injury. Journal of Neurotrauma 2019; 36: 2618-2630

Yun M, Choi AJ, Woo SR et al. Inhibition of Carbonyl Reductase 1 Enhances Metastasis of Head and Neck Squamous Cell Carcinoma through β-catenin-Mediated Epithelial-Mesenchymal Transition. Journal of Cancer 2020; 11 (3): 533-541. doi: 10.7150/jca.34303

Zhen Y, Zhao R, Wang M, Jiang X, Gao F, Fu L, Zhang, L, Zhou X-L. Flubendazole elicits anti-cancer effects via targeting EVA1A-modulated autophagy and apoptosis in Triple-negative Breast Cancer. Theranostics 2020; 10 (18): 8080-8097. doi: 10.7150/thno.43473

Zhou X, Liu J, Zhang J, Wei Y, Li H. Flubendazole inhibits glioma proliferation by G2/M cell cycle arrest and pro-apoptosis. Cell Death Discovery 2018; 4: 18 DOI 10.1038/s41420-017-0017-2

## Claims

**1.** A pharmaceutical composition comprising flubendazole or a pharmaceutically acceptable salt, solvate, hydrate or N-oxide thereof, and a moderate or strong cytochrome P450 1A2 isoenzyme (CYP1A2) inhibitor, wherein:

(a) the moderate CYP1A2 inhibitor is:

(i) a CYP1A2 inhibitor which reduces intrinsic clearance of flubendazole in human hepatocytes *ex vivo* by ≥two-fold to <five-fold compared to intrinsic clearance of flubendazole in human hepatocytes *ex vivo* in the absence of a CYP1A2 inhibitor; and/or
(ii) a CYP1A2 inhibitor which increases the area under the curve (AUC) *in vivo* of flubendazole by ≥two-fold to <five-fold compared to the AUC *in vivo* of flubendazole in the absence of a CYP1A2 inhibitor; and

(b) the strong CYP1A2 inhibitor is:

(i) a CYP1A2 inhibitor which reduces intrinsic clearance of flubendazole in human hepatocytes *ex vivo* by ≥five-fold compared to intrinsic clearance of flubendazole in human hepatocytes *ex vivo* in the absence of a CYP1A2 inhibitor; and/or
(ii) a CYP1A2 inhibitor which increases the AUC *in vivo* of flubendazole by ≥five-fold compared to the AUC *in vivo* of flubendazole in the absence of a CYP1A2 inhibitor.

**2.** The pharmaceutical composition according claim 1, wherein the moderate or strong CYP1A2 inhibitor is selected

from furafylline, ciprofloxacin, enoxacin, fluvoxamine, zafirlukast, 8-phenyltheophylline, methoxsalen, thiabendazole, mexiletine and cimetidine, or a pharmaceutically acceptable salt, solvate, hydrate or *N*-oxide thereof.

**3.** The pharmaceutical composition according to claim 1 or claim 2, wherein the strong CYP1A2 inhibitor is selected from fluvoxamine, thiabendazole, furafylline, mexiletine, 8-phenyltheophylline, ciprofloxacin, enoxacin, and zafirlukast, or a pharmaceutically acceptable salt, solvate, hydrate or *N*-oxide thereof; optionally wherein the strong CYP1A2 inhibitor is selected from fluvoxamine, thiabendazole, furafylline, and mexiletine, or a pharmaceutically acceptable salt, solvate, hydrate or *N*-oxide thereof.

**4.** The pharmaceutical composition according to any one of the preceding claims, wherein the moderate CYP1A2 inhibitor is selected from cimetidine and methoxsalen.

**5.** The pharmaceutical composition according to any one of the preceding claims, wherein the moderate or strong CYP1A2 inhibitor extends the *in vivo* half-life of flubendazole by more than 2, 3, 4, 6, 8, 10, 12, 14, 16, 18 or 20 times the *in vivo* half-life of flubendazole in the absence of the moderate or strong CYP1A2 inhibitor.

**6.** The pharmaceutical composition according to any one of the preceding claims for use in medicine.

**7.** The pharmaceutical composition according to any one of the preceding claims for use in a method of treating a disease treatable by inhibition and/or disruption of microtubule structure and function, wherein the disease treatable by inhibition and/or disruption of microtubule structure and function is:

(a) cancer, optionally wherein the cancer is a haematological cancer or a solid tumour; and/or a proliferative disease;
(b) an infectious disease; optionally wherein the infectious disease is a bacterial disease or a viral disease; optionally wherein the viral disease is an HIV infection;
(c) a fungal disease;
(d) an inflammatory disease; optionally wherein the inflammatory disease is:

(i) an autoimmune disease; or
(ii) gout;
or

(e) a fibrotic disease.

**8.** The pharmaceutical composition for use according to claim 7, wherein the infectious disease is a parasitic disease; optionally wherein the parasitic disease is:

(a) a helminth disease; optionally wherein the helminth disease is a filarial disease; or
(b) a protozoal disease.

**9.** The pharmaceutical composition according to any one of claims 1 to 5 for use in a method of treating a disease treatable by impairment or reversion of epithelial-mesenchymal transition (EMT), wherein the disease treatable by impairment or reversion of EMT is:

(a) cancer, optionally wherein the cancer is a solid tumour or a haematological cancer; and/or a proliferative disease;
(b) an inflammatory disease; optionally wherein the inflammatory disease is:

(i) an autoimmune disease; or
(ii) gout; or

(c) a fibrotic disease.

**10.** The pharmaceutical composition according to any one of claims 1 to 5 for use in a method of treating:

(a) a neurodegenerative disease; optionally wherein the neurodegenerative disease is Alzheimer's disease;
(b) liver disease;

(c) a spinal cord injury; or
(d) myopathy.

**11.** The pharmaceutical composition for use according to any one of claims 6 to 10, wherein the method comprises administering flubendazole or a pharmaceutically acceptable salt, solvate, hydrate or *N*-oxide thereof to a subject concurrently, separately or sequentially with the moderate or strong CYP1A2 inhibitor.

**12.** The pharmaceutical composition for use according to claim 7 or claim 9, wherein the cancer is a solid cancer, or a haematological cancer, optionally wherein the cancer is selected from Bronchial Tumors, Central Nervous System Cancer, Central Nervous System Embryonal Tumors, Carcinoma, Acute Myeloid Leukemia (AML), Carcinoid Tumor, Appendix Cancer, Astrocytomas, Chordoma, Atypical Teratoid/Rhabdoid Tumor, Sarcoma, Bladder Cancer, Thyroid Cancer, Primary Central Nervous System (CNS) Lymphoma, Extracranial Germ Cell Tumor, Esophageal Cancer, AIDS-Related Cancers, Hepatocellular (Liver) Cancer, Penile Cancer, Pleuropulmonary Blastoma, Gallbladder Cancer, Rhabdomyosarcoma, Waldenstrom Macroglobulinemia, Salivary Gland Cancer, Central Nervous System Germ Cell Tumors, Plasma Cell Neoplasm, Lip and Oral Cavity Cancer, Testicular Cancer, Extrahepatic Bile Duct Cancer, Ductal Carcinoma In Situ (DCIS), Nasopharyngeal Cancer, Nasal Cavity and Paranasal Sinus Cancer, Bone Cancer, Breast Cancer, Glioma, Hairy Cell Leukemia, Langerhans Cell Histiocytosis, Oral Cancer, Ependymoma, Cutaneous T-Cell Lymphoma, Gestational Trophoblastic Disease, Eye Cancer, Kaposi Sarcoma, Extragonadal Germ Cell Tumor, Gastric (Stomach) Cancer, Gastrointestinal Stromal Tumors (GIST), Papillomatosis, Small Intestine Cancer, Brain and Spinal Cord Tumors, Waldenstrom Macroglobulinemia, Pancreatic Cancer, Pharyngeal Cancer, Oropharyngeal Cancer, Paraganglioma, Nonmelanoma Skin Cancer, Myelodysplastic/Myeloproliferative Neoplasms, Squamous Cell Carcinoma, Malignant Fibrous Histiocytoma, Melanoma, Sezary Syndrome, Merkel Cell Carcinoma, Pituitary Tumor, Malignant Fibrous Histiocytoma of Bone and Osteosarcoma, Ovarian Cancer, Parathyroid Cancer, Skin Cancer, Mycosis Fungoides, Germ Cell Tumor, Fallopian Tube Cancer, Intraocular Melanoma, Leukemia, Pancreatic Neuroendocrine Tumors (Islet Cell Tumors), Endometrial Cancer, Lymphoma, Prostate Cancer, Renal Pelvis and Ureter Cancer, Osteosarcoma (Bone Cancer), Non-Hodgkin Lymphoma, Non-Small Cell Lung Cancer, Basal Cell Carcinoma, Laryngeal Cancer, Multiple Myeloma/Plasma Cell Neoplasm, Vaginal Cancer, Squamous Neck Cancer, Multiple Myeloma, Midline Tract Carcinoma Involving NUT Gene, Head and Neck Cancer, Heart Cancer, Intraocular (Eye), Renal Cell (Kidney) Cancer, Malignant Fibrous Histiocytoma of Bone, Liver Cancer, Rectal Cancer, Colon Cancer, Malignant Mesothelioma, Low Malignant Potential Tumor, Mouth Cancer, Soft Tissue Sarcoma, Hypopharyngeal Cancer, Wilms Tumor, Epithelial Cancer, Ewing Sarcoma Family of Tumors, Acute Lymphoblastic Leukemia (ALL), Retinoblastoma, Hodgkin Lymphoma, Brain Tumor/Cancer, Esthesioneuroblastoma, Embryonal Tumors, Cervical Cancer, Chronic Myeloproliferative Neoplasms, Pancreatic Neuroendocrine Tumors, Ureter and Renal Pelvis Cancer, Anal Cancer, Urethral Cancer, Brain Stem Cancer, Vulvar Cancer, Chronic Lymphocytic Leukemia (CLL), Uterine Sarcoma, Stomach (Gastric) Cancer, Brain Stem Glioma, Multiple Endocrine Neoplasia Syndromes, Myelodysplastic Syndromes, Craniopharyngioma, Small Cell Lung Cancer, Lip and Oral Cavity Cancer, Cutaneous T-Cell Lymphoma, Neuroblastoma, Acute Lymphoblastic Leukemia (ALL), Langerhans Cell Histiocytosis, Breast Cancer, Gastrointestinal Carcinoid Tumor, Paranasal Sinus and Nasal Cavity Cancer, Pheochromocytoma, Metastatic Squamous Neck Cancer with Occult Primary, Male Breast Cancer, Kidney (Renal) Cancer, Lung Cancer, Islet Cell Tumors, Extrahepatic Bile Duct Cancer, Endometrial Uterine Cancer, Chronic Myeloproliferative Neoplasms, Transitional Cell Cancer of the Renal Pelvis and Ureter, Thymoma and Thymic Carcinoma, Throat Cancer, Ewing Sarcoma, Chronic Myelogenous Leukemia (CML), Colorectal Cancer, Colon Cancer, Cardiac (Heart) Tumors, Burkitt Lymphoma, Carcinoma of Unknown Primary, Central Nervous System Atypical Teratoid/Rhabdoid Tumor, Childhood Cancers, and Non-Hodgkin Lymphoma, Adrenocortical Carcinoma, Adrenocortical Adenocarcinoma, Adrenocortical Adenoma, Kidney (Renal) Cancer, and P-gp expressing Multidrug Resistant Tumour.

**13.** The pharmaceutical composition for use according to claim 7 or claim 8, wherein the fungal disease or parasitic disease is selected from helminth diseases and protozoal diseases, optionally selected from filarial diseases, onchocerciasis, hookworm, echinococcosis diseases, ascariasis, and enterobiasis, *Acanthocephalans*, *Plasmodium* spp., African trypanosomes, *Trypanosoma cruzi, Leishmania* spp., *Giardia* spp., *Trichomonas vaginalis, Entamoeba histolytica, Encephalitozoon* spp., *Acanthamoeba castellani*, and *Enterocytozoon bieneusi,* and fungal diseases, including *Cryptococcus neoformans* and other *Cryptococcus* species.

**14.** The pharmaceutical composition for use according to any one of claims 6 to 13, wherein the method comprises:

(a) administering the composition to a human; and/or
(b) administering the pharmaceutical composition orally, intravenously, intramuscularly or subcutaneously.

## Patentansprüche

1. Eine pharmazeutische Zusammensetzung, die Folgendes beinhaltet: Flubendazol oder ein pharmazeutisch akzeptables Salz, Solvat, Hydrat oder N-Oxid davon und einen mittelstarken oder starken Cytochrom-P450-1A2-Isoenzym(CYP1A2)-Hemmer, wobei:

   (a) der mittelstarke CYP1A2-Hemmer Folgendes ist:

      (i) ein CYP1A2-Hemmer, der die intrinsische Clearance von Flubendazol in menschlichen Leberzellen ex vivo gegenüber der intrinsischen Clearance von Flubendazol in menschlichen Leberzellen ex vivo bei Abwesenheit eines CYP1A2-Hemmers um ≥ das Zweifache bis < das Fünffache reduziert; und/oder
      (ii) ein CYP1A2-Hemmer, der die Fläche unter der Kurve (AUC) in vivo von Flubendazol gegenüber der AUC in vivo von Flubendazol bei Abwesenheit eines CYP1A2-Hemmers um ≥ das Zweifache bis < das Fünffache erhöht; und

   (b) der starke CYP1A2-Hemmer Folgendes ist:

      (i) ein CYP1A2-Hemmer, der die intrinsische Clearance von Flubendazol in menschlichen Leberzellen ex vivo gegenüber der intrinsischen Clearance von Flubendazol in menschlichen Leberzellen ex vivo bei Abwesenheit eines CYP1A2-Hemmers um ≥ das Fünffache reduziert; und/oder
      (ii) ein CYP1A2-Hemmer, der die AUC in vivo von Flubendazol gegenüber der AUC in vivo von Flubendazol bei Abwesenheit eines CYP1A2-Hemmers um ≥ das Fünffache erhöht.

2. Pharmazeutische Zusammensetzung nach Anspruch 1, wobei der mittelstarke oder starke CYP1A2-Hemmer aus Furafyllin, Ciprofloxacin, Enoxacin, Fluvoxamin, Zafirlukast, 8-Phenyltheophyllin, Methoxsalen, Thiabendazol, Mexiletin und Cimetidin oder einem pharmazeutisch akzeptablen Salz, Solvat, Hydrat oder N-Oxid davon ausgewählt ist.

3. Pharmazeutische Zusammensetzung nach Anspruch 1 oder Anspruch 2, wobei der starke CYP1A2-Hemmer aus Fluvoxamin, Thiabendazol, Furafyllin, Mexiletin, 8-Phenyltheophyllin, Ciprofloxacin, Enoxacin und Zafirlukast oder einem pharmazeutisch akzeptablen Salz, Solvat, Hydrat oder N-Oxid davon ausgewählt ist; wobei der starke CYP1A2-Hemmer optional aus Fluvoxamin, Thiabendazol, Furafyllin und Mexiletin oder einem pharmazeutisch akzeptablen Salz, Solvat, Hydrat oder N-Oxid davon ausgewählt ist.

4. Pharmazeutische Zusammensetzung nach einem der vorhergehenden Ansprüche, wobei der mittelstarke CYP1A2-Hemmer aus Cimetidin und Methoxsalen ausgewählt ist.

5. Pharmazeutische Zusammensetzung nach einem der vorhergehenden Ansprüche, wobei der mittelstarke oder starke CYP1A2-Hemmer die In-vivo-Halbwertzeit von Flubendazol um mehr als das 2-, 3-, 4-, 6-, 8-, 10-, 12-, 14-, 16-, 18- oder 20-Fache der In-vivo-Halbwertzeit von Flubendazol bei Abwesenheit des mittelstarken oder starken CYP1A2-Hemmers verlängert.

6. Pharmazeutische Zusammensetzung nach einem der vorhergehenden Ansprüche zur Verwendung in der Medizin.

7. Pharmazeutische Zusammensetzung nach einem der vorhergehenden Ansprüche zur Verwendung in einem Verfahren zum Behandeln einer durch Hemmung und/oder Störung der Struktur und Funktion der Mikrotubuli behandelbaren Erkrankung, wobei die durch Hemmung und/oder Störung der Struktur und Funktion der Mikrotubuli behandelbare Erkrankung Folgende ist:

   (a) Krebs, wobei der Krebs optional Folgendes ist: ein Blutkrebs oder ein solider Tumor; und/oder eine proliferative Erkrankung;
   (b) eine infektiöse Erkrankung; wobei die infektiöse Erkrankung optional eine bakterielle Erkrankung oder eine virale Erkrankung ist; wobei die virale Erkrankung optional eine HIV-Infektion ist;
   (c) eine Pilzerkrankung;
   (d) eine entzündliche Erkrankung; wobei die entzündliche Erkrankung optional Folgendes ist:

      (i) eine Autoimmunkrankheit; oder
      (ii) Gicht;
      oder

(e) eine fibrotische Erkrankung.

**8.** Pharmazeutische Zusammensetzung zur Verwendung nach Anspruch 7, wobei die infektiöse Erkrankung eine parasitäre Erkrankung ist; wobei die parasitäre Erkrankung optional Folgende ist:

(a) eine Helminthen-Erkrankung; wobei die Helminthen-Erkrankung optional eine Filaria-Erkrankung ist; oder
(b) eine Protozoen-Erkrankung.

**9.** Pharmazeutische Zusammensetzung nach einem der Ansprüche 1 bis 5 zur Verwendung in einem Verfahren zum Behandeln einer durch Schädigung oder Reversion der epithelial-mesenchymalen Transition (EMT) behandelbaren Erkrankung, wobei die durch Schädigung oder Reversion der EMT behandelbare Erkrankung Folgende ist:

(a) Krebs, wobei der Krebs optional Folgendes ist: ein solider Tumor oder ein Blutkrebs; und/oder eine proliferative Erkrankung;
(b) eine entzündliche Erkrankung; wobei die entzündliche Erkrankung optional Folgende ist:

(i) eine Autoimmunkrankheit; oder
(ii) Gicht; oder

(c) eine fibrotische Erkrankung.

**10.** Pharmazeutische Zusammensetzung nach einem der Ansprüche 1 bis 5 zur Verwendung in einem Verfahren zum Behandeln:

(a) einer neurodegenerativen Erkrankung; wobei die neurodegenerative Erkrankung optional Alzheimer-Krankheit ist;
(b) einer Lebererkrankung;
(c) einer Verletzung des Rückenmarks; oder
(d) einer Myopathie.

**11.** Pharmazeutische Zusammensetzung zur Verwendung nach einem der Ansprüche 6 bis 10, wobei das Verfahren das Verabreichen von Flubendazol oder eines pharmazeutisch akzeptablen Salzes, Solvats, Hydrats oder *N*-Oxids davon gleichzeitig mit, separat von oder im Anschluss an die Verabreichung des mittelstarken oder starken CYP1A2-Hemmers an ein Individuum beinhaltet.

**12.** Pharmazeutische Zusammensetzung zur Verwendung nach Anspruch 7 oder Anspruch 9, wobei der Krebs ein solider Krebs oder ein Blutkrebs ist, wobei der Krebs optional aus Folgenden ausgewählt ist: Bronchialtumoren, Krebs des Zentralnervensystems, Embryonaltumoren des Zentralnervensystems, Karzinomen, akuter myeloischer Leukämie (AML), Karzinoidtumor, Appendixkrebs, Astrozytomen, Chordom, atypischem Teratoid-/Rhabdoid-Tumor, Sarkom, Blasenkrebs, Schilddrüsenkrebs, primärem Lymphom des Zentralnervensystems (ZNS), extrakraniellem Keimzelltumor, Speiseröhrenkrebs, Krebserkrankungen in Zusammenhang mit AIDS, Leberzellkrebs (Leberkrebs), Peniskrebs, pleuropulmonalem Blastom, Gallenblasenkrebs, Rhabdomyosarkom, Waldenström-Makroglobulinämie, Speicheldrüsenkrebs, Keimzelltumoren des Zentralnervensystems, Plasmazellneoplasie, Krebs der Lippen und Mundhöhle, Hodenkrebs, extrahepatischem Gallengangkrebs, duktalem Carcinoma in situ (DCIS), Nasopharynxkrebs, Krebs der Nasenhöhle und der Nasennebenhöhlen, Knochenkrebs, Brustkrebs, Gliom, Haarzellleukämie, Langerhans-Zell-Histiozytose, Mundkrebs, Ependymom, kutanem T-Zell-Lymphom, gestationsbedingter trophoblastärer Erkrankung, Augenkrebs, Kaposi-Sarkom, extragonadalem Keimzelltumor, Magenkrebs, gastrointestinalen Stromatumoren (GIST), Papillomatose, Dünndarmkrebs, Tumoren des Gehirns und Rückenmarks, Waldenström-Makroglobulinämie, Bauchspeicheldrüsenkrebs, Pharynxkrebs, Oropharynxkrebs, Paragangliom, Nicht-Melanom-Hautkrebs, myelodysplastischen/myeloproliferativen Neoplasien, Plattenepithelkarzinom, malignem fibrösem Histiozytom, Melanom, Sézary-Syndrom, Merkel-Zellkarzinom, Hypophysentumor, malignem fibrösem Histiozytom der Knochen und Osteosarkom, Eierstockkrebs, Nebenschilddrüsenkrebs, Hautkrebs, Mycosis fungoides, Keimzelltumor, Eileiterkrebs, intraokularem Melanom, Leukämie, neuroendokrinen Tumoren des Pankreas (Inselzelltumoren), Endometriumkrebs, Lymphom, Prostatakrebs, Krebs des Nierenbeckens und Harnleiters, Osteosarkom (Knochenkrebs), Non-Hodgkin-Lymphom, nichtkleinzelligem Lungenkrebs, Basalzellkarzinom, Larynxkrebs, multiplem Myelom/Plasmazellneoplasma, Vaginalkrebs, Plattenepithel-Halskrebs, multiplem Myelom, Midline-Tract-Karzinom unter Beteiligung des NUT-Gens, Kopf- und Hals-Krebs, Herzkrebs, intraokularem Krebs (Augenkrebs), Nierenzellkrebs (Nierenkrebs), malignem fibrösem Histiozytom der Knochen, Leberkrebs, Rektalkrebs, Kolonkrebs, malignem Mesotheliom, Tumor mit geringem malignem Potential, Mundkrebs, Weichteilsarkom, Hypopharynx-Krebs, Wilms-Tumor, Epithelkrebs, Tumoren der Ewing-Sarkom-Familie, akuter lymphatischer Leukämie (ALL), Retinoblastom, Hodgkin-Lymphom, Hirntumor/-krebs, Ästhesioneuroblastom, embryonalen Tumoren, Zervixkrebs, chronischen myeloproliferativen Neoplasien, pankreatischen neuroendokrinen Tumoren, Harnleiter- und Nierenbecken-Krebs, Analkrebs, Urethralkrebs, Krebs des Hirnstamms, Vulvakrebs, chronischer lymphatischer Leukämie (CLL), Uterussarkom, Magenkrebs, Hirnstammgliom, multiplen endokrinen Neoplasiesyndromen, myelodysplastischen Syndromen, Kraniopharyngeom, kleinzelligem Lungenkrebs, Lippen- und Mundhöhlenkrebs, kutanem T-Zell-Lymphom, Neuroblastom, akuter lymphatischer Leukämie (ALL), Langerhans-Zell-Histiozytose, Brustkrebs, gastrointestinalen Karzinoidtumoren, Krebs der Nasennebenhöhlen und der Nasenhöhle, Phäochromozytom, metastasierender Plattenepithel-Halskrebs mit okkultem Primärtumor, Brustkrebs des Mannes, Nierenkrebs, Lungenkrebs, Inselzelltumoren, extrahepatischem Gallengangkrebs, Endometrium-Gebärmutterkrebs, chronischen myeloproliferativen Neoplasien, Transitionalzellkrebs des Nierenbeckens und Harnleiters, Thymom und Thymuskarzinom, Rachenkrebs, Ewing-Sarkom, chronischer myeloischer Leukämie (CML), Kolorektalkrebs, Kolonkrebs, Herztumoren, Burkitt-Lymphom, Karzinom mit unbekanntem Primärtumor, atypischem Teratoid-/Rhabdoid-Tumor des Zentralnervensystems, Krebserkrankungen bei Kindern und Non-Hodgkin-Lymphom, Nebennierenrindenkarzinom, Nebennierenrinden-Adenokarzinom, Nebennierenrindenadenom, Nierenkrebs und P-gpexprimierendem multiresistentem Tumor.

**13.** Pharmazeutische Zusammensetzung zur Verwendung nach Anspruch 7 oder Anspruch 8, wobei die Pilzerkrankung oder parasitäre Erkrankung aus Folgenden ausgewählt ist: Helminthen-Erkrankungen und Protozoen-Erkrankungen, optional ausgewählt aus Filaria-Erkrankungen, Onchozerkiasis, Hakenwurm, Echinokokkose-Erkrankungen, Askariasis und Enterobiasis, *Acanthocephalans, Plasmodium* spp., afrikanischen Trypanosomen, *Trypanosoma cruzi, Leishmania* spp., *Giardia* spp., *Trichomonas vaginalis, Entamoeba histolytica, Encephalitozoon spp., Acanthamoeba castellani* und *Enterocytozoon bieneusi* und Pilzerkrankungen, einschließlich *Cryptococcus neoformans* und anderen Kryptokokkenspezies.

**14.** Pharmazeutische Zusammensetzung zur Verwendung nach einem der Ansprüche 6 bis 13, wobei das Verfahren Folgendes beinhaltet:

(a) Verabreichen der Zusammensetzung an einen Menschen; und/oder
(b) orales, intravenöses, intramuskuläres oder subkutanes Verabreichen der pharmazeutischen Zusammensetzung.

## Revendications

**1.** Composition pharmaceutique comprenant du flubendazole ou un sel, un solvate, un hydrate ou un N-oxyde pharmaceutiquement acceptable de celui-ci, et un inhibiteur modéré ou fort de l'isoenzyme 1A2 du cytochrome P450 (CYP1A2), dans laquelle :

(a) l'inhibiteur modéré de CYP1A2 est :

(i) un inhibiteur de CYP1A2 qui réduit la clairance intrinsèque du flubendazole dans des hépatocytes humains *ex vivo* de ≥ deux à < cinq fois par rapport à la clairance intrinsèque du flubendazole dans des hépatocytes humains *ex vivo* en l'absence d'inhibiteur de CYP1A2 ; et/ou
(ii) un inhibiteur de CYP1A2 qui augmente l'aire sous la courbe (ASC) *in vivo* du flubendazole de ≥ deux à < cinq fois par rapport à l'ASC *in vivo* du flubendazole en l'absence d'inhibiteur de CYP1A2 ; et

(b) l'inhibiteur fort de CYP1A2 est :

(i) un inhibiteur de CYP1A2 qui réduit la clairance intrinsèque du flubendazole dans des hépatocytes humains *ex vivo* de ≥ cinq fois par rapport à la clairance intrinsèque du flubendazole dans des hépatocytes humains ex *vivo* en l'absence d'inhibiteur de CYP1A2 ; et/ou
(ii) un inhibiteur de CYP1A2 qui augmente l'ASC *in vivo* du flubendazole de ≥ cinq fois par rapport à l'ASC *in vivo* du flubendazole en l'absence d'inhibiteur de CYP1A2.

**2.** Composition pharmaceutique selon la revendication 1, dans laquelle l'inhibiteur modéré ou fort de CYP1A2 est sélectionné parmi la furafylline, la ciprofloxacine, l'énoxacine, la fluvoxamine, le zafirlukast, la 8-phénylthéophylline,

le méthoxsalène, le thiabendazole, la mexilétine et la cimétidine, ou un sel, un solvate, un hydrate ou un *N*-oxyde pharmaceutiquement acceptable de ceux-ci.

**3.** Composition pharmaceutique selon la revendication 1 ou la revendication 2, dans laquelle l'inhibiteur fort de CYP1A2 est sélectionné parmi la fluvoxamine, le thiabendazole, la furafylline, la mexilétine, la 8-phénylthéophylline, la ciprofloxacine, l'énoxacine et le zafirlukast, ou un sel, un solvate, un hydrate ou un N-oxyde pharmaceutiquement acceptable de ceux-ci ; optionnellement dans laquelle l'inhibiteur fort de CYP1A2 est sélectionné parmi la fluvoxamine, le thiabendazole, la furafylline et la mexilétine, ou un sel, un solvate, un hydrate ou un *N*-oxyde pharmaceutiquement acceptable de ceux-ci.

**4.** Composition pharmaceutique selon l'une quelconque des revendications précédentes, dans laquelle l'inhibiteur modéré de CYP1A2 est sélectionné parmi la cimétidine et le méthoxsalène.

**5.** Composition pharmaceutique selon l'une quelconque des revendications précédentes, dans laquelle l'inhibiteur modéré ou fort de CYP1A2 prolonge la demi-vie *in vivo* du flubendazole de plus de 2, 3, 4, 6, 8, 10, 12, 14, 16, 18 ou 20 fois la demi-vie *in vivo* du flubendazole en l'absence de l'inhibiteur modéré ou fort de CYP1A2.

**6.** Composition pharmaceutique selon l'une quelconque des revendications précédentes, destinée à une utilisation en médecine.

**7.** Composition pharmaceutique selon l'une quelconque des revendications précédentes, destinée à une utilisation dans une méthode de traitement d'une maladie traitable par inhibition et/ou perturbation de la structure et de la fonction des microtubules, la maladie traitable par inhibition et/ou perturbation de la structure et de la fonction des microtubules étant :

(a) un cancer, le cancer étant optionnellement un cancer hématologique ou une tumeur solide ; et/ou une maladie proliférative ;
(b) une maladie infectieuse ; la maladie infectieuse étant optionnellement une maladie bactérienne ou une maladie virale ; la maladie virale étant optionnellement une infection par le VIH ;
(c) une maladie fongique ;
(d) une maladie inflammatoire ; la maladie inflammatoire étant optionnellement :

(i) une maladie auto-immune ; ou
(ii) la goutte ;
ou

(e) une maladie fibrotique.

**8.** Composition pharmaceutique destinée à une utilisation selon la revendication 7, la maladie infectieuse étant une maladie parasitaire ; la maladie parasitaire étant optionnellement :

(a) une helminthiase ; l'helminthiase étant optionnellement une filariose ; ou
(b) une maladie à protozoaire.

**9.** Composition pharmaceutique selon l'une quelconque des revendications 1 à 5, destinée à une utilisation dans une méthode de traitement d'une maladie traitable par altération ou réversion de la transition épithéliomésenchymateuse (TEM), la maladie traitable par altération ou réversion de la TEM étant :

(a) un cancer, le cancer étant optionnellement une tumeur solide ou un cancer hématologique ; et/ou une maladie proliférative ;
(b) une maladie inflammatoire ; la maladie inflammatoire étant optionnellement :

(i) une maladie auto-immune ; ou
(ii) la goutte ; ou

(c) une maladie fibrotique.

**10.** Composition pharmaceutique selon l'une quelconque des revendications 1 à 5, destinée à une utilisation dans une

méthode de traitement :

(a) d'une maladie neurodégénérative ; la maladie neurodégénérative étant optionnellement la maladie d'Alzheimer ;
(b) d'une maladie du foie ;
(c) d'une lésion de la moelle épinière ; ou
(d) d'une myopathie.

**11.** Composition pharmaceutique destinée à une utilisation selon l'une quelconque des revendications 6 à 10, la méthode comprenant l'administration de flubendazole ou d'un sel, d'un solvate, d'un hydrate ou d'un N-oxyde pharmaceutiquement acceptable de celui-ci à un sujet en concomitance avec, séparément de, ou séquentiellement avec l'inhibiteur modéré ou fort de CYP1A2.

**12.** Composition pharmaceutique destinée à une utilisation selon la revendication 7 ou la revendication 9, le cancer étant un cancer solide, ou un cancer hématologique, le cancer étant optionnellement sélectionné parmi des tumeurs bronchiques, un cancer du système nerveux central, des tumeurs embryonnaires du système nerveux central, un carcinome, une leucémie aiguë myéloïde (LAM), une tumeur carcinoïde, un cancer de l'appendice, des astrocytomes, un chordome, une tumeur tératoïde/rhabdoïde atypique, un sarcome, un cancer de la vessie, un cancer de la thyroïde, un lymphome primitif du système nerveux central (SNC), une tumeur germinale extracrânienne, un cancer de l'œsophage, des cancers liés au sida, un cancer hépatocellulaire (du foie), un cancer du pénis, un blastome pleuro-pulmonaire, un cancer de la vésicule biliaire, un rhabdomyosarcome, une macroglobulinémie de Waldenström, un cancer des glandes salivaires, des tumeurs germinales du système nerveux central, une tumeur à plasmocytes, un cancer des lèvres et de la cavité buccale, un cancer des testicules, un cancer des conduits biliaires extrahépatiques, un carcinome intracanalaire in situ (CIIS), un cancer rhinopharyngé, un cancer des fosses nasales et des sinus paranasaux, un cancer des os, un cancer du sein, un gliome, une leucémie à tricholeucocytes, une histiocytose à cellules de Langerhans, un cancer de la bouche, un épendymome, un lymphome cutané à cellules T, une maladie trophoblastique gestationnelle, un cancer de l'œil, un sarcome de Kaposi, une tumeur germinale extragonadique, un cancer gastrique (de l'estomac), des tumeurs stromales gastro-intestinales (TSGI), une papillomatose, un cancer de l'intestin grêle, des tumeurs du cerveau et de la moelle épinière, une macroglobulinémie de Waldenström, un cancer du pancréas, un cancer du pharynx, un cancer de l'oropharynx, un paragangliome, un cancer de la peau autre que le mélanome, des néoplasies myélodysplasiques/myéloprolifératives, un carcinome épidermoïde, un histiocytome fibreux malin, un mélanome, un syndrome de Sézary, un carcinome à cellules de Merkel, une tumeur hypophysaire, un histiocytome fibreux malin des os et un ostéosarcome, un cancer de l'ovaire, un cancer de la parathyroïde, un cancer de la peau, une mycose fongoïde, une tumeur germinale, un cancer des trompes de Fallope, un mélanome intraoculaire, une leucémie, des tumeurs neuroendocrines pancréatiques (tumeurs des îlots pancréatiques), un cancer de l'endomètre, un lymphome, un cancer de la prostate, un cancer du pelvis rénal et de l'uretère, un ostéosarcome (cancer des os), un lymphome non hodgkinien, un cancer du poumon non à petites cellules, un carcinome basocellulaire, un cancer du larynx, un myélome multiple/une tumeur à plasmocytes, un cancer du vagin, un cancer épidermoïde du cou, un myélome multiple, un carcinome de la ligne médiane NUT, un cancer de la tête et du cou, un cancer du cœur, un cancer intraoculaire (de l'œil), un cancer des cellules rénales (du rein), un histiocytome fibreux malin des os, un cancer du foie, un cancer du rectum, un cancer du côlon, un mésothéliome malin, une tumeur à faible potentiel malin, un cancer de la bouche, un sarcome des tissus mous, un cancer de l'hypopharynx, une tumeur de Wilms, un cancer épithélial, des tumeurs de la famille du sarcome d'Ewing, une leucémie aiguë lymphoblastique (LAL), un rétinoblastome, un lymphome hodgkinien, une tumeur/un cancer du cerveau, un esthésioneuroblastome, des tumeurs embryonnaires, un cancer du col de l'utérus, des tumeurs myéloprolifératives chroniques, des tumeurs neuroendocrines pancréatiques, un cancer de l'uretère et du pelvis rénal, un cancer anal, un cancer de l'urètre, un cancer du tronc cérébral, un cancer de la vulve, une leucémie chronique lymphoïde (LCL), un sarcome utérin, un cancer de l'estomac (gastrique), un gliome du tronc cérébral, des syndromes néoplasiques endocriniens multiples, des syndromes myélodysplasiques, un craniopharyngiome, un cancer du poumon à petites cellules, un cancer des lèvres et de la cavité buccale, un lymphome cutané à cellules T, un neuroblastome, une leucémie aiguë lymphoblastique (LAL), une histiocytose à cellules de Langerhans, un cancer du sein, une tumeur carcinoïde gastro-intestinale, un cancer des sinus paranasaux et des fosses nasales, un phéochromocytome, un cancer épidermoïde métastatique du cou avec un cancer primitif inconnu, un cancer du sein masculin, un cancer du rein (rénal), un cancer du poumon, des tumeurs des îlots pancréatiques, un cancer des conduits biliaires extrahépatiques, un cancer utérin de l'endomètre, des tumeurs myéloprolifératives chroniques, un cancer des cellules transitionnelles du pelvis rénal et de l'uretère, un thymome et un carcinome thymique, un cancer de la gorge, un sarcome d'Ewing, une leucémie chronique myéloïde (LCM), un cancer colorectal, un cancer du côlon, des tumeurs cardiaques (du cœur), un lymphome de Burkitt, un carcinome lié à un cancer primitif inconnu,

une tumeur tératoïde/rhabdoïde atypique du système nerveux central, des cancers de l'enfance, et un lymphome non hodgkinien, un carcinome corticosurrénalien, un adénocarcinome corticosurrénalien, un adénome corticosurrénalien, un cancer du rein (rénal) et une tumeur multirésistante exprimant P-gp.

**13.** Composition pharmaceutique destinée à une utilisation selon la revendication 7 ou la revendication 8, la maladie fongique ou parasitaire étant sélectionnée parmi des helminthiases et des maladies à protozoaires, optionnellement sélectionnée parmi des filarioses, une onchocercose, une ankylostomose, des échinococcoses, une ascaridiase et une entérobiase, *Acanthocephalans, Plasmodium* spp., des trypanosomes africains, *Trypanosoma cruzi, Leishmania* spp., *Giardia* spp., *Trichomonas vaginalis, Entamoeba histolytica, Encephalitozoon spp., Acanthamoeba castellani,* et *Enterocytozoon bieneusi,* et des maladies fongiques, y compris *Cryptococcus neoformans* et d'autres espèces *Cryptococcus.*

**14.** Composition pharmaceutique destinée à une utilisation selon l'une quelconque des revendications 6 à 13, la méthode comprenant :

(a) l'administration de la composition à un humain ; et/ou
(b) l'administration de la composition pharmaceutique par voie orale, intraveineuse, intramusculaire ou souscutanée.

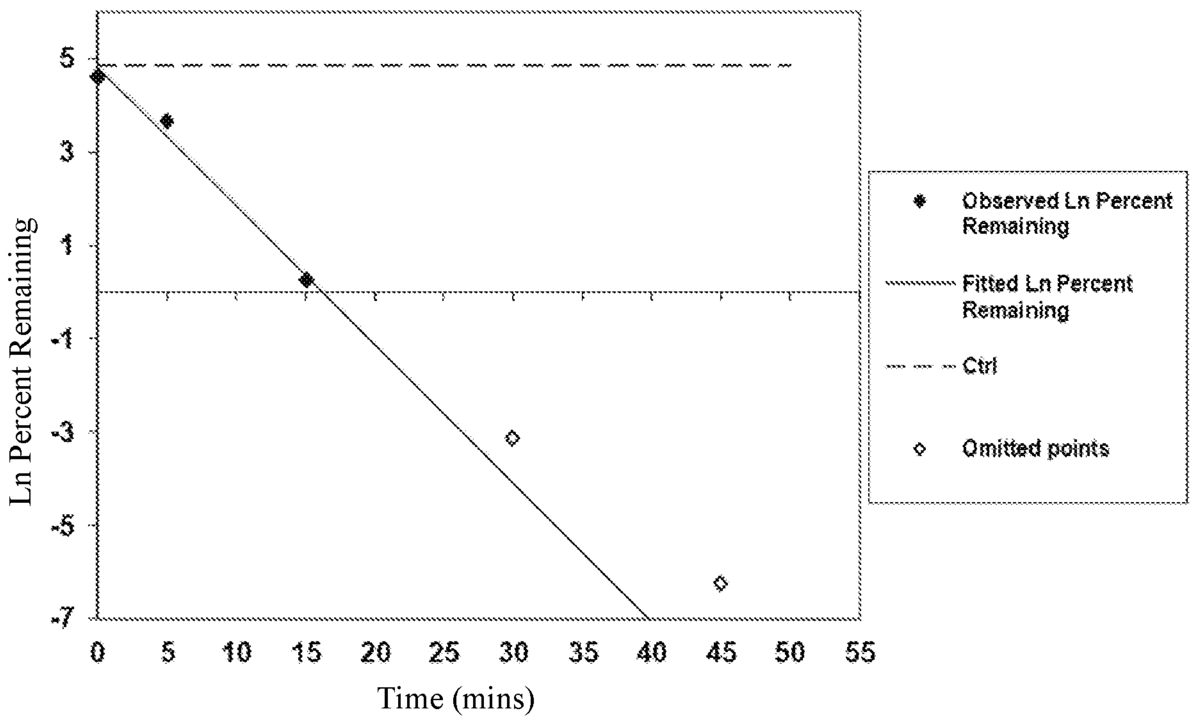


Figure 1

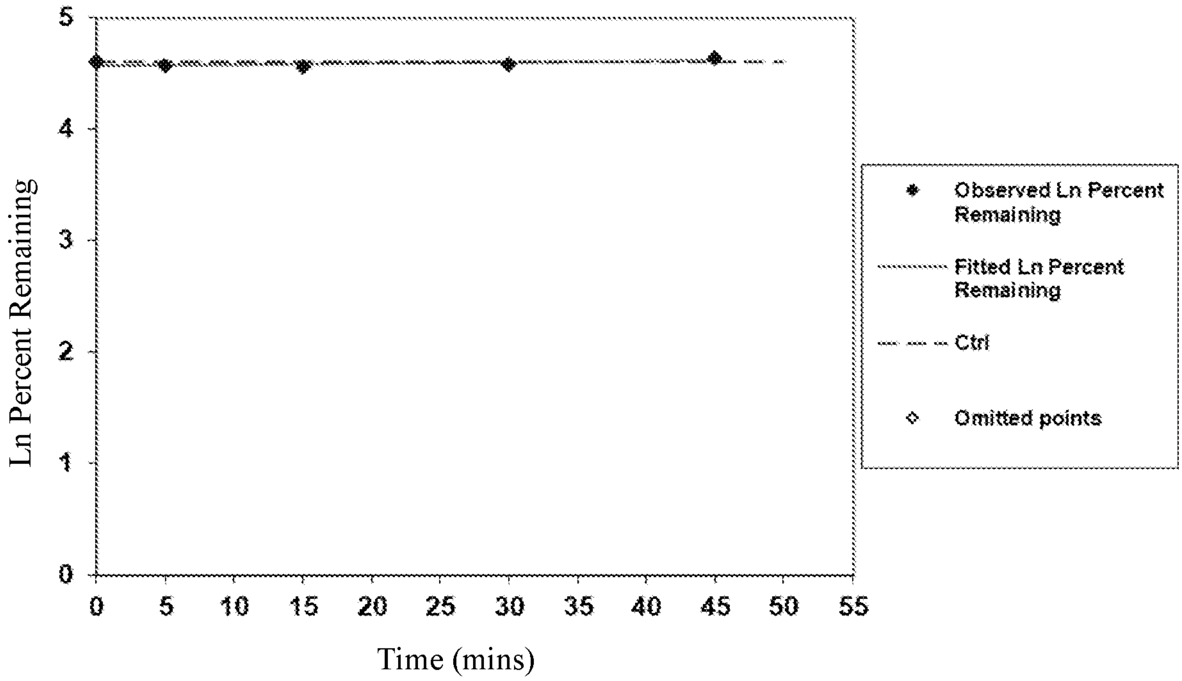


Figure 2

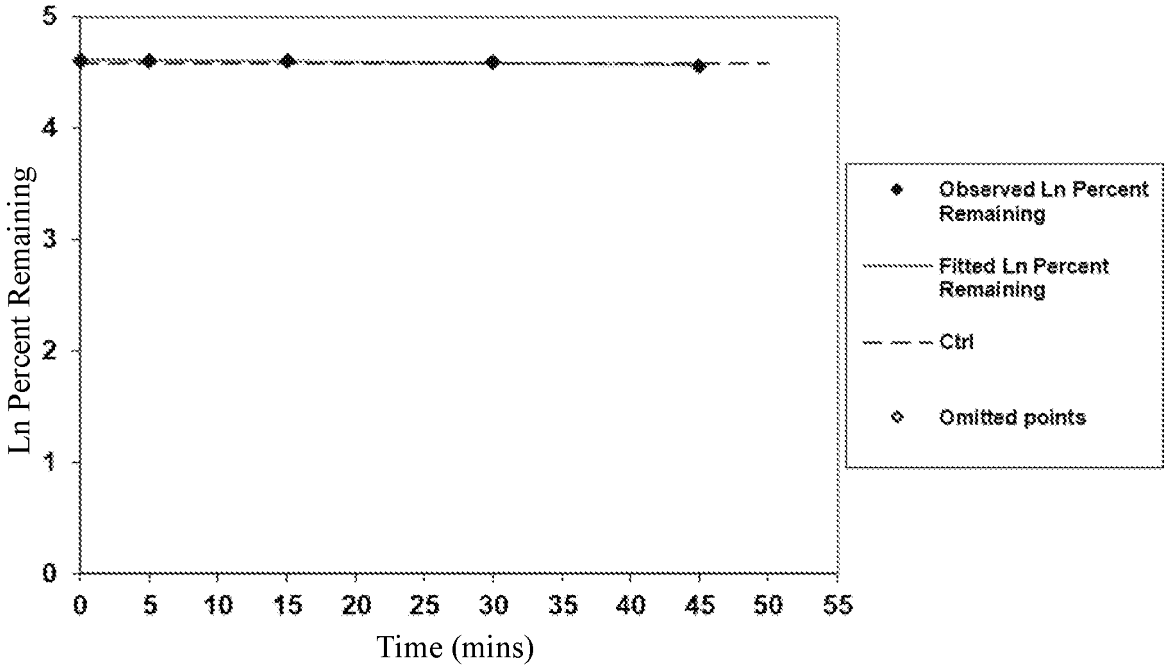


Figure 3

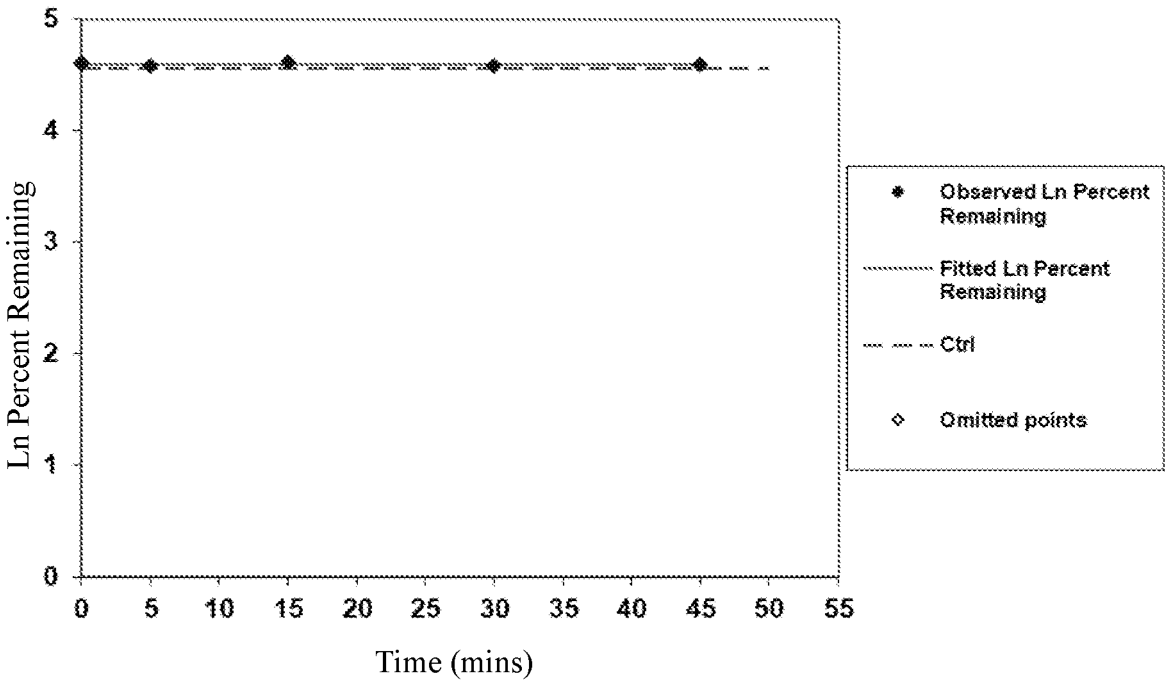


Figure 4

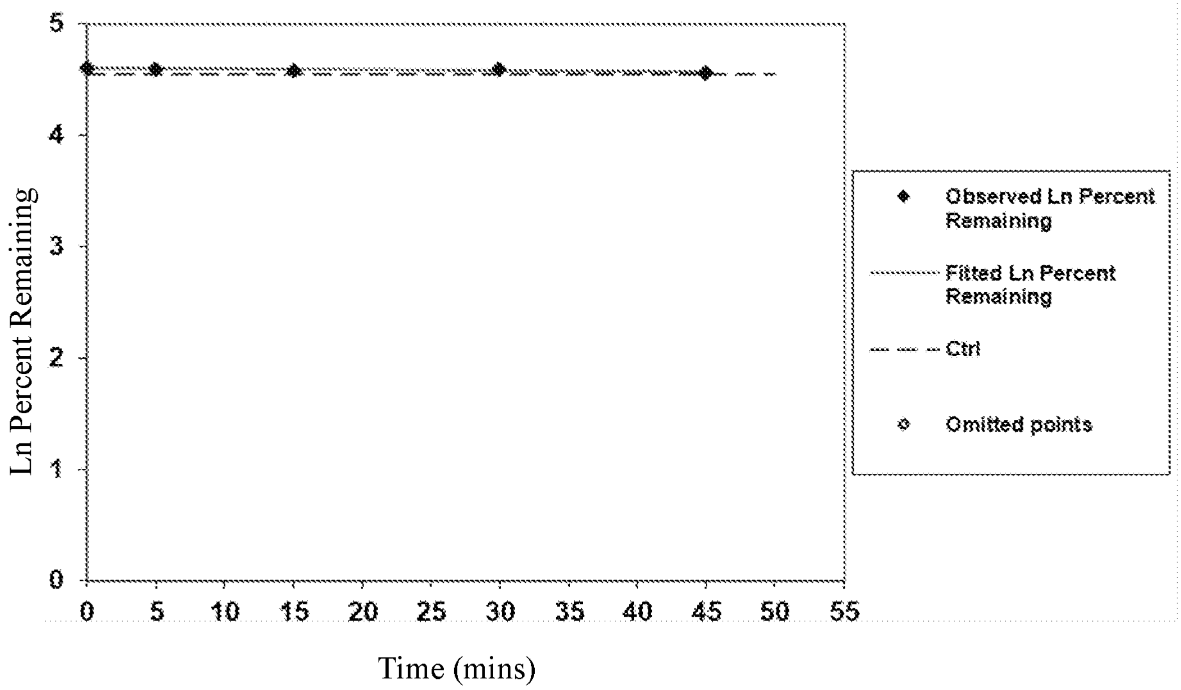


Figure 5

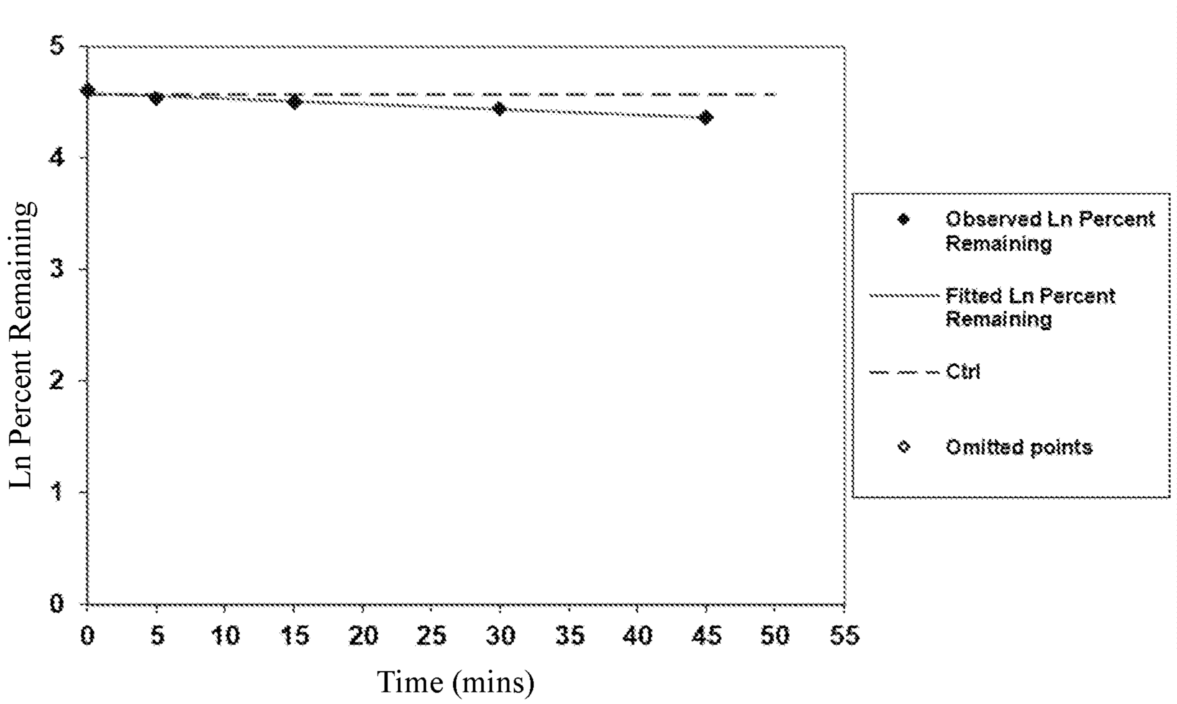


Figure 6

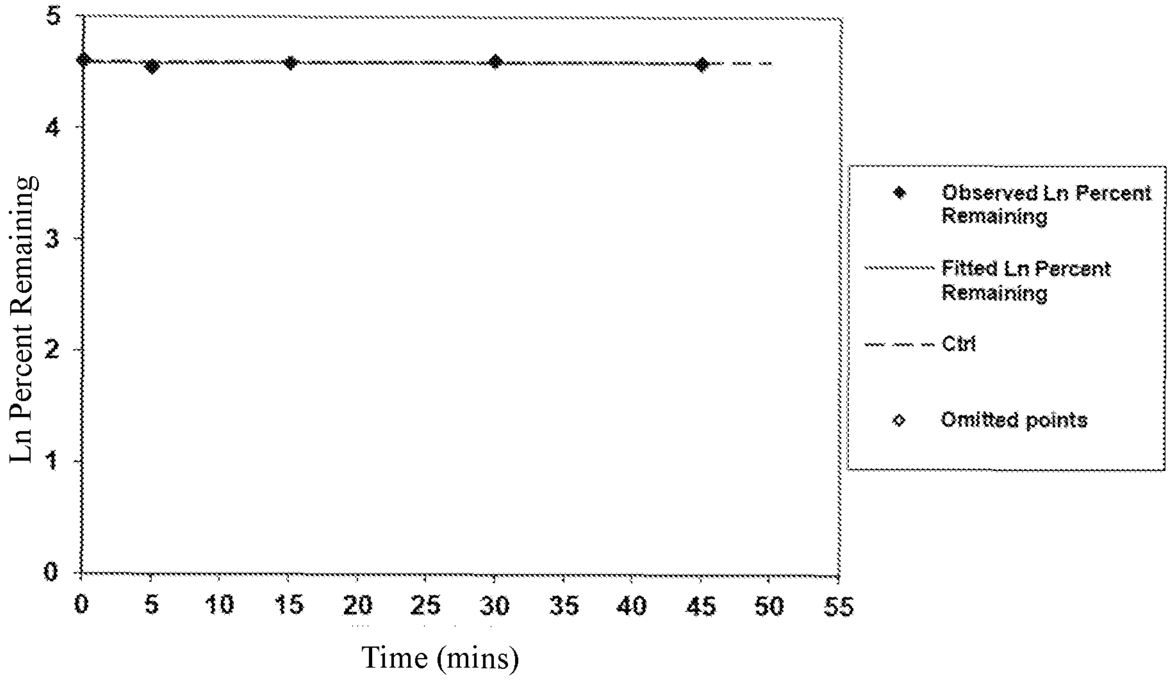


Figure 7

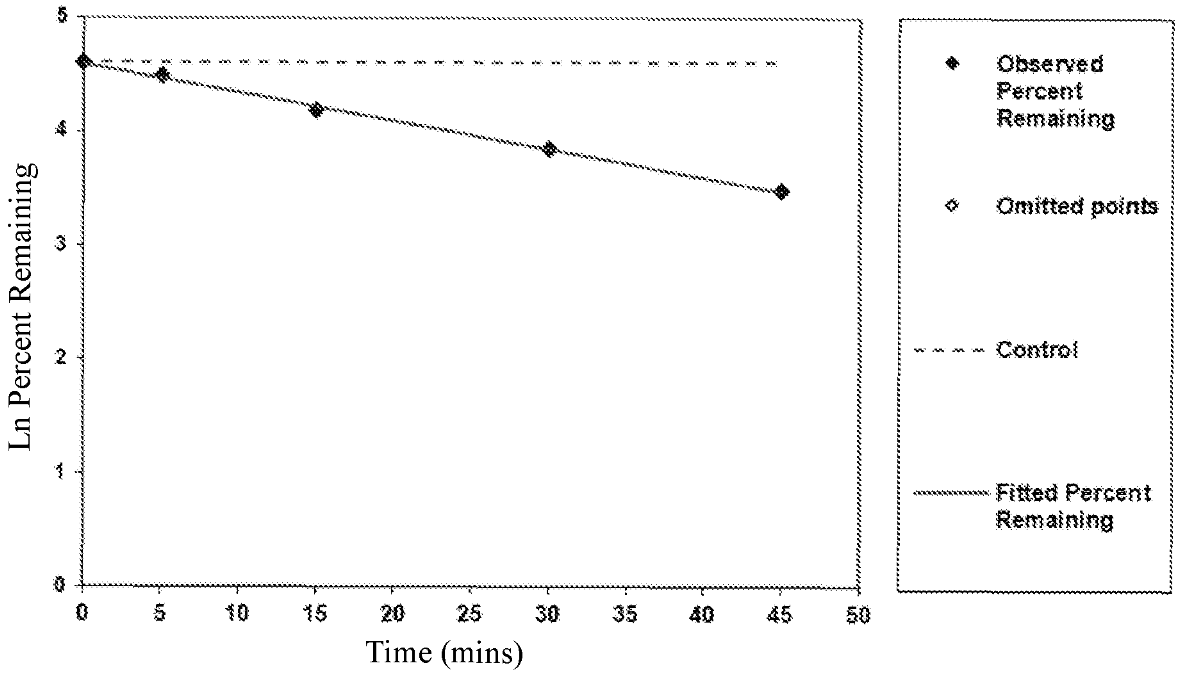


Figure 8

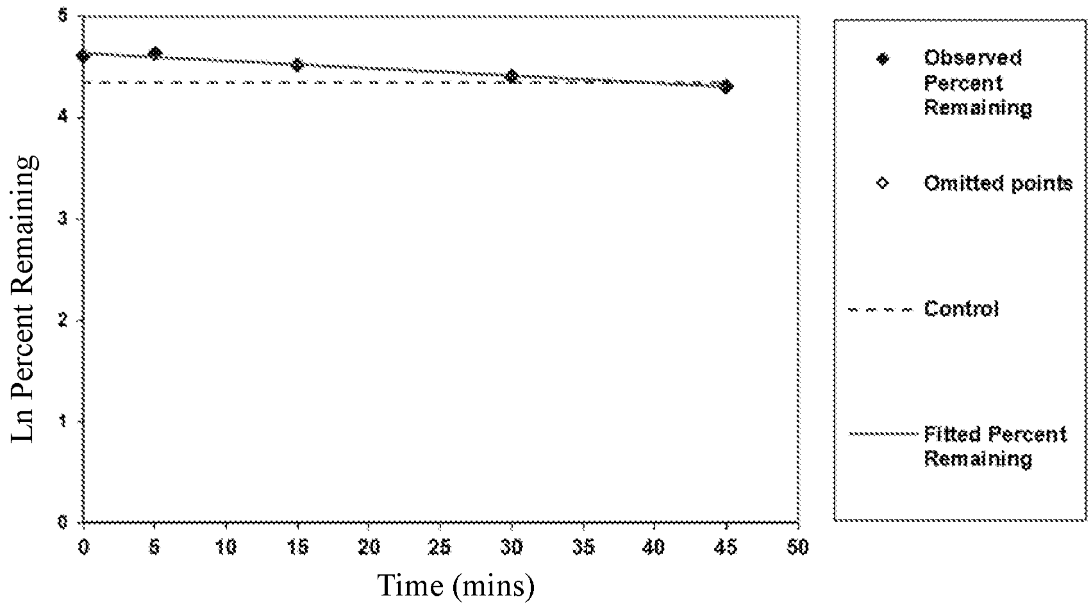


Figure 9

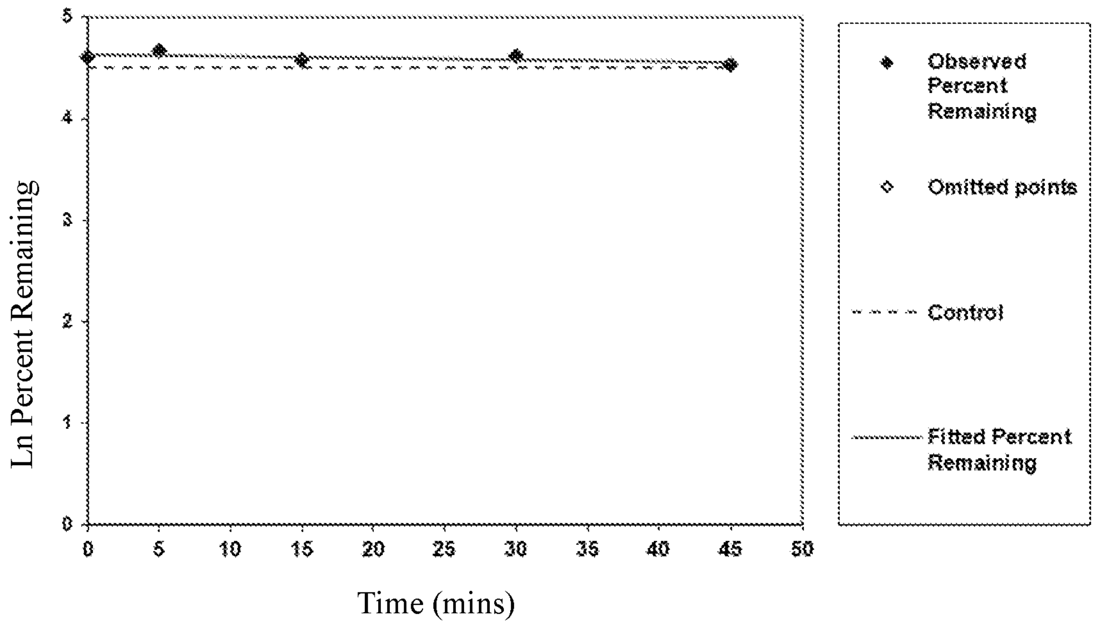


Figure 10

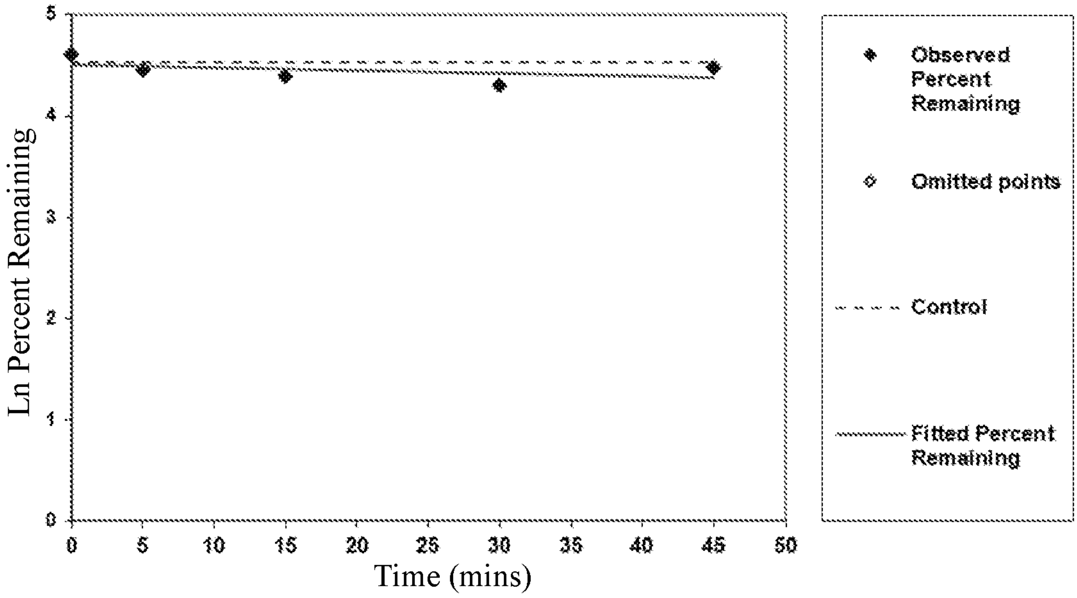


Figure 11

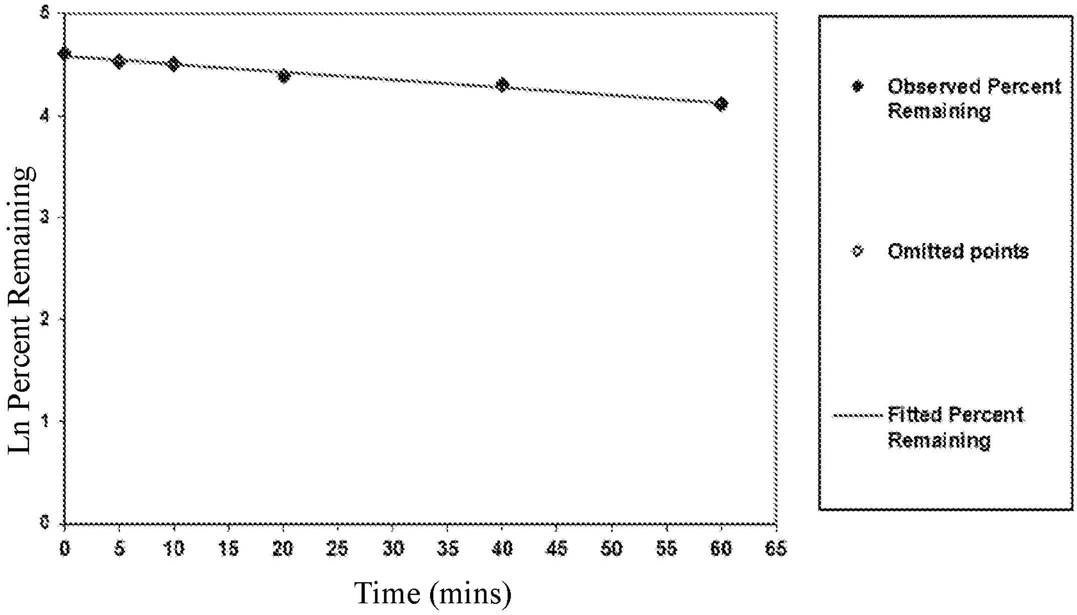


Figure 12

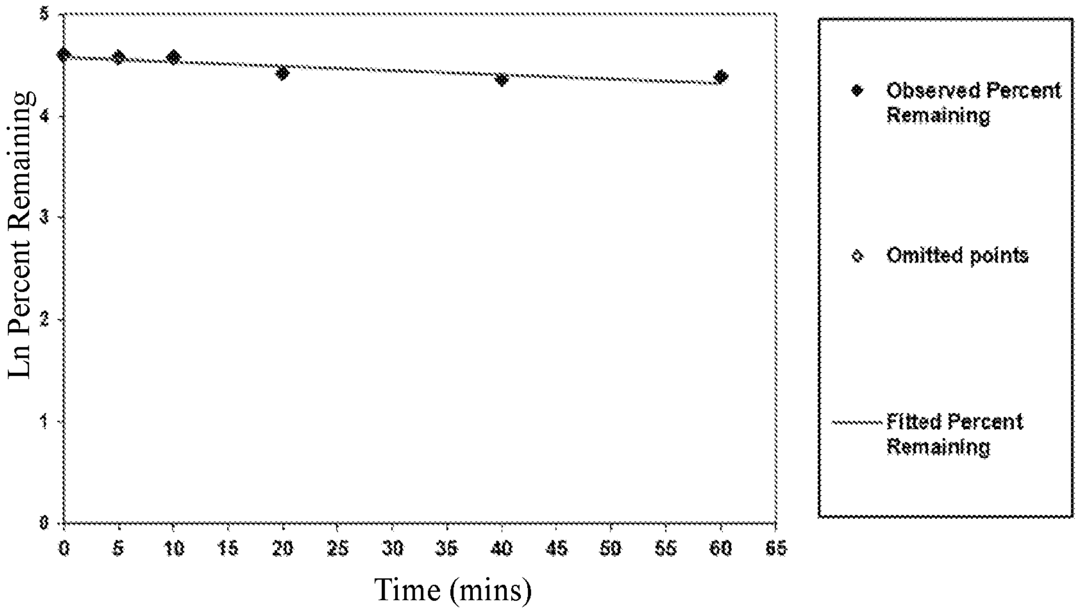


Figure 13

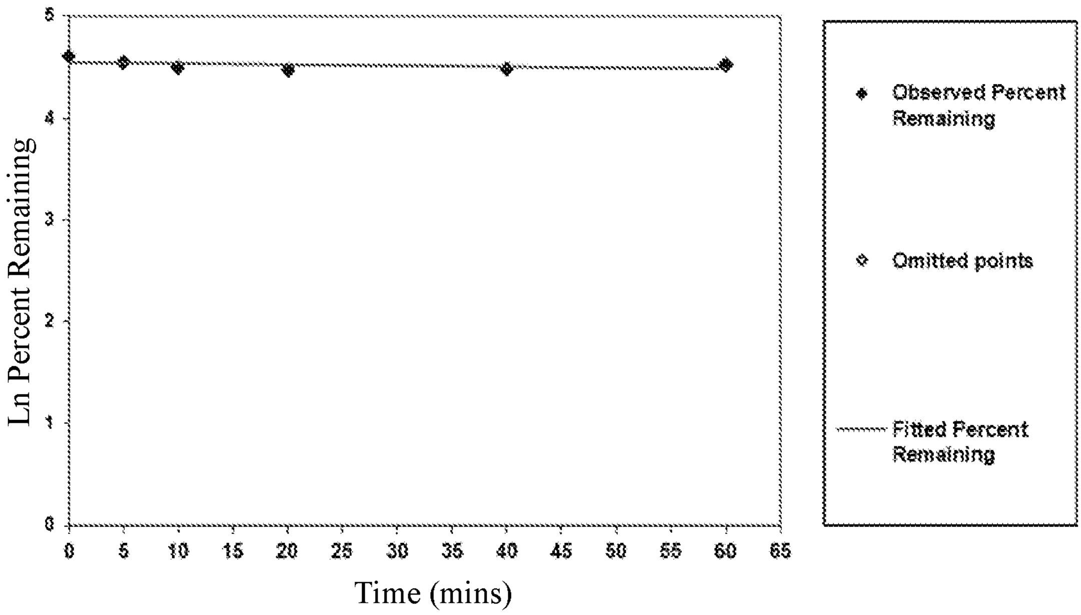


Figure 14

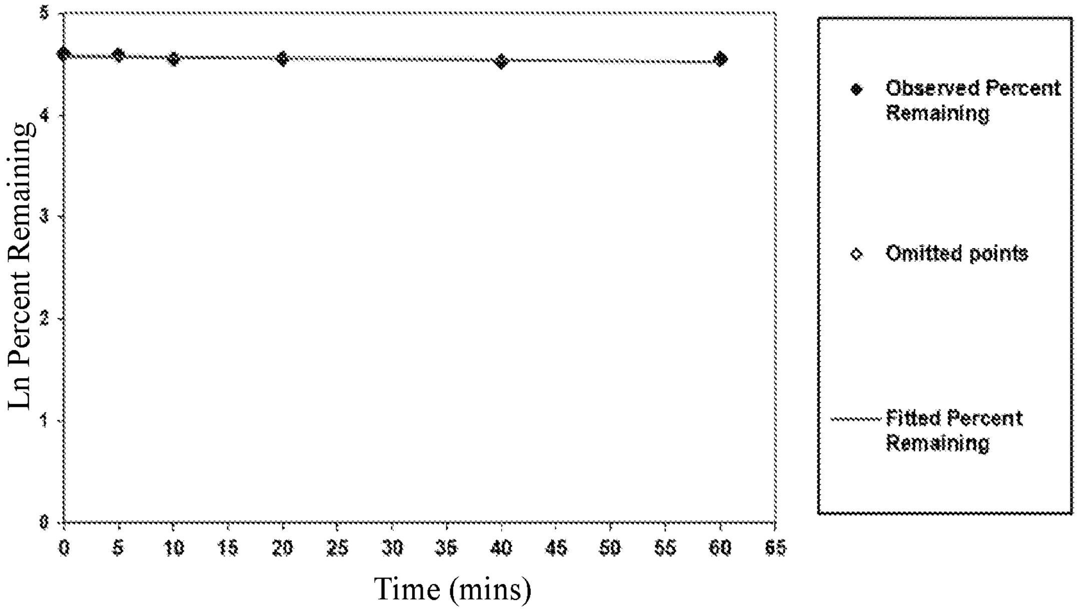


Figure 15

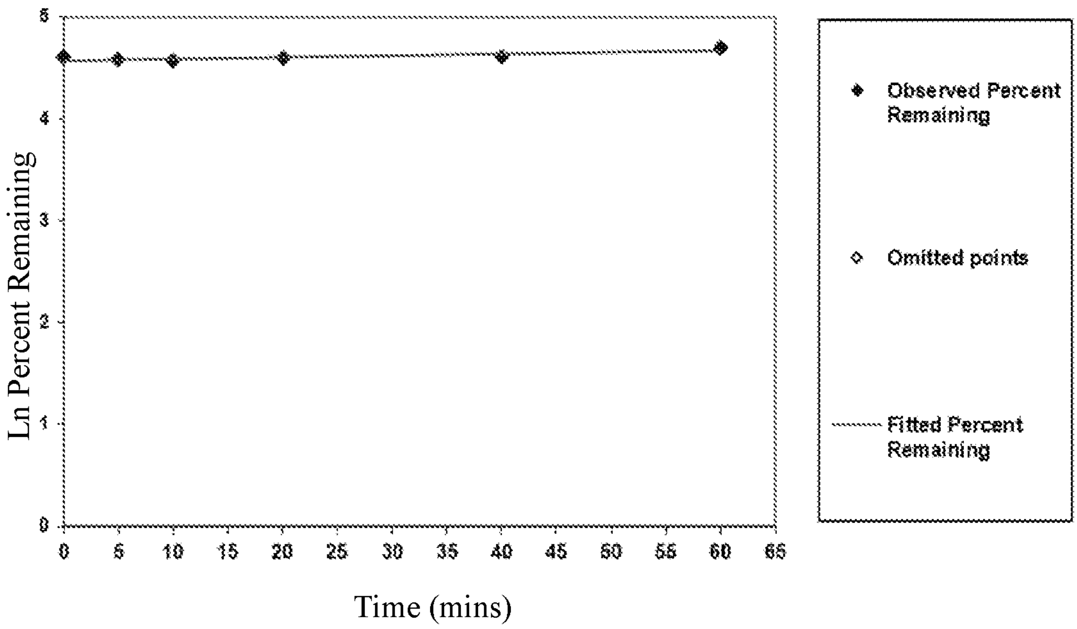


Figure 16

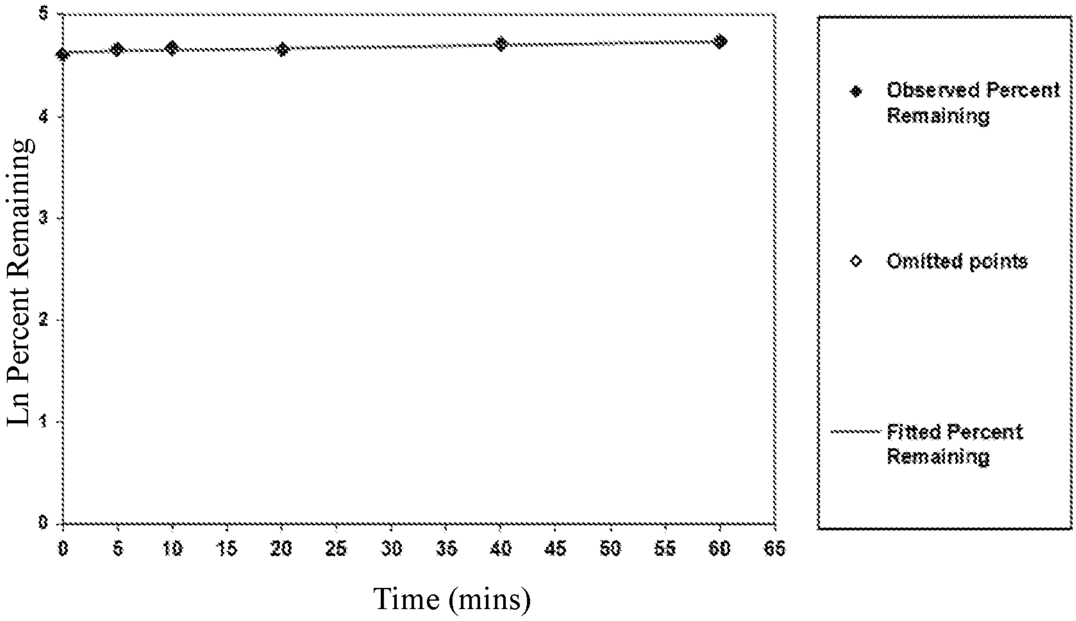


Figure 17

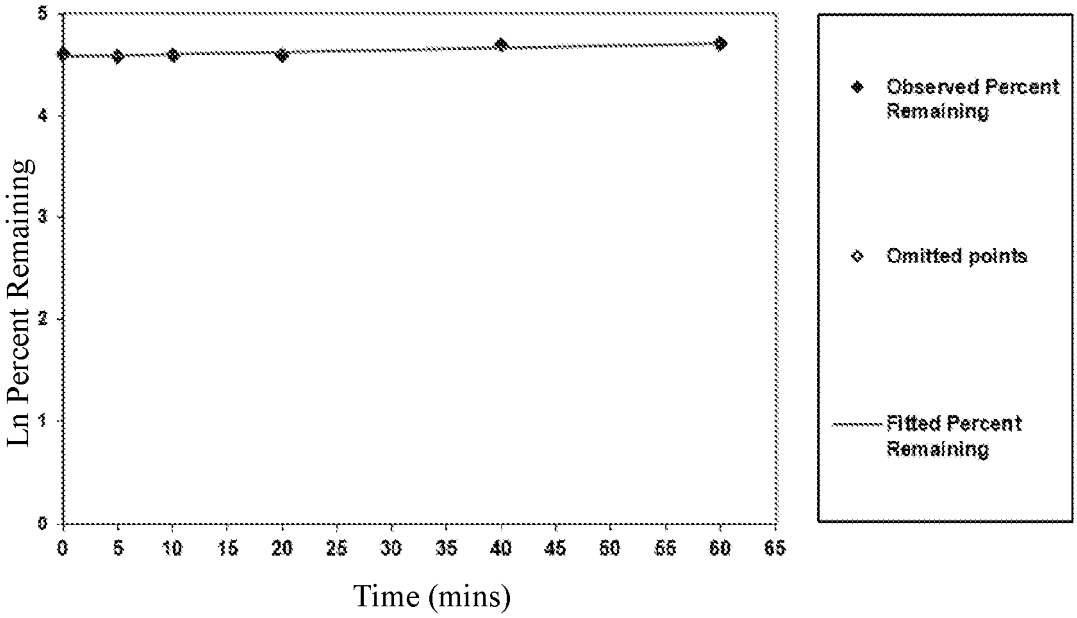


Figure 18

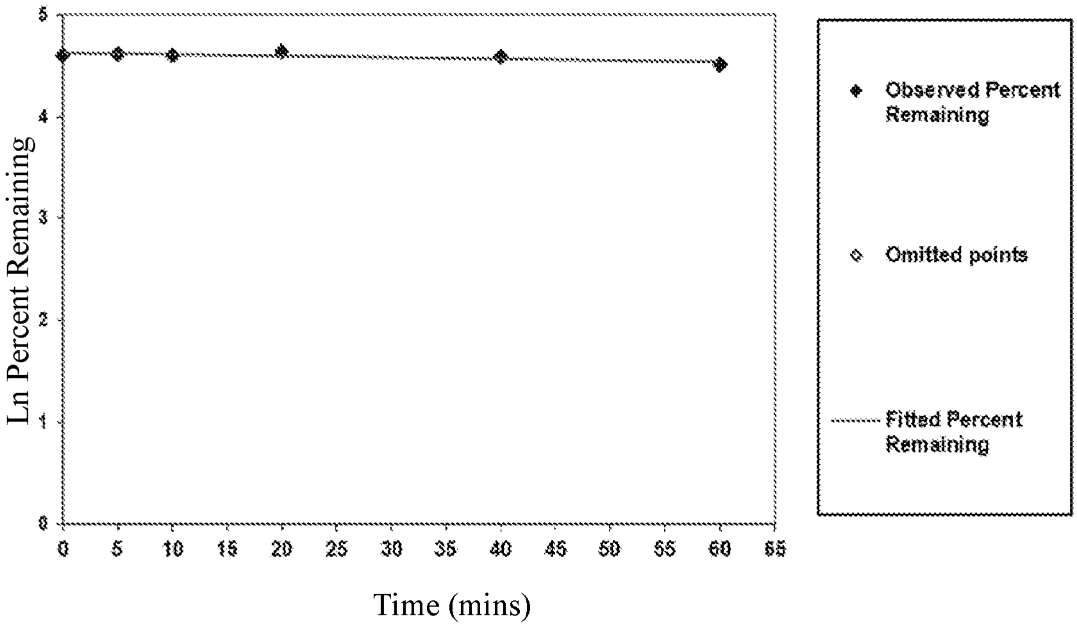


Figure 19

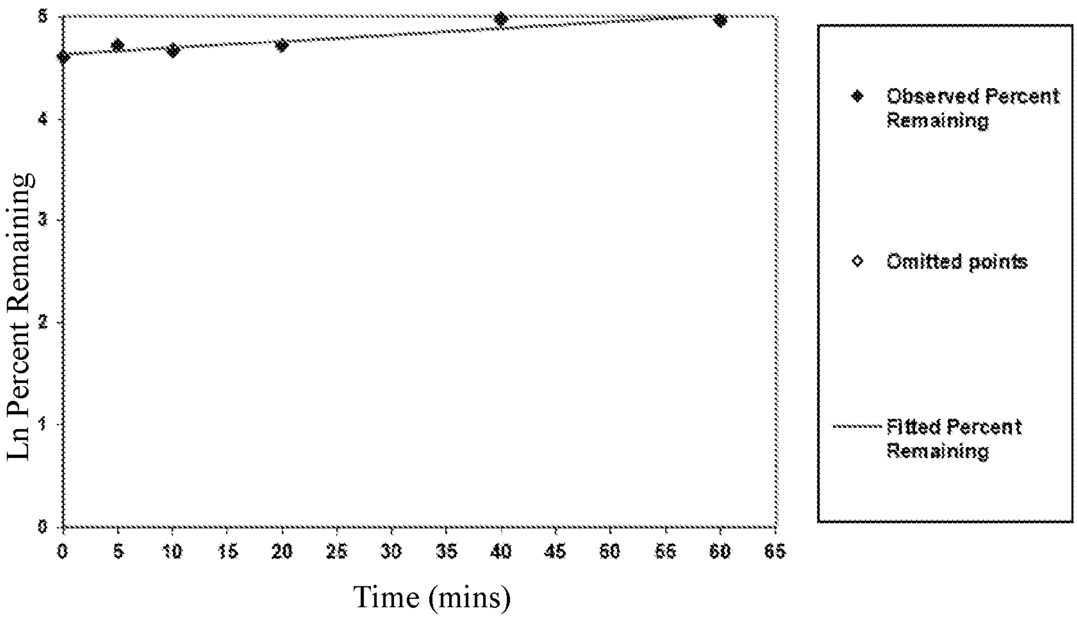


Figure 20

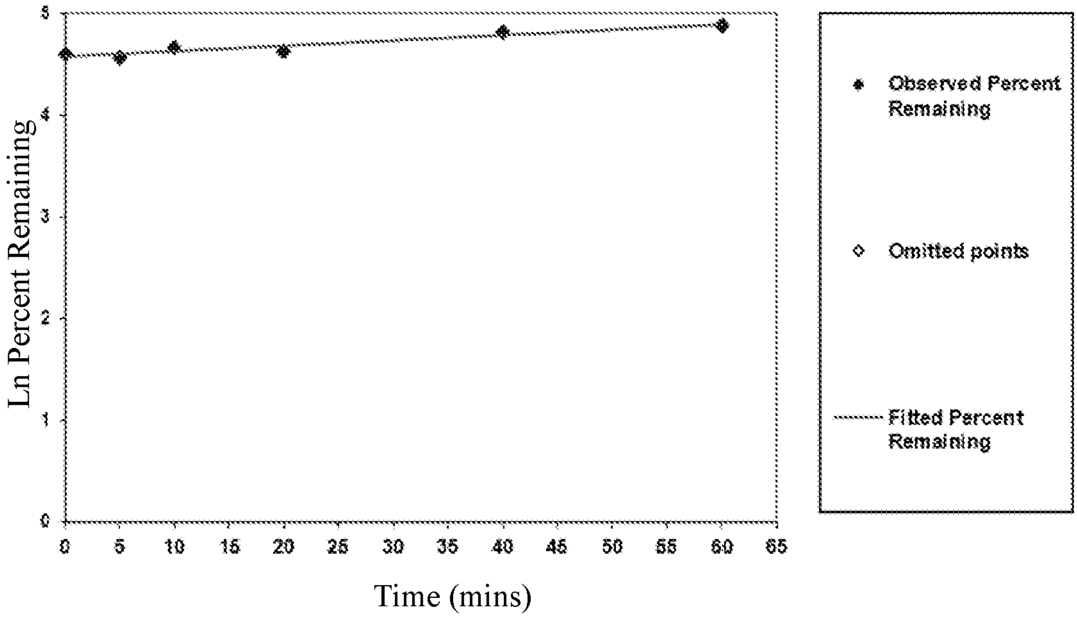


Figure 21

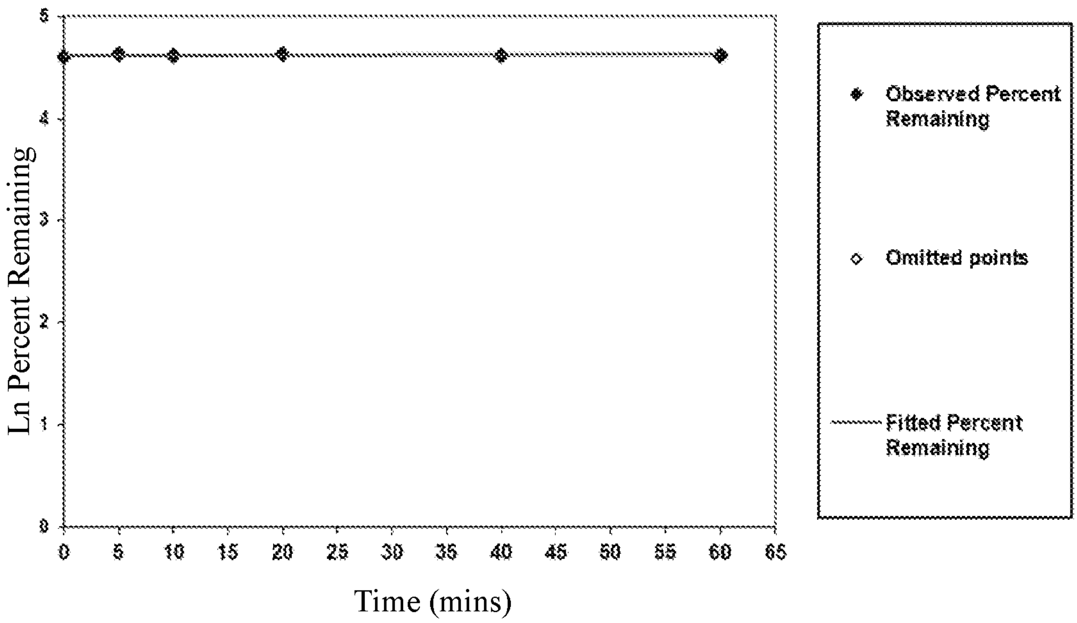


Figure 22

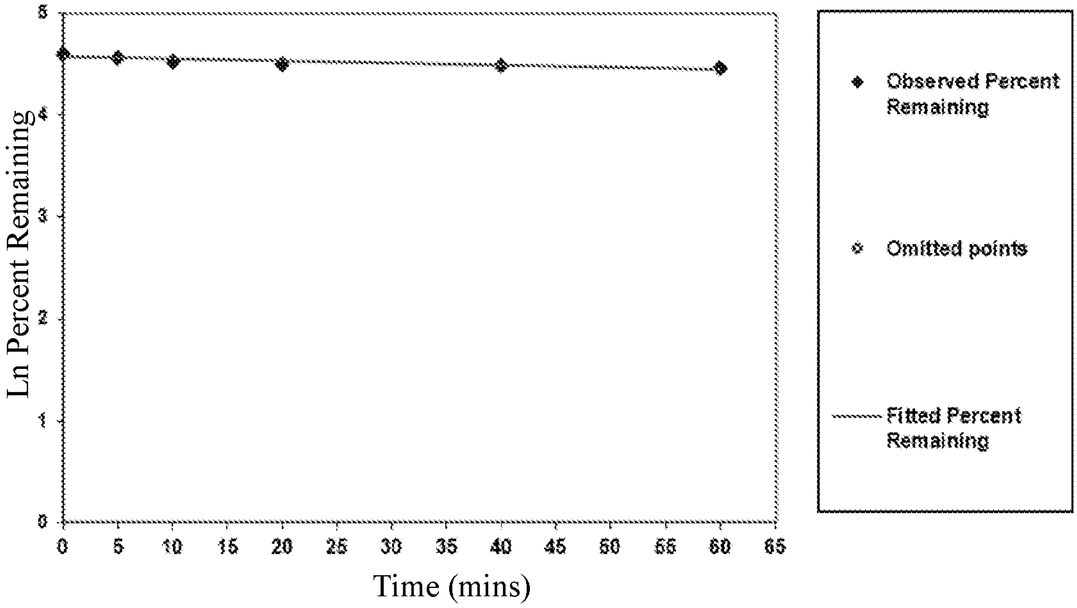


Figure 23

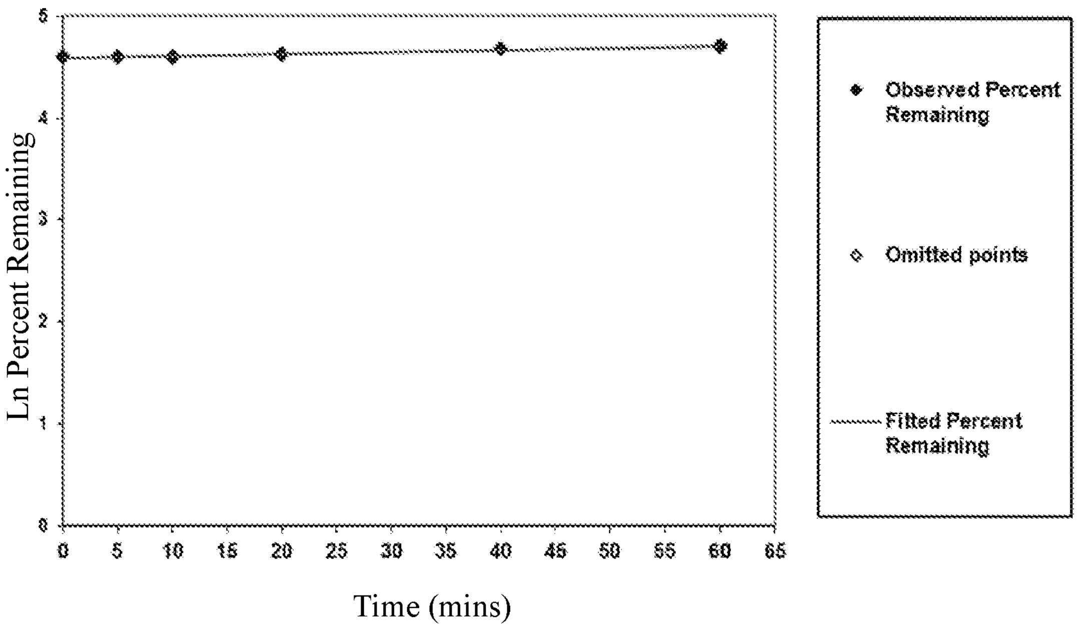


Figure 24

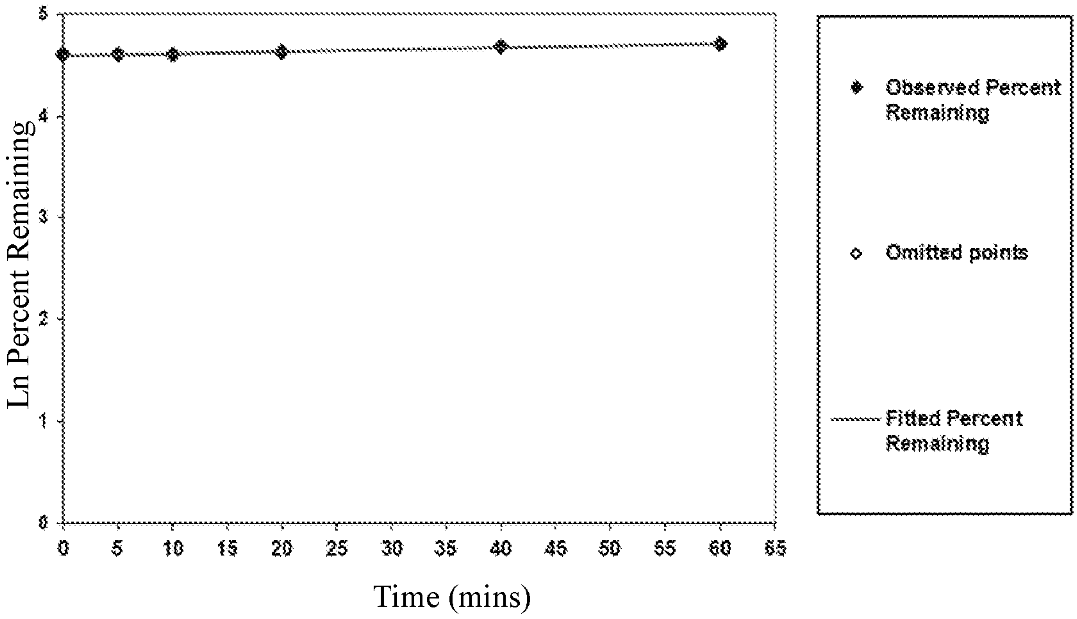


Figure 25

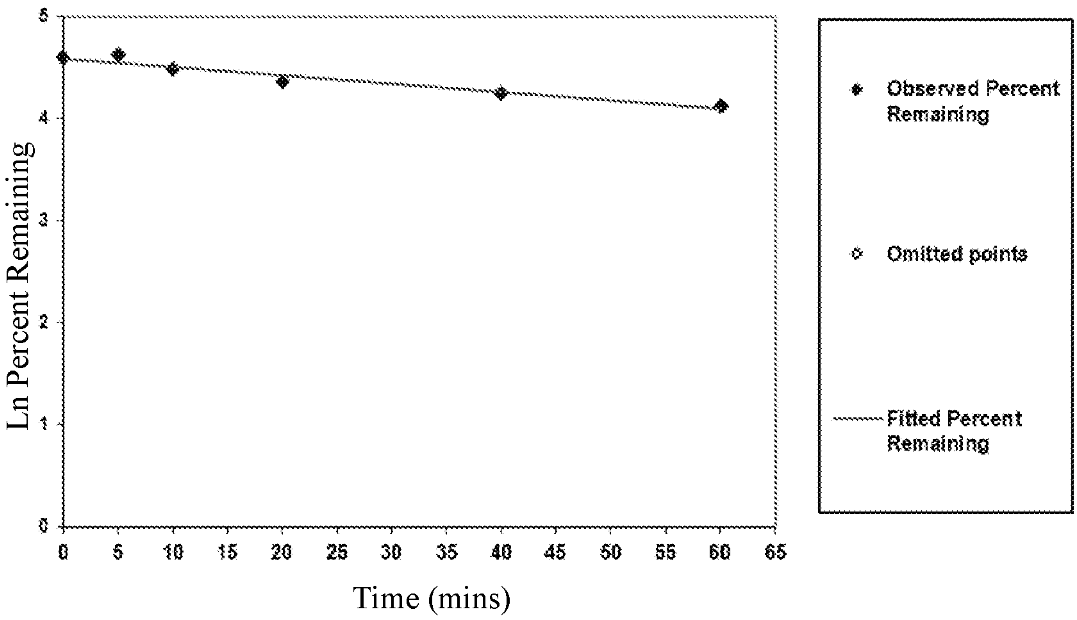


Figure 26

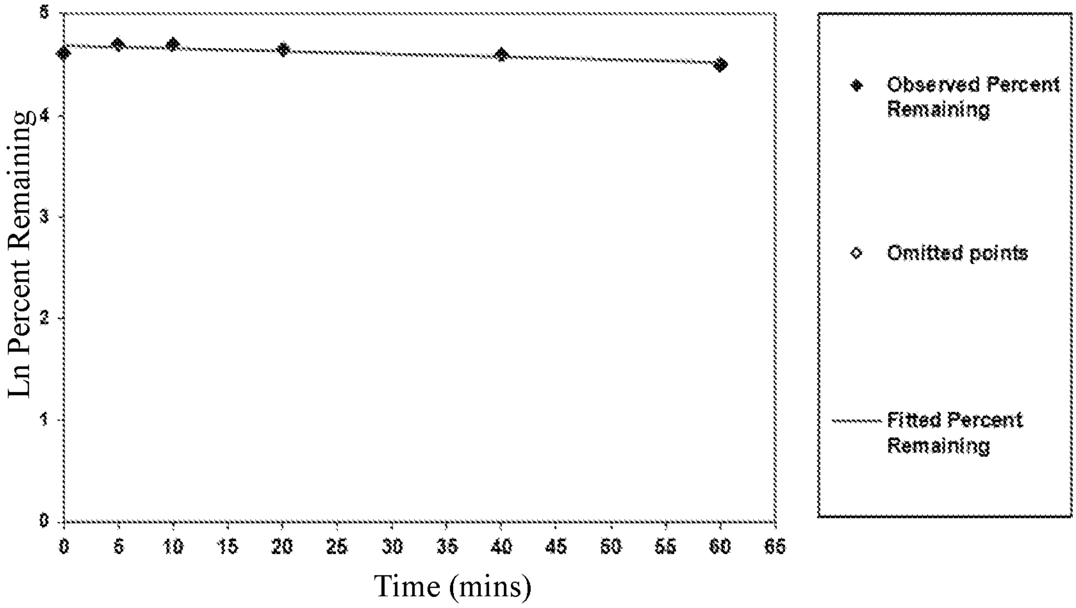


Figure 27

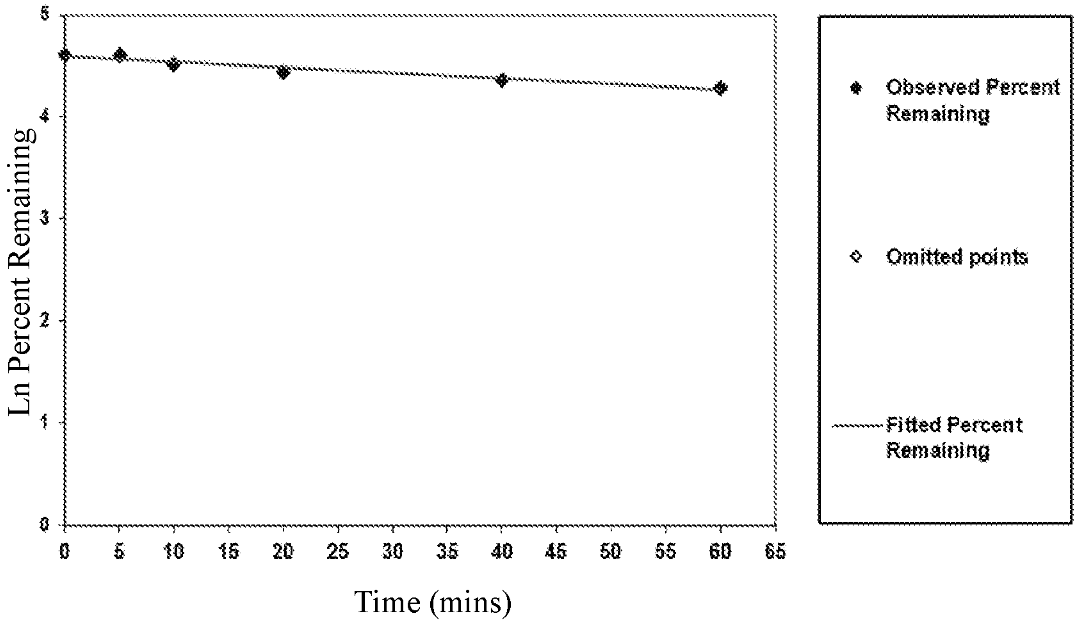


Figure 28

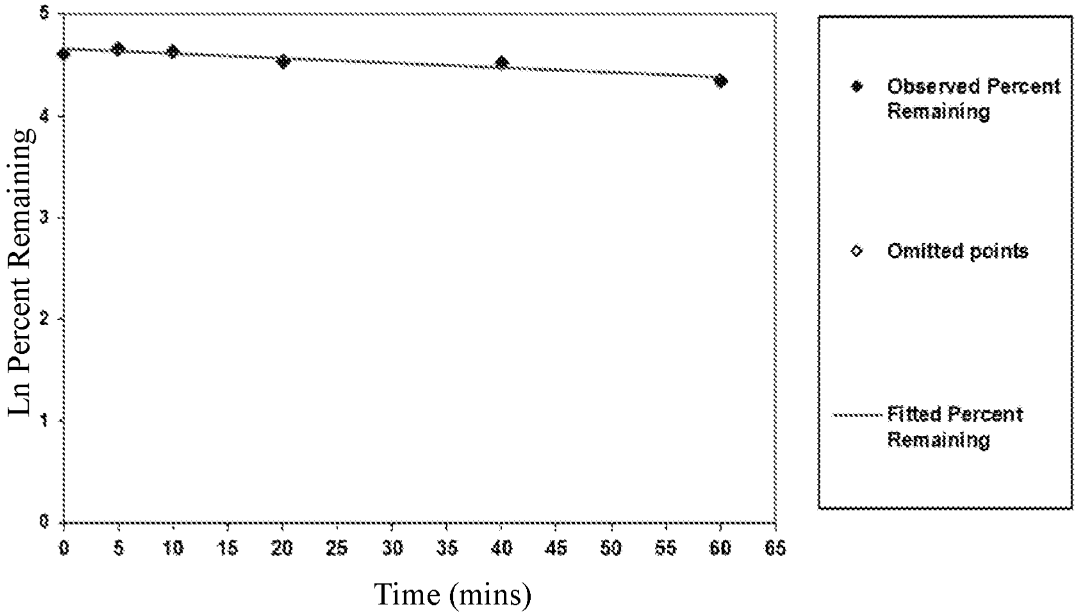


Figure 29

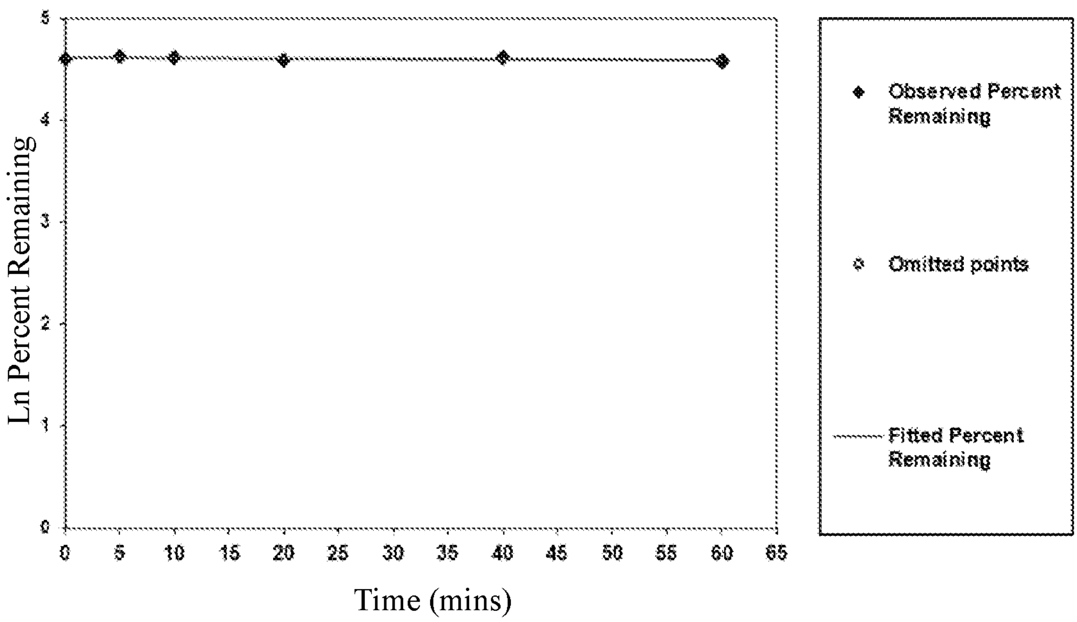


Figure 30

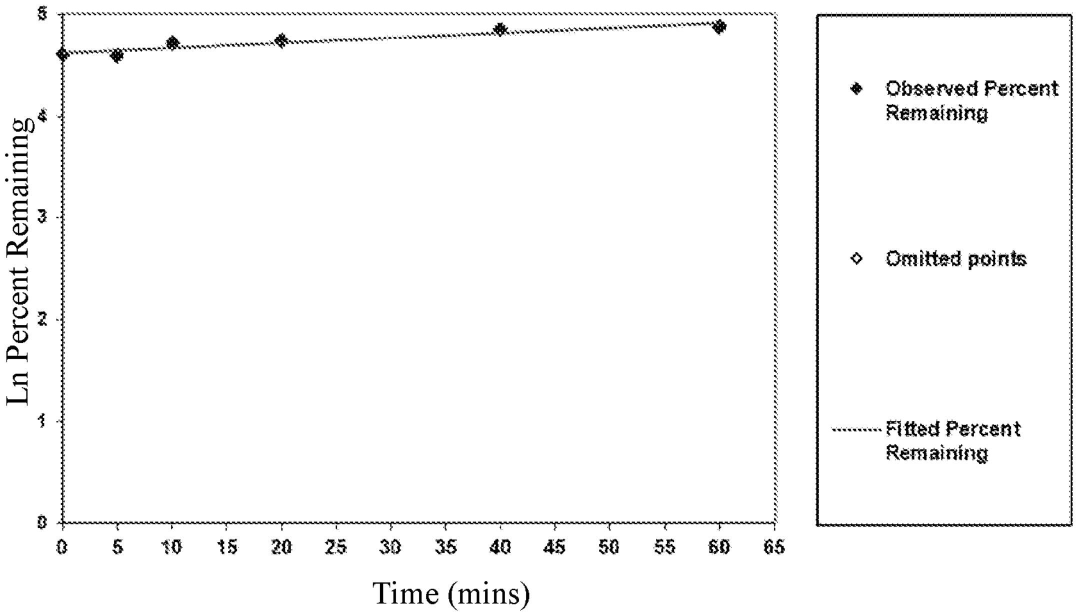


Figure 31

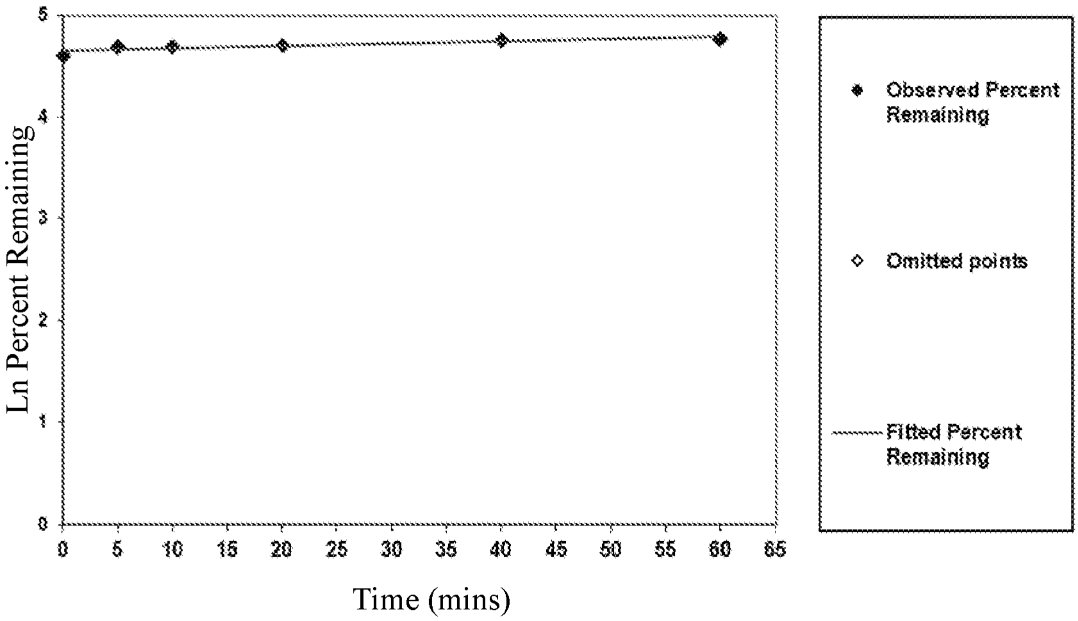


Figure 32

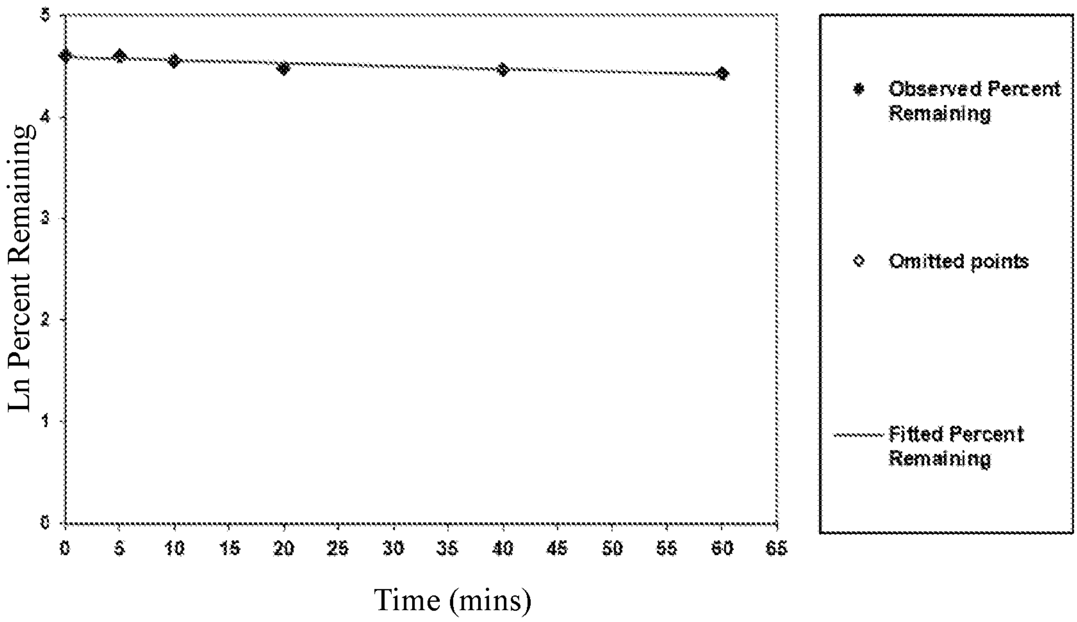
Ln Percent Remaining
0
1
2
3
4
5
0 5 10 15 20 25 30 35 40 45 50 55 60 65
Time (mins)
Observed Percent Remaining
Omitted points
Fitted Percent Remaining

Figure 33

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020165559 A
- GB 2020050287 W


### Non-patent literature cited in the description


- 2012 London Declaration on Neglected Tropical Diseases. Press Release, 30 March 2017
- **T. HIGUCHI ; W. STELLA.** ACS Symposium Series. *Pro-drugs as Novel Delivery Systems,* vol. 14
- Bioreversible Carriers in Drug Design. Pergamon Press, 1987
- **M. E. AULTON.** Pharmaceuticals - The Science of Dosage Form Designs. Churchill Livingstone, 1988
- **AGROTIS A ; KETTELER R.** On ATG4B as Drug Target for Treatment of Solid Tumours-The Knowns and the Unknowns. *Cells,* 2020, vol. 9, 53
- **BAPIRO TE ; SAYI J ; HASLER JA ; JANDE M ; RIMOY G ; MASSELLE A ; MASIMIREMBWA CM.** Artemisinin and thiabendazole are potent inhibitors of cytochrome P450 1A2 (CYP1A2) activity in humans. *Eur J Clin Pharmacol,* 2005, vol. 61, 755-761
- **CEBALLOS L ; ELISSONDO C ; BRUNI SS ; DENEGRI G ; LANUSSE C ; ALVAREZ L.** Comparative Performances of Flubendazole and Albendazole in Cystic Echinococcosis: Ex Vivo Activity, Plasma/Cyst Disposition, and Efficacy in Infected Mice. *Antimicrobial Agents and Chemotherapy,* 2011, vol. 55 (12), 5861-5867
- **CEBALLOS L ; MACKENZIE C ; GEARY T ; ALVAREZ L ; LANUSSE C.** Exploring the Potential of Flubendazole in Filariasis Control: Evaluation of the Systemic Exposure for Different Pharmaceutical Preparations. *PLoS Negl Trop Dis,* 2014, vol. 8 (5), e2838
- **CEBALLOS L ; ALVAREZ L ; MACKENZIE C ; GEARY T ; LANUSSE C.** Pharmacokinetic comparison of different flubendazole formulations in pigs: A further contribution to its development as a macrofilaricide molecule. *International Journal for Parasitology: Drugs and Drug Resistance,* 2015, vol. 5, 178-184
- **CHA HJ ; BYROM M ; MEAD PE ; ELLINGTON AD ; WALLINGFORD JB et al.** Evolutionarily Repurposed Networks Reveal the Well-Known Antifungal Drug Thiabendazole to Be a Novel Vascular Disrupting Agent. *PLoS Biol,* 2012, vol. 10 (8), e1001379
- **CHAUHAN S ; AHMED Z ; BRADFUTE SB ; ARKO-MENSAH J.** Pharmaceutical screen identifies novel target processes for activation of autophagy with a broad translational potential. *Nature Communications,* 2015, vol. 6, 8620
- **CRONSTEIN BN ; TERKELTAUB R.** The inflammatory process of gout and its treatment. *Arthritis Research & Therapy,* 2006, vol. 8 (1), S3
- **DINARELLO CA.** Anti-inflammatory Agents: Present and Future. *Cell,* 2010, vol. 140, 935-950
- **GEARY TG ; MACKENZIE CD ; SILBER SA.** Flubendazole as a macrofilaricide: History and background. *PLoS Negl Trop Dis,* 2019, vol. 13 (1), e0006436, https://doi.org/10.1371/journal.pntd.0006436
- **HAYASHI K ; MICHIUE H ; YAMADA H ; TAKATA K.** Fluvoxamine, an anti-depressant, inhibits human glioblastoma invasion by disrupting actin polymerization. *Nature Scientific Reports,* 2016, vol. 6, 23372
- **HOU Z-J ; LUO X ; ZHANG W ; PENG F ; CUI B ; WU S-J ; ZHENG F-M ; XU J ; XU L-Z ; LONG Z-J.** Flubendazole, FDA-approved anthelmintic, targets breast cancer stem-like cells. *Oncotarget,* 2015, vol. 6 (8), 6326-6340
- **KAJIMURA T ; SATO S ; MURAKAMI A et al.** Overexpression of carbonyl reductase 1 inhibits malignant behaviors and epithelial mesenchymal transition by suppressing TGF-β signaling in uterine leiomyosarcoma cells. *Oncology Letters,* 2019, vol. 18, 1503-1512
- **KALLURI R ; WEINBERG RA.** The basics of epithelial-mesenchymal transition. *J. Clin. Invest.,* 2009, vol. 119, 1420-1428
- **KARJALAINEN MJ ; NEUVONEN PJ ; BACKMAN JT.** In vitro Inhibition of CYP1A2 by Model Inhibitors, Anti-Inflammatory Analgesics and Female Sex Steroids: Predictability of in vivo Interactions. *Basic & Clinical Pharmacology & Toxicology,* 2008, vol. 103, 157-165

- **KRALOVA V ; HANUŠOVÁ V ; CALTOVÁ K ; ŠPAČEK P ; HOCHMALOVÁ M ; SKÁLOVÁ L ; RUDOLF E.** Flubendazole and mebendazole impair migration and epithelial to mesenchymal transition in oral cell lines. *Chem Biol Interact.,* 31 July 2018, vol. 293, 124-132
- **KUBICEK V ; SKÁLOVÁ L ; SKARKA A ; KRÁLOVÁ V ; HOLUBOVÁ J et al.** Carbonyl Reduction of Flubendazole in the Human Liver: Strict Stereospecificity, Sex Difference, Low Risk of Drug Interactions. *Front. Pharmacol,* 2019, vol. 10, 600
- **LACHAU-DURAND S ; LAMMENS L ; VAN DER LEEDE B ; VAN GOMPEL J ; BAILEY G ; ENGELEN M ; LAMPO A.** Preclinical toxicity and pharmacokinetics of a new orally bioavailable flubendazole formulation and the impact for clinical trials and risk/benefit to patients. *PLoS Negl Trop Dis,* 2019, vol. 13 (1), e0007026, https://doi.org/10.1371/journal. pntd.0007026
- **LIN S ; YANG L ; YAO Y ; XU L et al.** Flubendazole demonstrates valid antitumor effects by inhibiting STAT3 and activating autophagy. *Journal of Experimental & Clinical Cancer Research,* 2019, vol. 38, 293, https://doi.org/10.1186/s13046-019-1303-z
- **MICHAELIS M ; AGHA B ; ROTHWEILER F et al.** Identification of flubendazole as potential anti-neuroblastoma compound in a large cell line screen. *Sci Rep.,* 2015, vol. 5, 8202
- **NIXON GL ; MCENTEE L ; JOHNSON A ; FARRINGTON N ; WHALLEY S ; LIVERMORE J ; NATAL C ; WASHBOURN G ; BIBBY J ; BERRY N.** Repurposing and Reformulation of the Antiparasitic Agent Flubendazole for Treatment of Cryptococcal Meningoencephalitis, a Neglected Fungal Disease. *Antimicrobial Agents and Chemotherapy,* 2018, vol. 62 (4), e01909-17
- **NOBILIS M ; JIRA T ; LISA M et al.** Achiral and chiral high-performance liquid chromatographic determination of flubendazole and its metabolites in biomatrices using UV photodiode-array and mass spectrometric detection. *Journal of Chromatography A,* 2007, vol. 1149, 112-120
- **OBACH RS ; WALSKY RL ; VENKATAKRISHNAN K et al.** The Utility of in Vitro Cytochrome P450 Inhibition Data in the Prediction of Drug-Drug Interactions. *Journal of Pharmacology and Experimental; Therapeutics,* 2006, vol. 316, 336-348
- **OBACH RS ; WALSKY RL ; VENKATAKRISHNAN K.** Mechanism-Based Inactivation of Human Cytochrome P450 Enzymes and the Prediction of Drug-Drug Interactions. *Drug Metabolism and Disposition,* 2007, vol. 35, 246-255
- **OH E ; KIM Y-J ; AN H ; SUNG D.** Flubendazole elicits anti-metastatic effects in triple-negative breast cancer via STAT3 inhibition. *Int. J. Cancer,* 2018, vol. 143, 1978-1993
- **O'NEILL M ; MANSOUR A ; DICOSTY U ; GEARY J ; DZIMIANSKI M ; MCCALL SD ; MCCALL JW ; MACKENZIE CD ; GEARY TG.** An In Vitro/In Vivo Model to Analyze the Effects of Flubendazole Exposure on Adult Female Brugia malayi. *PLoS Negl Trop Dis,* 2016, vol. 10 (5), e0004698
- **PANDA PK ; FAHRNER A ; VATS S ; SERANOVA E ; SHARMA V ; CHIPARA M ; DESAI P ; TORRESI J ; ROSENSTOCK T ; KUMAR D.** Chemical Screening Approaches Enabling Drug Discovery of Autophagy Modulators for Biomedical Applications in Human Diseases. *Front. Cell Dev. Biol.,* 2019, vol. 7, 38
- **SESARDIC D ; BOOBIS AR ; MURRAY BP et al.** Furafylline is a potent and selective inhibitor of cytochrome P450IA2 in man Br. *J. clin. Pharmac.,* 1990, vol. 29, 651-663
- **SJOBERG HT ; PIONNIER N ; ALJAYYOUSSI G ; METUGE HM ; NJOUENDOU AJ ; CHUNDA VC et al.** Short-course, oral flubendazole does not mediate significant efficacy against Onchocerca adult male worms or Brugia microfilariae in murine infection models. *PLoS Negl Trop Dis,* 2019, vol. 13 (1), e0006356, https://doi.org/10.1371/journal. pntd.0006356
- **SPAGNUOLO PA ; HU JY ; HURREN R et al.** The antihelmintic flubendazole inhibits microtubule function through a mechanism distinct from Vinca alkaloids and displays preclinical activity in leukemia and myeloma. *Blood,* 2010, vol. 115 (23), 4824-4833
- **STUCHLÍKOVÁ LR ; KRÁLOVÁ V ; LNĚNIČKOVÁ K et al.** The metabolism of flubendazole in human liver and cancer cell lines. *Drug Test Anal,* 2018, vol. 10, 1139-1146
- **TAO J ; ZHAO H ; XIE X ; LUO M et al.** The anthelmintic drug flubendazole induces cell apoptosis and inhibits NF-κB signaling in esophageal squamous cell carcinoma. *OncoTargets and Therapy,* 2019, vol. 12, 471-478
- **TARRUS E ; CAMI J ; ROBERTS DJ et al.** Accumulation of caffeine in healthy volunteers treated with furafylline. *Br. J. clin. Pharmac.,* 1987, vol. 23, 9-18
- **THELINGWANI RS ; ZVADA SP ; DOLGOS H ; UNGELL A-L ; MASIMIREMBWA CM.** In Vitro and in Silico Identification and Characterization of Thiabendazole as a Mechanism-Based Inhibitor of CYP1A2 and Simulation of Possible Pharmacokinetic Drug-Drug Interactions. *Drug Metabolism and Disposition,* 2009, vol. 37, 1286-1294
- **VIALPANDO M ; SMULDERS S ; BONE S et al.** Evaluation of Three Amorphous Drug Delivery Technologies to Improve the Oral Absorption of Flubendazole. *Journal of Pharmaceutical Sciences,* 2016, vol. 105, 2782-2793

- **WILDENBERG ME ; LEVIN AD ; CERONI A ; GUO Z ; KOELINK PJ ; HAKVOORT TBM ; WESTERA L ; BLOEMENDAAL FM ; BRANDSE JF ; SIMMONS A.** Benzimidazoles Promote Anti-TNF Mediated Induction of Regulatory Macrophages and Enhance Therapeutic Efficacy in a Murine Model. *Journal of Crohn's and Colitis,* 2017, 1480-1490
- **YANG J et al.** Guidelines and definitions for research on epithelial-mesenchymal transition. Consensus Statement. *Nature Reviews. Molecular Cell Biology,* 2020, vol. 21, 341-352
- **YU CG ; BONDADA V ; GHOSHAL S ; SINGH R et al.** Repositioning Flubendazole for Spinal Cord Injury. *Journal of Neurotrauma,* 2019, vol. 36, 2618-2630
- **YUN M ; CHOI AJ ; WOO SR et al.** Inhibition of Carbonyl Reductase 1 Enhances Metastasis of Head and Neck Squamous Cell Carcinoma through β-catenin-Mediated Epithelial-Mesenchymal Transition. *Journal of Cancer,* 2020, vol. 11 (3), 533-541
- **ZHEN Y ; ZHAO R ; WANG M ; JIANG X ; GAO F ; FU L ; ZHANG, L ; ZHOU X-L.** Flubendazole elicits anti-cancer effects via targeting EVA1A-modulated autophagy and apoptosis in Triple-negative Breast Cancer. *Theranostics,* 2020, vol. 10 (18), 8080-8097
- **ZHOU X ; LIU J ; ZHANG J ; WEI Y ; LI H.** Flubendazole inhibits glioma proliferation by G2/M cell cycle arrest and pro-apoptosis. *Cell Death Discovery,* 2018, vol. 4, 18